# NEW EUROPEAN PATENT SPECIFICATION

(11) **EP 1 641 803 B3**
(45) Date of publication of this specification: **08.12.2010**
(45) Mention of the grant of the patent: 18.03.2009
(21) Application number: 04736524.2
(22) Date of filing: 10.06.2004
(51) Int. Cl.: C07D 495/04, A61K 31/495, A61P 9/00

(54) **THIENOPYRIMIDINE DERIVATIVES AS POTASSIUM CHANNEL INHIBITORS**
THIENOPYRIMIDIN-DERIVATE ALS KALIUMKANAL-INHIBITOREN
UTILISATION DE DERIVES DE LA THIENOPYRIMIDINE COMME INHIBITEURS DES CANAUX POTASSIQUES

(30) Priority: 11.06.2003 US 477518 P; 11.06.2003 GB 0315950
(43) Date of publication of application: 05.04.2006
(73) Proprietor: Xention Limited, Pampisford Cambridge Cambridgeshire CB22 3EG (GB)
(72) Inventor: FORD, John, Huntingdon PE28 9BN (GB); PALMER, Nicholas, John, Cambridge CB1 3HJ (GB); ATHERALL, John Frederick, Saffron Walden, Essex CB10 2LL (GB); MADGE, David John, East Grinstead, West Sussex RH19 4JZ (GB); SHERBORNE, Brad, Stirling FKB 2JT (GB); BUSHFIELD, Mark, Cambridge CB1 6DY (GB); STEVENS, Edward Benedict, Newmarket, Suffolk CB8 8HW (GB)
(74) Representative: Chapman, Paul William
(86) International application number: PCT/GB2004/002454
(87) International publication number: WO 2004/111057

(56) References cited:
- WO-A-01/46155
- US-A1- 2002 161 011
- US-A1- 2003 027 829
- HOZIEN Z A ET AL: "SYNTHESIS AND APPLICATION OF SOME NEW THIENOPYRIMIDINE DERIVATIVES AS ANTIMICROBIAL AGENTS" SYNTHETIC COMMUNICATIONS, MARCEL DEKKER, INC., BASEL, CH, vol. 26, no. 20, 1996, pages 3733-3755, XP002908881 ISSN: 0039-7911

## Description

The present invention relates to thienopyrimidine compounds which are potassium channel inhibitors. Pharmaceutical compositions comprising the compounds and their use in the treatment of arrhythmia are also provided.

Ion channels are proteins that span the lipid bilayer of the cell membrane and provide an aqueous pathway through which specific ions such as Na⁺, K⁺, Ca²⁺ and Cl⁻ can pass (Herbert, 1998). Potassium channels represent the largest and most diverse sub-group of ion channels and they play a central role in regulating the membrane potential and controlling cellular excitability (Armstrong & Hille, 1998). Potassium channels have been categorized into gene families based on their amino acid sequence and their biophysical properties (for nomenclature see Gutman *et al*., 2003).

Compounds which modulate potassium channels have multiple therapeutic applications in several disease areas including cardiovascular, neuronal, auditory, renal, metabolic and cell proliferation (Shieh *et al.,* 2000; Ford *et al.,* 2002). More specifically potassium channels such as Kv4.3, Kir2.1, hERG, KCNQ1/minK, and Kv1.5 are involved in the repolarisation phase of the action potential in cardiac myocytes. These potassium channels subtypes have been associated with cardiovascular diseases and disorders including long QT syndrome, hypertrophy, ventricular fibrillation, and atrial fibrillation, all of which can cause cardiac failure and fatality (Marban, 2002).

The human delayed rectifier voltage gated potassium channel subunit, Kv1.5, is exclusively expressed in atrial myocytes and is believed to offer therapeutic opportunities for the management of atrial fibrillation for several different reasons (see review of Brendel and Peukert, 2002): (i) There is evidence that Kv1.5 underlies the cardiac ultrarapid delayed rectifier (Kv₍ᵤᵣ₎) physiological current in humans due to similar biophysical and pharmacological properties (Wang *et al.,* 1993; and Fedida *et al.,* 1993). This has been supported with antisense oligonucleotides to Kv1.5 which have been shown to reduce Kv₍ᵤᵣ₎ amplitude in human atrial myocytes (Feng *et al.,* 1997). (ii) electrophysiological recordings have demonstrated that Kv₍ᵤᵣ₎ is selectively expressed in atrial myocytes, and therefore avoids inducing potentially fatal ventricular arrhythmia through interfering with ventricular repolarisation (Amos *et al.,* 1996; Li *et al.,* 1996; and Nattel, 2002). (iii) inhibiting Kv₍ᵤᵣ₎ in atrial fibrillation-type human atrial myocytes prolonged the action potential duration compared to normal healthy human atrial myocytes (Courtemanche *et al.,* 1999). (iv) Prolonging the action potential duration by selectively inhibiting Kv1.5 could present safer pharmacological interventions for protecting against atrial re-entrant arrhythmias such as atrial fibrillation and atrial flutter compared to traditional class III antiarrythmics, by prolonging the atrial refractory period while leaving ventricular refractoriness unaltered (Nattel *et al.,* 1999, Knobloch *et al.,* 2002; and Wirth *et al.,* 2003). Class III antiarrythmics have been widely reported as a preferred method for treating cardiac arrhythmias (Colatsky *et al.,* 1990).

Traditional and novel class III antiarrythmic potassium channel blockers have been reported to have a mechanism of action by directly modulating Kv1.5 or Kv₍ᵤᵣ₎. The known class III antiarrythmics ambasilide (Feng *etal.,* 1997), quinidine (Wang *et al.,* 1995), clofilium (Malayev *et al.,* 1995) and bertosamil (Godreau *et al.,* 2002) have all been reported as potassium channel blockers of Kv₍ᵤᵣ₎ in human atrial myocytes. The novel benzopyran derivative, NIP-142, blocks Kv1.5 channels, prolongs the atrial refractory period and terminates atrial fibrillation and flutter in *in vivo* canine models (Matsuda *et al.,* 2001), and S9947 inhibited Kv1.5 stably expressed in both Xenopus oocytes and Chinese hamster ovary (CHO) cells and Kv₍ᵤᵣ₎ in native rat and human cardiac myocytes (Bachmann *et al.,* 2001). Elsewhere, other novel potassium channel modulators which target Kv1.5 or Kv₍ᵤᵣ₎ have been described for the treatment of cardiac arrhythmias, these include biphenyls (Peukert et al 2003), thiophene carboxylic acid amides (WO0248131), bisaryl derivatives (WO0244137, W00246162), carbonamide derivatives (WO0100573, W00125189) anthranillic acid amides (W02002100825, WO02088073, W002087568), dihydropyrimidines (WO0140231), cycloakyl derivatives (WO03063797), indane derivatives (WO0146155 WO9804521), tetralin benzocycloheptane derivatives (W09937607), thiazolidone and metathiazanone derivatives (WOP9962891), benzamide derivatives (WO0025774), isoquinoline derivatives (WO0224655), pyridazinone derivatives (W09818475 W09818476), chroman derivatives (W09804542), benzopyran derivatives (WO0121610, W003000675, W00121609, W00125224, W002064581), benzoxazine derivatives (WO0012492), and the novel compound A1998 purified from Ocean material (Xu & Xu, 2000).

Thienopyrimidines have been reported to be useful as anti-inflammatory, anti-fungal, anti-osteoporosis and anti-microbial agents amongst others. Although also reported as cardiovascular agents (acting through modulation of the phosphodiesterase group of enzymes or through modulation of the sodium/proton exchange system), thienopyrimidines have not previously been reported as useful agents for modulating ion channels.

Thieno[2,3*-d*]-pyrimidines substituted in the 4-position with an optionally substituted benzylamine or phenethylamine moiety and in the 5-position with a methyl group may serve as anti-inflammatory or anti-osteoporosis agents (Katada *et al.,* 1999). Such compounds were shown to modulate the activity of several cell types including leukocytes, which originate from hematopoietic precursor cells in the bone marrow. Increased activity in leukocytes can lead to various inflammatory diseases; therefore compounds cytotoxic to leukocytes could function as anti-inflammatory drugs. Such compounds are thought to suppress cellular activity by binding to integrins on the surface of leukocytes and preventing downstream cellular signalling events. Thieno[2,3*-d*]pyrimidines substituted in the 4-position with heteroarylthiols, aryl thiols, arylmethyl thiols, heteroarylamines, benzylamine, hydroxyl and chloro groups may also be useful anti-inflammatory agents (Stewart *et al.,* 2001). This series of compounds were shown to inhibit induced expression of cell adhesion molecules on the luminal surface of vascular endothelial thus preventing the adhesion of leukocytes at the site of inflammation.

Thieno[2,3*-d*]pyrimidines with a substituted hydrazine in the 4-position and a phenyl group in the 5 position (Hozien *et al.,* 1996), tetrahydrobenzo[b]thieno[2,3*-d*]pyrimidines (Ismail *et al.,* 1995), thieno[2,3*-d*]pyrimidines which have a hydrogen, chloro, hydrazine, heterocyclyl, amino, methyl, ethyl or phenyl group in the 2-position, an alkylamino, alkylarylamino, amino, dialkylamino or hydrazino substituent in the 4-position, a hydrogen or methyl group in the 5-position, a hydrogen, methyl acetamide or phenyl group in the 6-position or a tetramethylene in the 5,6-position (GB7549025), and the lead series of 5-phenyl- and 5,6-tetramethylenethieno[2,3*-d*]pyrimidines with methyl or phenyl in the 2-position and alkylamino or arylamino in the 4- position (Konno *et al.,* 1989) have all been shown to have anti-microbial activity. Tetrahydrobenzothieno[2,3*-d*]pyrimidine with the 2-oxo-3-pyrrolidinylmethylene-hydrazino moiety in the 4-position showed some herbicidal activity against velvet leaf (Ram *et al.,* 1981). It has also been reported that 4-chlorotetrahydrobenzothieno[2,3*-d*]pyrimidine is herbicidal, tetrahydrobenzothieno-[2,3*-d*]pyrimidines with a thiol, hydrazine, 2-fluoroanilino, 3-fluoroanilino or 4-diethylanilino substituent in the 4-position are bactericidal against *Streptococcus fecales* and tetrahyrobenzothieno[2,3*-c*]pyrimidines with a 2,4-dichlorobenzylamino or 2-fluoroanilino substituent in the 4-position are fungicidal against Pythium (Ram, 1979). Thieno[2,3*-d*]pyrimidines with a hydrogen, hydroxyl, thiol, halogen or cyano group in the 2-position, alkylamino, arylalkylamino or hydroxyalkyl amino groups in the 4-position, a hydrogen, alkyl or halogen in the 5- and/or 6- position or alkylene in the 5,6-position have been reported as tick-control agents (AU 521790).

Elsewhere, tetrahydrobenzo[b]thieno[2,3*-d*]pyrimidines exhibited anti-tumour activity (Shehata *et al.,* 1996) and analgesic activity half that of aspirin (Moneer *et al.,* 1994), a series of thieno[2,3*-d*]pyrimidines with 4-alkylamino or arylamino, 5-H or 5-methyl, 6-methyl or 5,6-tetramethylene were shown to have potential as anticytokinins (Jordis *et al*., 1986), a series of 5,6-dimethyl-thieno[2,3*-d*]pyrimidines and 5,6-tetramethylenethieno[2,3*-d*]pyrimidines, both substituted in the 2-position with arylamines or heterocyclic amines and in the 4-position with arylamines displayed blood platelet aggregation inhibiting properties (DD 226893), pyrano- and thiopyrano[3,4-b]thieno[5,4*-d*]pyrimidines with the 4-position substituted with amino, butylamine, aniline, cyclohexylamine, benzylamine, phenethylamine and 2-hydroxyethylamine have been reported to exhibit anticonvulsive activity (Noravyan *et al.,* 1977), and 4-[(Benzo-2,1,3-thiadiazolyl-4)amino]-5,6,7,8-tetrahydrobenzothieno-(2,3*-d*)-pyrimidine has been reported to possess anthelmintic activity in larval alveolar echinococcosis (RU 2116309).

Thieno[2,3*-d*]pyrimidines with a substituted amino group at the 4-position, hydrogen, alkyl or halo substitution at the 5 and 6-positions and an alkyl chain at the 2-position are claimed to be inhibitors of phosphodiesterase V and useful in the treatment of cardiovascular diseases and for disturbances in potency (DE10104802).

Elsewhere, 5-alkyl thieno[2,3*-d*]pyrimidines with a piperazinyl substituent at the 4-position were found to be inhibitors of the sodium/proton exchanger and useful in the treatment of various cardiovascular disorders, including angina pectoris and arrhythmia (WO 01/27107).

4-[(Phenyl)amino]-thieno[2,3*-d*]pyrimidines bearing a 5-thiophenyl substituent and a 2-methyl substituent were found to have molluscicidal activity (Hosni et al, Acta Poloniae Pharmaceutica, 1999, 56(1), 49-56).

Recently thienopyrimidines have also been reported as potent VEGFR inhibitors (Munchhof, 2004).

Several publications disclose compounds which are indicated as acting on potassium channels. Thus, US6531495 discloses 2'-aminomethylbiphenyl-2-carboxamides, W02002/100825 discloses anthranillic acid amides as antiarrhythmics and W02002/036556 discloses acylaminoalkylbenzenesulfonamides as cardiovascular agents.

This invention provides compounds that are potassium channel inhibitors. These compounds are particularly useful for inhibiting potassium channels Kv1.5 or Kv₍ᵤᵣ₎, which are known targets for the treatment of cardiac arrhythmia in the atria such as atrial fibrillation (Nattel metal, 1999; Wang *et al*., 1993). This invention is not limited to the compounds disclosed for treating cardiac arrhythmias, the compounds also being useful to treat diseases which require potassium channel inhibition (e.g. Shieh *et al.,* 2000; Ford *et al.,* 2002). Thus, in a first aspect, the present invention provides a compound of formula (I) 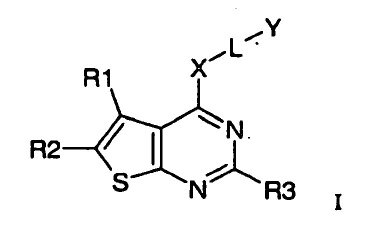 Wherein
R1 is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃₋₆ cycloalkyl;
R2 is H, nitro, -CO₂R7, CONR4R5 halo, or C₁₋₆alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, NHC(O)R8, halo, trifluoromethyl, nitrile, C₁₋₃ alkoxy, or C₁₋₆ alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O) NR⁹R¹⁰, NHC(O)R8 or SO₂NR⁹R¹⁰;
R4 and R5 may be the same or different, and may be H; C₁₋₆alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O) R8 or SO₂NR⁹R¹⁰; C₆-C₁₀ aryl which is unsubstituted or substituted with cyano, halogen, nitro, trifluoromethyl, alkyl, alkylthio, alkoxy, NR⁹R10, CO2R7, C(O)NR9R10, NHC(O)R8, SO2NR9R10 0 or hydroxyl; heteroaryl which is unsubstituted or substituted with cyano, nitro, halogen, alkyl, alkylthio, alkoxy, NR9R10, CO2R7, C(O)NR9R10, NHC(O)R8, SO2NR9R10 or hydroxyl; or C₃-C₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁₋₆alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is C₆-C₁₀ aryl which is unsubstituted or substituted with cyano, halogen, nitro, trifluoromethyl, alkyl, alkylthio, alkoxy, NR9R10, CO2R7, C(O)NR9R10, NHC(O)R8, SO2NR9R10 or hydroxyl; an heterocyclic group, which is unsubstituted or substituted with cyano, nitro, halogen, alkyl, alkylthio, alkoxy, NR9R10, CO2R7, C(O)NR9R10, NHC(O)R8, SO2NR9R10 or hydroxyl;alkenyl; C₃-C₆ cycloalkyl; an unsubstituted C₁-C₆ alkyl or C₁-C₆ alkyl substituted with halogen, cyano, nitro, NR9R10, alkoxy, unsubstituted aryl, unsubstituted heteroaryl, C(O)NR9R10, NHC(O)R8 or SO₂NR9R10; R9 and R10 0 can be the same or different, and may be selected from H, unsubstituted C₁₋₆ alkyl, unsubstituted C₆-C₁₀ aryl, unsubstituted heteroaryl, unsubstituted cycloalkyl, aminoethyl, methylaminoethyl, dimethylaminoethyl, hydroxylethyl, alkoxyethyl, or R9 and R10 may together form a saturated or partially saturated 4-7 member ring, wherein said ring may optionally comprise one, two or three further heteroatoms.
or pharmaceutically acceptable salts thereof;
with the proviso that when Y is phenyl, phenyl monosubstituted by Cl or methoxy, furanyl, tetrahydrofurayl, pyrimidinyl, pyrrolidinyl or 1,3-benzodioxolyl, then R1 is not phenyl, phenyl monosubstituted by halogen or phenyl substituted by methyl;
and wherein the compound is not:
N-butyl-5-phenylthieno[2,3-d]pyrimidin-4-amine;
5-phenyl-N-(pyridin-2-ylmethyl)thieno[2,3-d]pyrimidin-4-amine;
5-(4-chlorophenyl)-N-[3-(1H-imidazol-1-yl)propyl]thieno[2,3-d]pyrimidin-4-amine;
5-(4-chlorophenyl)-N-(pyridin-2-ylmethyl)thieno[2,3-d]pyrimidin-4-amine;
5-(4-Chlomphenyl)-N-(2-cyclohex-1-en-1-ylethyl)thieno[2,3-d]pyrimidin4-amine;
5-(4-Chlorophenyl)-N-(pyridin-3-ylmemyl)thieno[2,3-d]pyrimidin-4-amine;
5-(4-Chlorophenyl)-N-(2-furylmethyl)thieno[2,3-d]pyrimidin-4-amine;
5-(4-Fluorophenyl)-N-(pyridin-3-ylmethyl)thieno[2,3-d]pyrimidin-4-amine;
N-Allyl-5-phenylthieno[2,3-d]pyrimidin-4-amine;
5-(4-Methylphenyl)-N-(2-thien-2-ylethyl)thieno[2,3-d]pyrimidin-4-amine;
N-(2-Furymethyl)-5-phenylthieno[2,3-d]pyrimidin-4-amine;
5-(4-Chlorophenyl)-N-(2-thien-2-ylethyl)thieno[2,3-d]pyrimidin-4-amine;
5-(4-Fluorophenyl)-N-(2-thien-2-ylethyl)thieno[2,3-d]pyrimidin-4-amine;
N-Allyl-5-(4-chlorophenyl)thieno[2,3-d]pyrimidin-4-amine;
5-(4-Chlorophenyl)-N-(tetrahydrofuran-2-ylmethyl)thieno[2,3-d]pyrimidin-4-amine;
5-Phenyl-N-(tetrahydrofuran-2-ylmethyl)thieno[2,3-d]pyrimidin-4-amine;
5-(4-Bromophenyl)-N-(pyridin-3-ylmethyl)thieno[2,3-d]pyrimidin-4-amine;
N-[3-(1H-Imidazol-1-yl)propyl]-5-phenylthieno[2,3-d]pyrimidin-4-amine;
1-(2-{[5-(4-Methylphenyl)thieno[2,3-d]pyrimidin-4-yl]amino}ethyl)imidazolidin-2-one; or
N-(2-Furylmethyl)-5-(4-methylphenyl)thieno[2,3-d]pyrimidin-4-amine.
N-ethyl-2-methyl-5-(thiophen-2-yl)thieno[2,3-d]pyrimidin-4-amine

As used herein, an alkyl group or moiety is typically a linear or branched alkyl group or moiety containing from 1 to 6 carbon atoms, such as a C₁-C₄ alkyl group or moiety, for example methyl, ethyl, n-propyl, i-propyl, butyl, 1-butyl and t-butyl. An alkyl group or moiety may be unsubstituted or substituted at any position. Typically, it is unsubstituted or carries one or two substituents. Suitable substituents include halogen, cyano, nitro, NR9R10, alkoxy, hydroxyl, unsubstituted aryl, unsubstituted heteroaryl, CO₂R7, C(O)NR9R10, NHC(O)R8 and SO₂NR9R10.

As used herein, an aryl group is typically a C₆-C₁₀ aryl group such as phenyl or napthyl. A preferred aryl group is phenyl. An aryl group may be unsubstituted or substituted at any position. Typically, it carries 1, 2, 3 or 4 substituents. Suitable substituents include cyano, halogen, nitro, trifluoromethyl, alkyl, alkylthio, alkoxy, NR9R10, CO₂R7, C(O) NR9R10, NHC(O)R8 and SO₂NR9R10 and hydroxyl.

As used herein, a heterocyclic group is a heteroaryl group, typically a 5- to 10- membered aromatic ring, such as a 5- or 6- membered ring, containing at least one heteroatom selected from O, S and N. Examples include pyridyl, pyrazinyl, pyrimidinyl, pyridazinyl, furanyl, thienyl, pyrazolidinyl, pyrrolyl and pyrazolyl groups. Preferred heteroaryl groups are furanyl, thienyl and pyridyl. Examples of polycyclic heterocycles include indolyl, benzofuranyl, benzothiophenyl and benzodioxolyl. Non-aryl heterocyclic groups are also included, such as tetrahydrofuranyl or pyrrolidinyl. A heterocyclic group may be unsubstituted or substituted at any position. Suitable substituents include cyano, nitro, halogen, alkyl, alkylthio, alkoxy, NR9R10, CO₂R7, C(O)NR9R10, NHC(O)R8 and SO₂NR9R10 and hydroxyl.

R9 and R10 can be the same or different, and may be selected from H, unsubstituted alkyl, unsubstituted aryl, unsubstituted heteroaryl, unsubstituted cycloalkyl, aminoethyl, methylaminoethyl, dimethylaminoethyl, hydroxyethyl, alkoxyethyl, or R9 and R10 may together form a saturated, unsaturated or partially saturated 4 to 7 member ring.

When R4 and R5 or R9 and R10 together form a saturated, unsaturated or partially saturated 4 to 7 member ring, the ring may optionally comprise one, two, or three further heteroatoms.

As used herein, alkoxy means C₁₋₃ alkoxy, cycloalkyl means C₃₋₆ cycloalkyl and halogen means Cl, F, Br, or I, preferably Cl, F or Br.
Preferred compounds of formula I are those wherein R1 is aryl or heteroaryl, R2 is H or alkyl, R3 is H, NR4R5, alkoxy or alkyl, X is O or NR6, R6 is H, n is 1 or 2 and Y is alkyl, cycloalkyl, aryl or heteroaryl.

More preferred compounds of formula I are those wherein R1 is aryl or heteroaryl, R2 is H or methyl, R3 is H, NR4R5, alkoxy or alkyl, X is NR6, R6 is H, n is 1 and Y is heteroaryl. Preferably Y is furanyl, thienyl or pyridyl. More preferably Y is optionally substituted furan-2-yl or optionally substituted pyridin-2-yl.

Preferred compounds include:
2-{5-Phenyl-4-[(pyridin-2-ylmethyl)-amino]-thieno[2,3-d]pyrimidin-2-yl}-propane-1,3-diol;
2-{5-(4-Fluorophenyl)-4-[(pyridin-2-ylmethyl)-amino]-thieno[2,3-d]pyrimidin-2-ylamino}-ethanol;
Pyridin-2-ylmethyl-(5-p-tolyl-thieno[2,3-d]pyrimidin-4-yl)-amine;
2-{5-Phenyl-4-[(pyridin-2-ylmethyl)-amino]-thieno[2,3-d]pyrimidin-2-ylamino}-ethanol;
2-{5-Phenyl-4-[(pyridin-2-ylmethyl)amino]thieno[2,3*-d*]pylimidin-2-yl}ethanol;
2-((2-Hydroxy-ethyl)-{5-phenyl-4-[(pyridin-2-ylmethyl)-amino]-thieno[2,3-d]pyrimidin-2-yl}-amino)-ethanol;
2-Methyl-N-(2-pyridyl)methyl-5-phenylthieno[2,3-d]pyrimidin-4-ylamine;
2-{4-[(Furan-2-ylmethyl)-amino]-5-phenyl-thieno[2,3-d]pyrimidin-2-yl}-ethanol;
[2-(2-Methoxy-ethoxy)-5-phenyl-thieno[2,3-d]pyrimidin-4-yl]-pyridin-2-ylmethyl-amine;
(2-Methoxy-5-phenyl-thieno[2,3-d]pyrimidin-4-yl)-pyridin-2-ylmethyl-amine;
5-(4-Fluorophenyl)-N²-(2-methoxy-ethyl)-N⁴-pyridin-2-ylmethyl-thieno[2,3-d]pyrimidine-2,4-diamine;
[5-(4-Dimethylamino-phenyl)-thieno[2,3-d]pyrimidin-4-yl]-pyridin-2-ylmethyl-amine;
5-(4-Fluorophenyl)-N²,N²-dimethyl-N⁴-pyridin-2-ylmethyl-thieno[2,3-d]pyrimidine-2,4-diamine;
Pyridin-2-ylmethyl-[5-(4-trifluoromethyl-phenyl)-thieno[2,3-d]pyrimidin-4-yl]-amine;
[5-(1H-Indol-6-yl)-thieno[2,3-d]pyrimidin-4-yl]-pyridin-2-ylmethyl-amine;
(5-Benzo[1,3]dioxol-5-yl-thieno[2,3-d]pyrimidin-4-yl)-pyridin-2-ylmethyl-amine;
2-{5-Phenyl-4-[(pyridin-2-ylmethyl)-amino]-thieno[2,3-d]pyrimidin-2-ylamino}-propane-1,3-diol;
3-{5-Phenyl-4-[(pyridin-2-ylmethyl)-amino]-thieno[2,3-d]pyrimidin-2-ylamino}-propane-1,2-diol;
N-Methyl-2-{5-phenyl-4-[(pyridin-2-ylmethyl)amino]thieno[2,3-d]pyrimidin-2-yl}acetamide; or
6-Methyl-N-[(6-methylpyridin-2-yl)methyl]-5-phenylthieno[2,3-d]pyrimidin-4-amine;
and pharmaceutically acceptable salts thereof.

Compounds of formula I wherein R3 is H, alkyl or trifluoroalkyl are synthesised from a compound of formula II by reaction with a suitable nucleophile X-L-Y, where X, Y and L are as defined herein, optionally in the presence of a solvent and a base, and optionally at elevated temperature or with microwave irradiation. Preferably the solvent (if present) is an alcohol, preferably ethanol, and the base is a hindered nitrogen base such as triethylamine. If a solvent is present the reaction is carried out at the reflux temperature of the solvent, or under sealed conditions and with microwave irradiation at a temperature of 120-160°C.

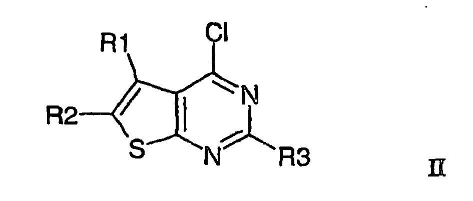

A compound of formula II may be obtained from a compound of formula III by reaction with a chlorinating reagent such as phenylphosphonic dichloride or phosphorous oxychloride in a suitable solvent or no solvent, and with heating. Preferably the chlorinating reagent is phosphorous oxychloride and the reaction is carried out at reflux temperature and in the absence of additional solvent.

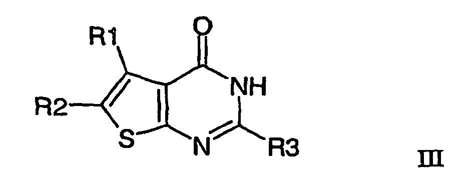

Compounds of formula III may be obtained by the reaction of a compound of formula IV with a suitable substituted or unsubstituted amidine of formula V, or its salt equivalent. The reaction may be carried out in the presence of a suitable solvent at elevated temperature. Preferably the solvent is ethanol and the reaction is carried out under reflux conditions.

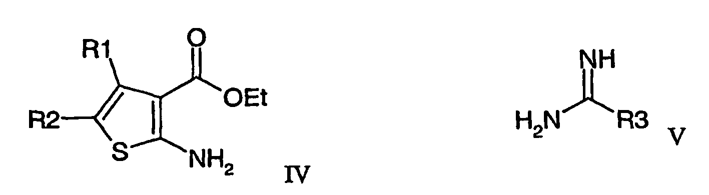

In an alternative synthesis of compounds of formula III, also applicable to those examples wherein R3 is an alkyl group, reaction of a compound of formula VI under basic conditions in a suitable solvent is performed. Suitable bases include alkali metal alkoxides such as sodium methoxide. Suitable solvents include alcohols such as methanol.

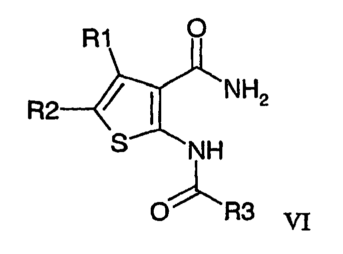

A compound of formula VI can be prepared by reaction of a compound of formula VII under acylating conditions, for example in the presence of an acid chloride and a base. Exemplified acid chlorides include acetyl chloride. Suitable bases include the nitrogenous bases such as triethylamine and pyridine.

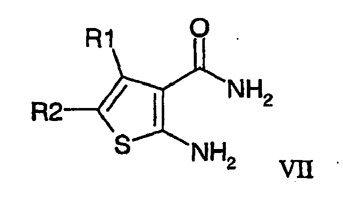

Compounds of formula VII are widely available from standard commercial sources or may be obtained from compounds of formula IV by simple functional group interconversions.

Compounds of formula V are widely available from standard commercial sources or can be synthesised by routine organic chemistry procedures.

A compound of formula IV can be prepared by reaction of a compound of formula VIII, under basic conditions and in a suitable solvent, with powdered sulphur. Preferably the base is diethylamine and the reaction is carried out at 25 to 65°C. The solvent may be an alcohol, preferably ethanol.

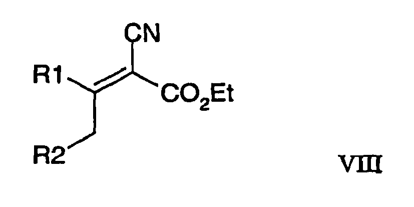

Compounds of formula VIII can be prepared by heating a compound of formula IX with ethylcyanoacetate (NCCH₂CO₂Et) in the presence of an acid and ammonium acetate in a suitable solvent, optionally with azeotropic water removal. Preferably the acid is acetic acid.

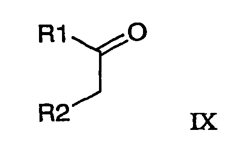

Compounds of formula IX are widely available from commercial sources or can be readily synthesised using standard synthetic organic chemistry procedures.

Alternatively, compounds of formula I wherein R3 is other than H, alkyl or trifluoroalkyl, can be prepared from a compound of formula X by displacement of the 2-chloro substituent with a suitable nucleophilic species. Such a reaction may be carried out with heating or microwave irradiation.

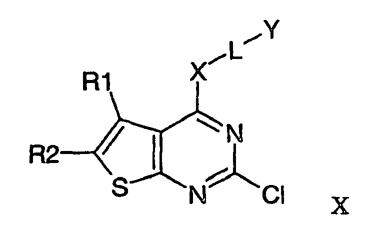

Compounds of formula X are readily synthesised from compounds of formula XI by reaction with a suitable nucleophile X-L-Y, optionally in the presence of a solvent and a base, and optionally at elevated temperature or with microwave irradiation. Preferably the solvent (if present) is an alcohol, preferably propan-2-ol, and the base is a hindered nitrogen base such as triethylamine. The reaction is carried out at ambient temperatures.

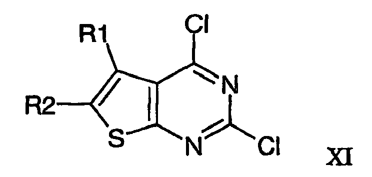

A compound of formula XI may be synthesised by reaction of a compound of formula XII with a chlorinating reagent such as phenylphosphonic dichloride or phosphorous oxychloride.

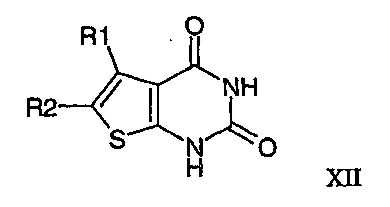

Compounds of formula XII are available by the reaction of a compound of formula IV with an alkali metal cyanate, preferably potassium cyanate.

Alternatively, compounds of formula I wherein R3 is an ester-substituted alkyl group, in particular an acetic acid ester, can be prepared by the reaction of a compound of formula XIII, by reaction with a suitable nucleophile X-L-Y optionally in the presence of a solvent and a base, and optionally at elevated temperature or with microwave irradiation. Preferably the solvent (if present) is an alcohol, preferably ethanol, and the base is a hindered nitrogen base such as triethylamine. If a solvent is present the reaction is carried out at the reflux temperature of the solvent, or under sealed conditions and with microwave irradiation at a temperature of 120-160°C.

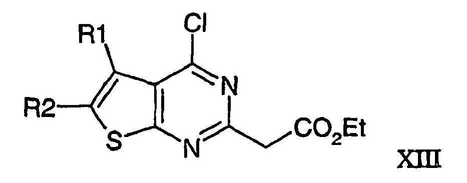

Compounds of formula XIII may be synthesized from compounds of formula XIV by reaction with a chlorinating g reagent such as phenylphosphonic dichloride or phosphorous oxychloride.

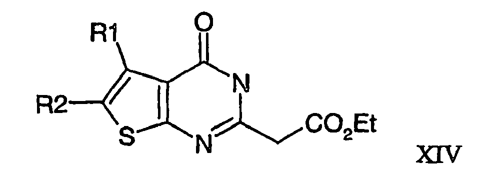

Compounds of formula XIV are synthesized by reaction of compounds of formula IV with ethyl cyanoacetate, performed under acidic conditions with or without the presence of solvent. Suitable acids include gaseous hydrogen chloride.

It is understood that compounds of formula I wherein R3 is a carboethoxy group may undergo functional group transformation of the ester moiety using methods familiar to those skilled in the art. In a preferred instancesuch compounds may undergo amidation by reaction with an alkyl or dialkylamine. In another preferred instance compounds of formula I wherein R3 is a 1-hydroxyethyl group can be prepared by reaction with a reducing agent such as diisobutylaluminium hydride or lithium aluminium hydride. In a further instance compounds of formula I wherein R3 is a carboethoxy group may be reacted with a dialkyl carbonate under basic conditions to provide a compound of formula I wherein R3 is a dialkylmalonyl group. Such compounds may be reduced, preferably with a reducing agent such as diisobutylaluminium hydride or lithium aluminium hydride, to provide compounds of formula I wherein R3 is a propanediol group.

Compounds of formula I wherein R3 is a chloromethyl group may be synthesized from compounds of formula XV by reaction with a suitable nucleophile X-L-Y optionally in the presence of a solvent and a base, and optionally at elevated temperature or with microwave irradiation. Preferably the solvent (if present) is an alcohol, preferably propan-2-ol, and the base is a hindered nitrogen base such as triethylamine. The reaction is carried out at ambient temperature.

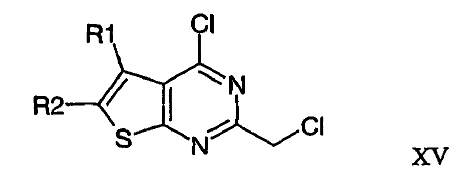

Compounds of formula XV may be synthesized from compounds of formula XVI by reaction with a chlorinating reagent such as phenylphosphonic dichloride or phosphorous oxychloride.

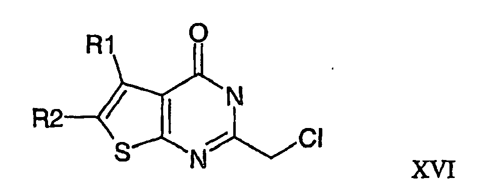

Compounds of formula XVI may be synthesized by reaction of a compound of formula IV under acidic conditions in a suitable solvent with chloroacetonitrile. Suitable acids include gaseous hydrogen chloride. Suitable solvents include alkyl ethers such as 1,4-dioxane.

It is understood that compounds of formula I wherein R3 is a chloromethyl group may undergo standard functional group transformations of the chloromethyl moiety using methods familiarto those skilled in the art. In a preferred instance reaction with a nucleophile is carried out. Suitable nucleophiles may include an alkyl or dialkyl amine, an alcohol or a thiol, or anion derivatives thereof.

In an alternative synthesis of compounds of formula I, particularly applicable to those examples wherein R1 comprises an aryl or heteroaryl group, a compound of formula XVII is reacted with an aryl or heteroaryl boronic acid, preferably under coupling conditions such as in the presence of a palladium(0) catalyst, preferably tetrakis(triphenylphosphine) palladium(0) which may be generated in situ or attached to a polymer resin. Alternative coupling conditions will be familiar to those skilled in the art. If a solvent is present the reaction is carried out at the reflux temperature of the solvent, or under sealed conditions and with microwave irradiation at a temperature of 120-160°C.

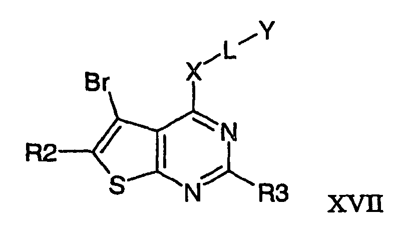

A compound of formula XVII may be synthesised from a compound of formula XVIII by reaction with a suitable nucleophile X-L-Y optionally in the presence of a solvent and a base, and optionally at elevated temperature or with microwave irradiation. Preferably the solvent (if present) is an alcohol, preferably ethanol, and the base is a hindered nitrogen base such as triethylamine. If a solvent is present the reaction is carried out at the reflux temperature of the solvent, or under sealed conditions and with microwave irradiation at a temperature of 120-160°C.

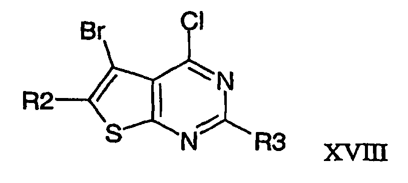

Compounds of formula XVIII wherein R2 is H may be synthesized by reaction of a compound of formula SIX under basic conditions at reduced temperature in a suitable solvent. Preferably the base is an alkyllithium, in the most preferred instance lithium diisopropylamide and the solvent is an alkylether, in the most preferred instance tetrahydrofuran. The reaction may be carried out from -80°C to ambient temperature.

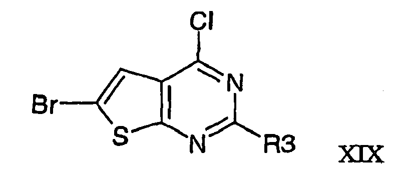

A compound of formula XIX may be obtained from a compound of formula XX by reaction with a chlorinating reagent such as phenylphosphonic dichloride or phosphorous oxychloride in a suitable solvent or no solvent, and with heating. Preferably the chlorinating reagent is phosphorous oxychloride and the reaction is carried out at reflux temperature and in the absence of additional solvent.

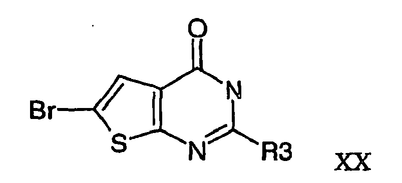

Compounds of formula XX may be synthesized by reactions of compounds of formula III wherein R1 is H and R2 is H with an electrophilic halogenating reagent preferably bromine in a suitable solvent preferably glacial acetic acid.

Alternatively, compounds of formula XVIII wherein R3 is other than H, alkyl ortrifluoroalkyl and R2 is other than hydrogen may be synthesized from compounds of formula XXI by displacement of the 2-chloro substituent with a suitable nucleophilic species. Such a reaction may be carried out with heating or microwave irradiation.

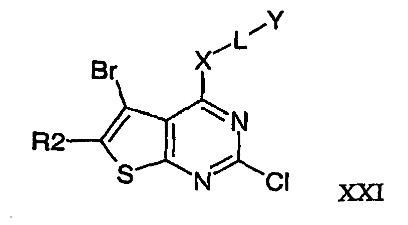

Compounds of formula XXI are readily synthesised from compounds of formula XXII by reaction with a suitable nucleophile X-L-Y, optionally in the presence of a solvent and a base, and optionally at elevated temperature or with microwave irradiation. Preferably the solvent (if present) is an alcohol, preferably ethanol, and the base is a hindered nitrogen base such as triethylamine.

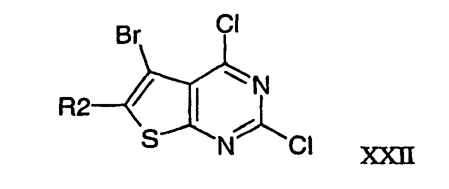

A compound of formula XXII may be synthesised by reaction of a compound of formula XXIII with a chlorinating reagent such as phenylphosphonic dichloride or phosphorous oxychloride.

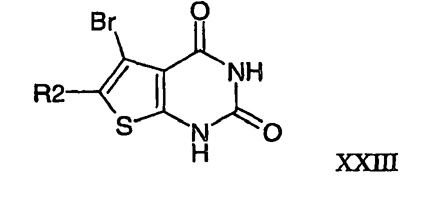

A compound of formula XXIII may be synthesized by reaction of a compound of formula XII wherein R1 is H and R2 is alkyl with an electrophilic halogenating reagent, preferably bromine, in a suitable solvent, preferably glacial acetic acid.

Many of the starting materials referred to in the reactions described above are available from commercial sources or can be made by methods cited in the literature references. Synthetic methods can also be found in reviews; thiophenes for example can be found in references cited in Comprehensive Heterocyclic Chemistry, Eds Katritzky, A. R., Rees, C. R., (4), 863-934, and Comprehensive Heterocyclic Chemistry (II), Eds Katritzky, A. R., Rees, C. W., Scriven, E. F. V., (2). 607-678.

Suitable starting materials include:

| Material | Reference | Supplier |
|---|---|---|
| Formamidine Hydrochloride | 26,860-7 | Aldrich |
| 2-Methyl Propionamidine | M23802 | Tyger |
| Acetamidine Hydrochloride | 15,915-8 | Aldrich |
| Trifluoroacetamidine | 12422 | Lancaster |
| 4-Fluoroacetophenone | F-320-7 | Aldrich |
| Acetophenone | A1070-1 | Aldrich |
| 2-Methylacetophenone | M2,659-3 | Aldrich |
| 3,4-Dimethylacetophenone | 13,723-5 | Aldrich |
| 4-Methylacetophenone | M2,661-5 | Aldrich |
| 2-Acetylpyridine | A2,100-2 | Aldrich |
| 2-Acetyl-4-methylpyridine | 49,923-4 | Aldrich |
| 3-Acetylpyridine | A2,120-7 | Aldrich |
| 3-Acetylthiophene | 19,632-0 | Aldrich |
| 2-Acetylthiophene | A2,260-2 | Aldrich |
| 2-Acetylfuran | A1,625-4 | Aldrich |
| Furfurylamine | F2,000-9 | Aldrich |
| Benzylamine | B1,630-5 | Aldrich |
| 2-(Aminomethyl)pyridine | A6,520-4 | Aldrich |
| 2-Pyridinemethanol | P6,660-2 | Aldrich |
| Propiophenone | P5,160-5 | Aldrich |
| N-Butyrophenone | 12,433-8 | Aldrich |
| 4-Chloro-5-(2-thienyl)thieno[2,3d]pyrimidine[2,3-d]pyrimidine | AW00007 | Maybridge |
| 4-Chloro-5-phenylthieno[2,3d]pyrimidine[2,3-d]pyrimidine | 30\08-39 | Buttpark |
| 4-Chloro-5-(4-chlorophenyl) thieno[2,3d]pyrimidine[2,3-d]pyrimidine | 17097 | Fluorochem |
| Ethyl-2-cyano-3-phenyl-2-butenoate | 39,875-6 | Aldrich |
| 4-Fluorophenylboronic acid | 41,755-6 | Aldrich |
| 4-Trifluoromethylphenylboronic acid | 43,932-0 | Aldrich |
| 3,4-Methylenedioxyphenylboronic acid | 49,999-4 | Aldrich |
| Phenylboronic acid | P2,000-9 | Aldrich |
| 4-Methoxyphenylboronic acid | M1,920-1 | Aldrich |
| 2-Amino-4-phenylthiophene-3-carboxamide | B014343 | Art-Chem-BB |
| 2-Amino-4-(4-fluorophenyl)-5-methylthiophene-3-carboxamide | B006163 | Art-Chem-BB |
| 2-Amino-5-methyl-4-phenylthiophene-3-carboxamide | B014344 | Art-Chem-BB |

As discussed herein, the compounds of the invention are useful in the treatment of various conditions. Thus, in a second aspect, the present invention provides a pharmaceutical formulation comprising at least one compound and optionally one or more excipients, carriers or diluents; wherein said compound has the formula: 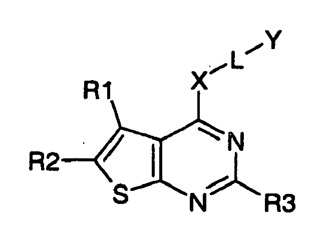 Wherein
R1 is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl;
R2 is H;C₁₋₆ alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰; nitro; -CO₂R7;CONR4R5; or halo;
R3 is H, NR4R5, NHC(O)R8, halo, trifluoromethyl, C₁₋₆alkyl which is unsubstituted or substituted with halogen, cyano, nitro, NR⁹NR¹⁰, alkoxyl, hydroxyl, unsubstituted aryl, unsubstituted heteroaryl, CO₂R7, C(O)NR⁹R¹⁰, NHC(O)R8 or SO₂NR⁹R¹⁰;nitrile or alkoxy;
R4 and R5 may be the same or different, and may be H, C₁₋₆ alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O) R8 or SO₂NR⁹R¹⁰; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁₋₆ alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl;, heterocyclic group which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; alkenyl; C₃-C₆ cycloalkyl; C₁₋₆ alkyl which is unsubstituted or substituted with halogen, cyano, nitro, NR9R10, alkoxy, unsubstituted aryl, unsubstituted heteroaryl, C(O)NR9R10, NHC(O)R8 or SO₂NR9R10;
R9 and R10 can be the same or different, and may be selected from H, unsubstituted C₁₋₆ alkyl, unsubstituted C₆-C₁₀ aryl, unsubstituted heteroaryl, unsubstituted cycloalkyl, aminoethyl, methylaminoethyl, dimethylaminoethyl, hydroxylethyl, alkoxyethyl, or R9 and R10 may together form a saturated or partially saturated 4-7 member ring, wherein said ring may optionally comprise one, two or three further heteroatoms
or pharmaceutically acceptable salts thereof;
with the proviso that when Y is phenyl, phenyl monosubstituted by Cl or methoxy, furanyl, tetrahydrofurayl, pyrimidinyl, pyrrolidinyl or 1,3-benzodioxolyl, then R1 is not phenyl, phenyl monosubstituted by halogen or phenyl substituted by methyl.

The compositions of the invention may be presented in unit dose forms containing a predetermined amount of each active ingredient per dose. Such a unit may be adapted to provide 5-1 00mg/day of the compound, preferably either 5-15mg/day, 10-30mg/day, 25-50mg/day 40-80mg/day or 60-100mglday. For compounds of formula I, doses in the range 100-1000mg/day are provided, preferably either 1 00-400mg/day, 300-600mg/day or 500-1000mg/day. Such doses can be provided in a single dose or as a number of discrete doses. The ultimate dose will depend on the condition being treated, the route of administration and the age, weight and condition of the patient and will be at the doctor's discretion.

The compositions of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For applications to the eye or other external tissues, for example the mouth and skin, the formulations are preferably applied as a topical ointment orcream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administration to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

Preferred unit dosage formulations are those containing a daily dose or sub-dose, as herein above recited, or an appropriate fraction thereof, of an active ingredient.

It should be understood that in addition to the ingredients particularly mentioned above, the formulations may also include other agents conventional in the art having regard to the type of formulation in question, for example those suitable for oral administration may include flavouring agents.

In a further aspect the present invention provides a compound, or a pharmaceutical composition comprising said compound for use in medicine, wherein said compound has the formula: 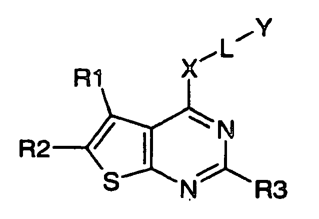 Wherein
R1 is C₃-C₆ cycloalkyl; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; or heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl;
R2 is H, nitro, -CO₂R7, CONR4R5, halo or C₁-₆alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, NHC(O)R8, halo, trifluoromethyl, nitrile, alkoxy, or C₁-₆ Alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R4 and R5 may be the same or different, and may be H; C₁-₆ alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O) R8 or SO₂NR⁹R¹⁰; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁-₆ alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is ethyl or methyl;
R9 and R10 can be the same or different, and may be selected from H, unsubstituted C₁-₆ alkyl, unsubstituted C₆-C₁₀ aryl, unsubstituted heteroaryl, unsubstituted cycloalkyl, aminoethyl, methylaminoethyl, dimethylaminoethyl, hydroxylethyl, alkoxyethyl, or R9 and R10 0 may together form a saturated or partially saturated 4-7 member ring, wherein said ring may optionally comprise one, two or three further heteroatoms
L is (CH₂)ₙ, where n is 1, 2 or 3; and
Y is alkenyl, C₃-C₆ cycloalkyl, C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heterocyclic group which is unsubstituted or substituted with cyano, nitro, halogen, alkyl, alkylthio, alkoxy, NR⁹R¹⁰, CO₂R7, C(O)NR⁹R¹⁰, NHC (O)R8, SO₂NR⁹R¹⁰ or hydroxyl; or C₁-₆ alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰,
or pharmaceutically acceptable salts thereof;
with the proviso that when Y is phenyl, phenyl monosubstituted by Cl or methoxy, furanyl, tetrahydrofurayl, pyrimidinyl, pyrrolidinyl or 1,3-benzodioxolyl, then R1 is not phenyl, phenyl monosubstituted by halogen or phenyl substituted by methyl.

The compositions of the invention can be used to treat conditions which require inhibition of potassium channels, for example in the treatment of arrhythmia. Thus the compositions can be used in a method of treating or preventing a disorder which requires potassium channel inhibition, eg arrhythmia, comprising administering to a subject an effective amount of at least one compound or of a pharmaceutical composition comprising said at least one compound and optionally one or more excipients, diluents and/or carriers wherein said compound has the formula: 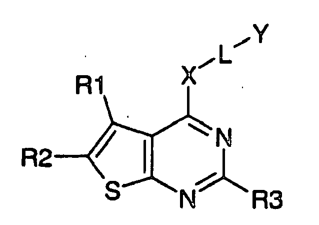 Wherein
R1 is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; C₃-C₆cycloalkyl; or C₁₋₆ alkyl which is unsubstituted orsubstituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R2 is H, nitro, -CO₂R7, CONR4R5, halo, or C₁₋₆alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, halo, trifluoromethyl, nitrile, alkoxy or C₁₋₆alkyl which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O) R8 or SO₂NR⁹R¹⁰;
R4 and R5 may be the same or different, and may be H; C₁₋₆ alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O) R8 or SO₂NR⁹R¹⁰; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl; or R4 and R5 may together form a saturated, unsaturated or partially saturated 4 to 7 member ring wherein said ring may optionally comprise one or more further heteroatoms selected from N, O or S;
X is O, S or NR6;
R6 is H or C₁₋₆ alkyl, which is unsubstituted or substituted with halogen, cyano, nitro, NR⁹NR'^{O}, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is alkenyl; C₃-C₆ cycloalkyl; C₆-C₁₀ aryl which is unsubstituted orsubstituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; a heterocyclic group which is unsubstituted or substituted with cyano, nitro, halogen, alkyl, alkylthio, alkoxy, NR⁹R¹⁰, CO₂R7, C(O)NR⁹R¹⁰, NHC (O)R8, SO₂NR⁹R¹⁰ or hydroxyl; or C₁₋₆ alkyl, which is unsubstituted orsubstituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8,
or pharmaceutically acceptable salts thereof.

Furthermore the present invention relates to the use of a compound of the invention in the manufacture of a medicament for use in potassium channel inhibition; wherein the compound has the formula: 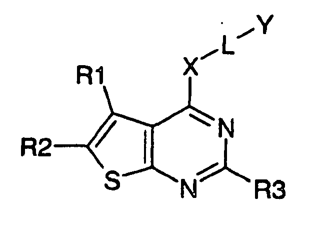 Wherein
R1 is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆cycloalkyl;
R2 is H, nitro, -CO₂R7, CONR4R5, halo or C₁₋₆alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, NHC(O)R8, halo, trifluoromethyl, nitrile, alkoxy or C₁-₆ alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R4 and R5 may be the same or different, and may be H; C₁-₆ alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O) R8 or SO₂NR⁹R¹⁰; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl or C₃-C₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁-₆alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is alkenyl; C₃-C₆ cycloalkyl; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl, an heterocyclic group which is unsubstituted or substituted with cyano, nitro, halogen, alkyl, alkylthio, alkoxy, NR⁹R¹⁰, CO₂R7, C(O)NR⁹R¹⁰, NHC (O)R8, SO₂NR⁹R¹⁰ or hydroxyl, cycloalkyl; or C₁₋₆ alkyl, which is unsubstituted or substituted with halogen, cyano, nitro, NR⁹NR¹⁰, alkoxyl, hydroxyl, unsubstituted aryl, unsubstituted heteroaryl, CO₂R⁷, C(O)NR⁹R¹⁰, NHC(O)R8 or SO₂NR⁹R¹⁰,
or pharmaceutically acceptable salts thereof.

### Examples

Using the information outlined herein the following compounds can be synthesised which are given by way of example only. The pharmacological profile of compounds of the present invention can readily be assessed by those skilled in the art using routine experimentation, such as procedures and techniques illustrated herein and described in detail in Ford et al., 2002.

### Example 1.

### 2-Cyano-3-phenyl-but-2-enoic acid ethyl ester

A stirred mixture of acetophenone (180g,1.5mol), ethyl cyanoacetate (170g, 1.3ml), ammonium acetate (23.1 g), acetic acid (72g) and toluene (300ml) was heated under reflux for 18 hours while water was removed from the reaction by azeotropic distillation. The mixture was allowed to cool to ambient temperature, toluene (100ml) was added, then the mixture was washed with water (3 x 100ml). The combined aqueous washings were shaken with toluene (50ml), then the combined toluene solutions were dried (MgSO₄) and the solvent was removed *in vacuo.* The residual oil was distilled under reduced pressure to give 2-cyano-3-phenyl-but-2-enoic acid ethyl ester as an oil which was used without further purification.

### Examples 2 to 18

The compounds set out below were prepared in the same way as in Example 1, using the appropriate starting materials.

| Example | Compound |
|---|---|
| 2 | 2-Cyano-3-(5-methyl-pyridin-2-yl)-but-2-enoicacid ethyl ester |
| 3 | 2-Cyano-3-(4-fluoro-phenyl)-but-2-enoic acid ethyl ester |
| 4 | 2-Cyano-3-thiophen-2-yl-but-2-enoic acid ethyl ester |
| 5 | 2-Cyano-3-methyl-but-2-enoic acid ethyl ester |
| 6 | 2-Cyano-3-p-tolyl-but-2-enoic acid ethyl ester |
| 7 | 3-(4-Chloro-phenyl)-2-cyano-but-2-enoic acid ethyl ester |
| 8 | 2-Cyano-3-(4-methoxy-phenyl)-but-2-enoic acid ethyl ester |
| 9 | 2-Cyano-3-phenyl-hex-2-enoic acid ethyl ester |
| 10 | 2-Cyano-3-o-tolyl-but-2-enoic acid ethyl ester |
| 11 | 2-Cyano-3-(3,4-dimethyl-phenyl)-but-2-enoic acid ethyl ester |
| 12 | 3-(4-Bromo-phenyl)-2-cyano-but-2-enoic acid ethyl ester |
| 13 | 2-Cyano-3-cyclohexyl-but-2-enoic acid ethyl ester |
| 14 | 3-(4-tert-Butyl-phenyl)-2-cyano-but-2-enoic acid ethyl ester |
| 15 | 2-Cyano-3-phenyl-pent-2-enoic acid ethyl ester |
| 16 | 3-Benzo[1,3]dioxol-5-yl-2-cyano-but-2-enoic acid ethyl ester |
| 17 | 3-Benzo[1,3]dioxol-5-yl-2-cyano-pent-2-enoic acid ethyl ester |
| 18 | 2-Cyano-3-(4-fluoro-phenyl)-pent-2-enoic acid ethyl ester |

### Example 19

### 2-Amino-4-phenyl-thiophene-3-carboxylic acid ethyl ester

2-Cyano-3-phenyl-but-2-enoic acid ethyl ester (513.25g, 2.3mol) was added at ambient temperature to a vigorously-stirred suspension of powdered sulfur (76g, 2.3mols) in ethanol (500ml). Diethylamine (200ml) was added in portions over 20 minutes, during which time the temperature of the reaction rose to 62°C. The mixture was allowed to cool to 36°C, then it was heated to 50°C and stirring at that temperature was continued for 1 hour. After this time, stirring was discontinued, the hot solution was removed by decantation from unreacted sulfur, then it was allowed to cool to ambient temperature. The resulting solid was collected by filtration, washed with a little cold ethanol and dried *in vacuo* to give 2-amino-4-phenylthiophene-3-carboxylic acid ethyl ester as an orange solid which was used without further purification.

### Examples 20 to 36

The compounds set out below were prepared in the same way as in Example 19, using the appropriate starting materials.

| Example | Compound |
|---|---|
| 20 | 2-Amino-4-(5-methyl-pyridin-2-yl)-thiophene-3-carboxylic acid ethyl ester |
| 21 | 2-Amino-4-(4-fluoro-phenyl)-thiophene-3-carboxylic acid ethyl ester |
| 22 | 5'-Amino-[2,3']bithiophenyl-4'-carboxylic acid ethyl ester |
| 23 | 2-Amino-4-methyl-thiophene-3-carboxylic acid ethyl ester |
| 24 | 2-Amino-4-p-tolyl-thiophene-3-carboxylic acid ethyl ester |
| 25 | 2-Amino-4-(4-chloro-phenyl)-thiophene-3-carboxylic acid ethyl ester |
| 26 | 2-Amino-4-(4-bromo-phenyl)-thiophene-3-carboxylic acid ethyl ester |
| 27 | 2-Amino-4-(4-methoxy-phenyl)-thiophene-3-carboxylic acid ethyl ester |
| 28 | 2-Amino-4-(3,4-dimethyl-phenyl)-thiophene-3-carboxylic acid ethyl ester |
| 29 | 2-Amino-4-o-tolyl-thiophene-3-carboxylic acid ethyl ester |
| 30 | 2-Amino-5-ethyl-4-phenyl-thiophene-3-carboxylic acid ethyl ester |
| 31 | 2-Amino-4-(4-tert-butyl-phenyl)-thiophene-3-carboxylic acid ethyl ester |
| 32 | 2-Amino-4-cyclohexyl-thiophene-3-carboxylic acid ethyl ester |
| 33 | 2-Amino-5-methyl-4-phenyl-thiophene-3-carboxylic acid ethyl ester |
| 34 | 2-Amino-4-benzo[1,3]dioxol-5-yl-thiophene-3-carboxylic acid ethyl ester |
| 35 | 2-Amino-4-benzo[1,3]dioxol-5-yl-5-methyl-thiophene-3-carboxylic acid ethyl ester |
| 36 | 2-Amino-4-(4-fluoro-phenyl)-5-methyl-thiophene-3-carboxylic acid ethyl ester |

### Example 37

### 5-Phenyl-3H-thleno[2,3-d]pyrimidin-4-one

A stirred mixture of 2-amino-4-phenylthiophene-3-carboxylic acid ethyl ester (350.43g, 1.535mol), formamidine acetate (799.13g, 7.7mol) and ethanol (1500ml) was heated under reflux for 18 hours then allowed to cool to ambient temperature. The resulting solid was collected by filtration, washed with a little cold ethanol, then crystallised from ethanol to give 5-Phenyl-3H-thieno[2,3-d]pyrimidin-4-one as a yellow solid which was used without further purification.

### Examples 38 to 54

The compounds set out below were prepared in the same way as in Example 37, using the appropriate starting materials.

| Example | Compound |
|---|---|
| 38 | 5-(5-Methyl-pyridin-2-yl)-3H-thieno[2,3-d]pyrimidin-4-one; |
| 39 | 5-(4-Fluoro-phenyl)-3H-thieno[2,3-d]pyrimidin-4-one; |
| 40 | 5-Thiophen-2-yl-3H-thieno[2,3-d]pyrimidin-4-one; |
| 41 | 5-Methyl-3H-thieno[2,3-d]pyrimidin-4-one; |
| 42 | 5-p-Tolyl-3H-thieno[2,3-d]pyrimidin-4-one; |
| 43 | 5-(4-Chloro-phenyl)-3H-thleno[2,3-d]pyrimidin-4-one; |
| 44 | 5-(4-Bromo-phenyl)-3H-thieno[2,3-d]pyrimidin-4-one; |
| 45 | 5-(4-Methoxy-phenyl)-3H-thieno[2,3-d]pyrimidin-4-one; |
| 46 | 5-(3,4-Dimethyl-phenyl)-3H-thieno[2,3-d]pyrimidin-4-one; |
| 47 | 5-(4-tert-Butyl-phenyl)-3H-thieno[2,3-d]pyrimidin-4-one; |
| 48 | 6-Ethyl-5-phenyl-3H-thieno[2,3-d]pyrimidin-4-one; |
| 49 | 5-Cyclohexyl-3H-thieno[2,3-d]pyrimidin-4-one; |
| 50 | 5-o-Tolyl-3H-thieno[2,3-d]pyrimidin-4-one; |
| 51 | 6-Methyl-5-phenyl-3H-thieno[2,3-d]pyrimidin-4-one |
| 52 | 5-Benzo[1,3]dioxol-5-yl-3H-thieno[2,3-d]pyrimidin-4-one |
| 53 | 5-Benzo[1,3]dioxol-5-yl-6-methyl-3H-thieno[2,3-d]pyrimidin-4-one |
| 54 | 5-(4-Fluoro-phenyl)-6-methyl-3H-thieno[2,3-d]pyrimidin-4-one |

### Example 55

### 2-Methyl-5-phenyl-3H-thieno[2,3-d]pyrimidin-4-one

A stirred mixture of 2-amino-4-phenylthiophene-3-carboxylic acid ethyl ester (350.43g, 1.54ml), acetamidine hydrochloride (725.13g, 7.676mol) and ethanol (1500ml) was heated under reflux for 18 hours then allowed to cool to ambient temperature. The resulting solid was collected by filtration, washed with a little cold ethanol, then crystallised from ethanol to give 2-Methyl-5-phenyl-3H-thieno[2,3-d]pyrimidin-4-one as a yellow solid which was used without further purification.

### Example 56

The compound set out below was prepared in the same way as in Example 55, using the appropriate starting materials.

| Example | Compound |
|---|---|
| 56 | 5-(4-Fluoro-phenyl)-2-methyl-3H-thieno[2,3-d]pyrimidin-4-one |

### Example 57

### 4-phenyl-2-propionylaminothiophene-3-carboxylic amide

A mixture of 2-amino-4-phenylthiophene-3-carboxylic acid amide (3.0g, 13.8mmol) and anhydrous pyridine (12ml) was treated with propionyl chloride (1.32ml), 15.2mmol) and stirred at ambient temperature for 3 hours. The excess pyridine was removed *in vacuo* to give a residue, which was treated with ethanol (50ml) and sodium methoxide (2.24g, 41.4mmol) and the resultant mixture was heated under reflux for 18 hours. The cooled mixture was then diluted with water (300ml) and acidified with concentrated hydrochloric acid. The resulting solid was collected by filtration, washed with water and dried *in vacuo* to give 4-phenyl-2-propionylaminothiophene-3-carboxylic amide.

### Example 58

### 2-ethyl-5-phenyl-3H-thieno[2,3-d]pyrimidin-4-one

The solid 4-phenyl-2-propionylaminothiophene-3-carboxylic amide was added to a mixture of ethanol (50ml) and sodium methoxide (2.24g) and the resultant mixture was heated under reflux with stirring for 18 hours. The cooled mixture was diluted with water (300ml) and acidified with concentrated hydrochloric acid. The resulting solid was collected by filtration, washed with water followed by acetonitrile and dried *in vacuo* to give 2-ethyl-5-phenyl-3*H-*thieno[2,3*-d*] pyrimidin-4-one (2.38g), which was used without further purification.

### Example 59

### 5-Phenyl-2-trifluoromethyl-3H-thieno[2,3-d]pyrimidin-4-one

A stirred mixture of 2-amino-4-phenylthiophene-3-carboxylic acid ethyl ester (350.43g, 1.535mol), trifluoromethylacetamidine hydrochloride (1167.36g, 7.676mol) and ethanol (1500ml) was heated under reflux for 18 hours then allowed to cool to ambient temperature. The resulting solid was collected by filtration, washed with a little cold ethanol, then crystallised from ethanol to give 5-Phenyl-2-trifluoromethyl-3H-thieno[2,3-d]pyrimidin-4-one as a yellow solid which was used without further purification.

### Example 60

### 2-Isopropyl-5-phenyl-3H-thieno[2,3-d]pyrimidin-4-one

A stirred mixture of 2-amino-4-phenylthiophene-3-carboxylic acid ethyl ester (350.43g, 1.54mol), isopropylacetamidine hydrochloride (958.75g, 7.7mol) and ethanol (1500ml) was heated under reflux for 18 hours then allowed to cool to ambient temperature. The resulting solid was collected by filtration, washed with a little cold ethanol, then crystallised from ethanol to give 2-Isopropyl-5-phenyl-3H-thieno[2,3-d]pyrimidin-4-one as a yellow solid which was used without further purification.

### Example 61

### 4-Chloro-5-phenyl-thieno[2,3-d]pyrimidine

5-Phenyl-3H-thieno[2,3-d]pyrimidin-4-one (294.6g, 1.29mol) was added in portions to stirred phosphoryl chloride (1000ml), then the stirred suspension was warmed gently to reflux temperature, heated under reflux for 4 hours, and allowed to stand at ambient temperature for 18 hours. The resulting dark solution was removed by decantation from a solid residue and concentrated *in vacuo* to give a gummy solid. The two solids were combined and added to crushed ice (1000ml). The product was extracted into dichloromethane (3 x 500ml), then the combined extracts were washed with water (2 x 300ml) and saturated aqueous sodium hydrogen carbonate solution (500ml) and dried and decolourised (MgSO₄ + charcoal). The solvent was removed *in vacuo* to give 4-Chloro-5-phenyl-thieno[2,3-d]pyrimidine as a yellow/orange solid which was used without further purification.

### Examples 62 to 82

The compounds set out below were prepared in the same way as in Example 61, using the appropriate starting materials.

| Example | Compound |
|---|---|
| 62 | 4-Chloro-5-(5-methyl-pyridin-2-yl)-thieno[2,3-d]pyrimidine |
| 63 | 4-Chloro-5-(4-fluoro-phenyl)-thieno[2,3-d]pyrimidine |
| 64 | 4-Chloro-5-thiophen-2-yl-thieno[2,3-d]pyrimidine |
| 65 | 4-Chloro-5-methyl-thieno[2,3-d]pyrimidine |
| 66 | 4-Chloro-5-p-tolyl-thieno[2,3-d]pyrimidine |
| 67 | 4-Chloro-5-(3,4-dimethyl-phenyl)-thieno[2,3-d]pyrimidine |
| 68 | 4-Chloro-5-(4-chloro-phenyl)-thieno[2,3-d]pyrimidine |
| 69 | 5-(4-Bromo-phenyl)-4-chloro-thieno[2,3-d]pyrimidine |
| 70 | 4-Chloro-5-(4-methoxy-phenyl)-thieno[2,3-d]pyrimidine |
| 71 | 4-Chloro-6-ethyl-5-phenyl-thieno[2,3-d]pyrimidine |
| 72 | 4-Chloro-5-o-tolyl-thieno[2,3-d]pyrimidine |
| 73 | 5-(4-tert-Butyl-phenyl)-4-chloro-thieno[2,3-d]pyrimidine |
| 74 | 4-Chloro-5-cyclohexyl-thieno[2,3-d]pyrimidine |
| 75 | 4-Chloro-2-methyl-5-phenyl-thieno[2,3-d]pyrimidine |
| 76 | 4-Chloro-5-phenyl-2-trifluoromethyl-thieno[2,3-d]pyrimidine |
| 77 | 4-Chloro-2-isopropyl-5-phenyl-thieno[2,3-d]pyrimidine |
| 78 | 4-Chloro-2-ethyl-5-phenyl-thieno[2,3-d]pyrimidine |
| 79 | 4-Chloro-6-methyl-5-phenyl-thieno[2,3-d]pyrimidine |
| 80 | 4-Chloro-5-(4-fluoro-phenyl)-2-methyl-thieno[2,3-d]pyrimidine |
| 81 | 5-(1,3-Benzodioxol-5-yl)-4-chloro-6-methylthieno[2,3-d]pyrimidine |
| 82 | 4-Chloro-5-(4-fluorophenyl)-6-methylthieno[2,3-d]pyrimidine |

### Example 83

### Furan-2-ylmethyl-(5-phenyl-thieno[2,3-d]pyrimidin-4-yl)-amine

A stirred mixture of 4-chloro-5-phenylthieno[2,3-d]pyrimidine (2.2g, 0.009ml), furfurylamine (1.26g, 0.013mol), triethylamine (1.3g) and ethanol (20ml) was heated under reflux for 2 hours then cooled to ambient temperature and poured into water (50ml). The resulting solid was collected by filtration, washed with water (30ml), dried *in vacuo* and crystallised from a mixture of hexane and toluene to give furan-2-ylmethyl-(5-phenyl-thieno[2,3-d]pyrimidin-4-yl)-amine as a pale yellow solid, m.pt. 77-79°C.

### Examples 84 to 100

The compounds set out below were prepared in the same way as in Example 83, using the appropriate starting materials.

| Example | Compound |
|---|---|
| 84 | Cyclopropylmethyl-(5-phenyl-thieno[2,3-d]pyrimidin-4-yl)-amine |
| 85 | (5-Phenyl-thieno[2,3-d]pyrimidin-4-yl)-thiophen-2-ylmethyl-amine |
| 86 | Benzyl-(5-phenyl-thieno[2,3-d]pyrimidin-4-yl)-amine |
| 87 | Allyl-(5-phenyl-thieno[2,3-d]pyrimidin-4-yl)-amine |
| 88 | Furan-2-ylmethyl-methyl-(5-phenyl-thieno[2,3-d]pyrimidin-4-yl)-amine |
| 89 | Cyclohexylmethyl-(5-phenyl-thieno[2,3-d]pyrimidin-4-yl)-amine |
| 90 | Phenethyl-(5-phenyl-thieno[2,3-d]pyrimidin-4-yl)-amine |
| 91 | Cyclohexyl-(5-phenyl-thieno[2,3-d]pyrimidin-4-yl)-amine |
| 92 | Furan-2-ylmethyl-(5-thiophen-2-yl-thieno[2,3-d]pyrimidin-4-yl)-amine |
| 93 | (4-Nitro-benzyl)-(5-thiophen-2-yl-thieno[2,3-d]pyrimidin-4-yl)-amine |
| 94 | (5-Thiophen-2-yl-thieno[2,3-d]pyrimidin-4-yl)-(3,4,5-trimethoxy-benzyl)-amine |
| 95 | Cyclopropylmethyl-(5-thiophen-2-yl-thieno[2,3-d]pyrimidin-4-yl)-amine |
| 96 | Isobutyl-(5-thiophen-2-yl-thieno[2,3-d]pyrimidin-4-yl)-amine |
| 97 | Benzyl-(5-thiophen-2-yl-thieno[2,3-d]pyrimidin-4-yl)-amine |
| 98 | Thiophen-2-ylmethyl-(5-thiophen-2-yl-thieno[2,3-d]pyrimidin-4-yl)-amine |
| 99 | Allyl-(5-thiophen-2-yl-thieno[2,3-d]pyrimidin-4-yl)-amine |
| 100 | Furan-2-ylmethyl-methyl-(5-thiophen-2-yl-thieno[2,3-d]pyrimidin-4-yl)-amine |

### Example 101

### 2-Methyl-N-(2-pyridyl)methyl-5-phenylthieno[2,3-d]pyrimidin-4-ylamine

In a 10 ml glass tube were placed 2-methyl-4-chloro-5-phenylthieno[2,3-d]pyrimidin-4-ylamine (0.076g, 0.293 mmol), 2-(aminomethyl)pyridine (0.0364g, 0.03mmol) and ethanol (2.5ml). The vessel was sealed with a septum and placed in the microwave cavity. Microwave irradiation of 200W was used, the temperature being ramped from room temperature to 150°C. Once 150°C was reached, the reaction mixture was held at this temperature for 10 minutes. After cooling to ambient temperature, water (4ml) was added and the mixture stirred for 2.5 hours. The resulting solid was collected by filtration, washed with water and dried in vacuo to give 2-methyl-N-(2-pyridyl)methyl-5-phenylthieno[2,3-d] pyrimidin-4-ylamine (0.084g) as an off white solid, m.pt. 110-112°C.

### Examples 102 to 167

The compounds set out below were prepared in the same way as in Example 101, using the appropriate starting materials.

| Example | Compound |
|---|---|
| 102 | (1-Ethyl-pyrrolidin-2-ylmethyl)-(5-phenyl-thieno[2,3-d]pyrimidin-4-yl)-amine |
| 103 | N,N-Dimethyl-2-(5-phenyl-thieno[2,3-d]pyrimidin-4-ylamino)-acetamide |
| 104 | (3-Imidazol-1-yl-propyl)-(5-phenyl-thieno[2,3-d]pyrimidin-4-yl)-amine |
| 105 | (5-Phenyl-thieno[2,3-d]pyrimidin-4-yl)-pyridin-2-ylmethyl-amine |
| 106 | 5-Phenyl-4-(pyridin-2-ylmethoxy)-thieno[2,3-d]pyrimidine |
| 107 | Furan-3-ylmethyl-(5-phenyl-thieno[2,3-d]pyrimidin-4-yl)-amine |
| 108 | (5-Methyl-furan-2-ylmethyl)-(5-phenyl-thieno[2,3-d]pyrimidin-4-yl)-amine |
| 109 | 4-(Furan-2-ylmethoxy)-5-phenyl-thieno[2,3-d]pyrimidine |
| 110 | (1-Methyl-1H-pyrrol-2-ylmethyl)-(5-phenyl-thieno[2,3-d]pyrimidin-4-yl)-amine |
| 111 | (5-Phenyl-thieno[2,3-d]pyrimidin-4-yl)-(2-pyridin-2-yl-ethyl)-amine |
| 112 | (5-Phenyl-thieno[2,3-d]pyrimidin-4-ylamino)-acetic acid methyl ester |
| 113 | (2-Methoxy-ethyl)-(5-phenyl-thieno[2,3-d]pyrimidin-4-yl)-amine |
| 114 | (5-Phenyl-thieno[2,3-d]pyrimidin-4-yl)-(tetrahydro-furan-2-ylmethyl)-amine |
| 115 | Thiophen-2-ylmethyl-(5-thiophen-2-yl-thieno[2,3-d]pyrimidin-4-yl)-amine |
| 116 | [1,3]Dioxolan-2-ylmethyl-(5-phenyl-thieno[2,3-d]pyrimidin-4-yl)-amine |
| 117 | 4-(Furan-2-ylmethylsulfanyl)-5-phenyl-thieno[2,3-d]pyrimidine |
| 118 | (5-Phenyl-thieno[2,3-d]pyrimidin-4-yl)-(2-thiophen-2-yl-ethyl)-amine |
| 119 | 4-Benzyloxy-5-phenyl-thieno[2,3-d]pyrimidine |
| 120 | 5-Phenyl-4-(pyridin-2-ylmethylsulfanyl)-thieno[2,3-d]pyrimidine |
| 121 | [5-(5-Methyl-pyridin-2-yl)-thieno[2,3-d]pyrimidin-4-yl]-pyridin-2-ylmethyl-amine |
| 122 | Furan-2-ylmethyl-[5-(5-methyl-pyridin-2-yl)-thieno[2,3-d]pyrimidin-4-yl]-amine |
| 123 | [5-(4-Fluoro-phenyl)-thieno[2,3-d]pyrimidin-4-yl]-pyridin-2-ylmethyl-amine |
| 124 | [5-(4-Fluoro-phenyl)-thieno[2,3-d]pyrimidin-4-yl]-furan-2-ylmethyl-amine |
| 125 | [2-(4-Chloro-phenyl)-ethyl]-(5-methyl-thieno[2,3-d]pyrimidin-4-yl)-amine |
| 126 | Furan-2-ylmethyl-(5-p-tolyl-thieno[2,3-d]pyrimidin-4-yl)-amine |
| 127 | [2-(3,4-Dimethoxy-phenyl)-ethyl]-(5-p-tolyl-thieno[2,3-d]pyrimidin-4-yl)-amine |
| 128 | 1-[2-(5-p-Tolyl-thieno[2,3-d]pyrimidin-4-ylamino)-ethyl]-imidazolidin-2-one |
| 129 | 4-(Naphthalen-2-yloxy)-5-p-tolyl-thieno[2,3-d]pyrimidine |
| 130 | Phenethyl-(5-p-tolyl-thieno[2,3-d]pyrimidin-4-yl)-amine |
| 131 | 4-(5-p-Tolyl-thieno[2,3-d]pyrimidin-4-yloxy)-benzoic acid methyl ester |
| 132 | [2-(2,3-Dihydro-benzo[1,4]dioxin-6-yl)-ethyl]-[5-(3,4-dimethyl-phenyl)-thieno[2,3-d]pyrimidin-4-yl]-amine |
| 133 | [5-(3,4-Dimethyl-phenyl)-thieno[2,3-d]pyrimidin-4-yl]-furan-2-ylmethyl-amine |
| 134 | 4-(4-tert-Butyl-phenoxy)-5-(4-chloro-phenyl)-thieno[2,3-d]pyrimidine |
| 135 | [5-(4-Chloro-phenyl)-thieno[2,3-d]pyrimidin-4-yl]-furan-2-ylmethyl-amine |
| 136 | [5-(4-Chloro-phenyl)-thieno[2,3-d]pyrimidin-4-yl]-phenethyl-amine |
| 137 | [5-(4-Bromo-phenyl)-thieno[2,3-d]pyrimidin-4-yl]-[2-(2,3-dihydro-benzo[1,4]dioxin-6-yl)-ethyl]-amine |
| 138 | [5-(4-Methoxy-phenyl)-thieno[2,3-d]pyrimidin-4-yl]-pyridin-2-ylmethyl-amine |
| 139 | Furan-2-ylmethyl-[5-(4-methoxy-phenyl)-thieno[2,3-d]pyrimidin-4-yl]-amine |
| 140 | (6-Ethyl-5-phenyl-thieno[2,3-d]pyrimidin-4-yl)-furan-2-ylmethyl-amine |
| 141 | (6-Ethyl-5-furan-3-yl-thieno[2,3-d]pyrimidin-4-yl)-furan-2-ylmethyl-amine |
| 142 | Pyridin-2-ylmethyl-(5-o-tolyl-thieno[2,3-d]pyrimidin-4-yl)-amine |
| 143 | Furan-2-ylmethyl-(5-o-tolyl-thieno[2,3-d]pyrimidin-4-yl)-amine |
| 144 | [5-(4-tert-Butyl-phenyl)-thieno[2,3-d]pyrimidin-4-yl]-pyridin-2-ylmethyl-amine |
| 145 | [5-(4-tert-Butyl-phenyl)-thieno[2,3-d]pyrimidin-4-yl]-furan-2-ylmethyl-amine |
| 146 | (5-Cyclohexyl-thieno[2,3-d]pyrimidin-4-yl)-pyridin-2-ylmethyl-amine |
| 147 | (5-Cyclohexyl-thieno[2,3-d]pyrimidin-4-yl)-furan-2-ylmethyl-amine |
| 148 | (2-Isopropyl-5-phenyl-thieno[2,3-d]pyrimidin-4-yl)-pyridin-2-ylmethyl-amine |
| 149 | (5-Phenyl-thieno[2,3-d]pyrimidin-4-yl)-(1-pyridin-2-yl-ethyl)-amine |
| 150 | Furan-2-ylmethyl-(2-methyl-5-phenyl-thieno[2,3-d]pyrimidin-4-yl)-amine |
| 151 | (5-Phenyl-2-trifluoromethyl-thieno[2,3-d]pyrimidin-4-yl)-pyridin-2-ylmethyl-amine |
| 152 | (2-Ethyl-5-phenyl-thieno[2,3-d]pyrimidin-4-yl)-pyridin-2-ylmethyl-amine |
| 153 | (2-Ethyl-5-phenyl-thieno[2,3-d]pyrimidin-4-yl)-furan-2-ylmethyl-amine |
| 154 | (6-Methylpyridin-2-ylmethyl)-(5-phenyl-thieno[2,3-d]pyrimidin-4-yl)-amine |
| 155 | [5-(4-Fluorophenyl)-2-methyl-thieno[2,3-d]pyrimidin-4-yl]-pyridin-2-ylmethyl-amine |
| 156 | (3-methyl-pyridin-2-ylmethyl)-(5-phenyl-thieno[2,3-d]pyrimidin-4-yl)-amine |
| 157 | (6-Methyl-5-phenyl-thieno[2,3-d]pyrimidin-4-yl)-pyridin-2-ylmethyl-amine |
| 158 | (5-Phenyl-thieno[2,3-d]pyrimidin-4-yl)-(1,2,3,4-tetrahydro-naphthalen-1-yl)-amine |
| 159 | 6-Methyl-5-phenyl-4-piperidin-1-yl-thieno[2,3-d]pyrimidine |
| 160 | 2-[(5-Phenyl-thieno[2,3-d]pyrimidin-4-ylamino)-methyl]-nicotinic acid ethyl ester |
| 161 | (5-Phenyl-thieno[2,3-d]pyrimidin-4-yl)-(2-thiophen-2-yl-thiazol-4-ylmethyl)-amine |
| 162 | (2-Phenyl-thiazol-4-ylmethyl)-(5-phenyl-thieno[2,3-d]pyrimidin-4-yl)-amine |
| 163 | Phenethyl-(5-thiophen-2-yl-thieno[2,3-d]pyrimidin-4-yl)-amine |
| 164 | (6-Bromo-5-phenyl-thieno[2,3-d]pyrimidin-4-yl)-pyrdin-2-ylmethyl-amine |
| 165 | (6-Bromo-5-phenyl-thieno[2,3-d]pyrimidin-4-yl)-furan-2-ylmethyl-amine |
| 166 | 6-methyl-N-[(6-methylpyridin-2-yl)methyl]-5-phenylthieno[2,3-d]pyrimidin-4-amine |
| 167 | 6-bromo-N-[(6-methylpyridin-2-yl)methyl]-5-phenylthieno[2,3-d]pyrimidin-4-amine |

### Example 168

### 5-Phenyl-1,3H-thieno[2,3-d]pyrimidine-2,4-dione

A mixture of ethyl 2-amino-4-phenyl-thiophene-3-carboxylate (2.0g, 8.1 mmol) and potassium cyanate (2.0g, 24.3 mmol) in glacial acetic acid (20ml) was stirred at ambient temperature for 72 hours. The resultant solid material was filtered off. The filtrate was diluted with water (50ml) and the precipitated solid was filtered off. The two solids were combined, suspended in water (100ml) and made alkaline by the addition of concentrated sodium hydroxide solution. The resultant suspension was heated at 100°C for 2 hours with stirring, then allowed to cool to ambient temperature and acidified by the addition of glacial acetic acid. The resulting solid was collected by filtration to give 5-phenyl-1,3*H-*thieno[2,3*-d*]pyrimidin-2,4-dione (1.1 g) as a white solid which was used without further purification.

### Examples 169 to 174

The compounds set out below were prepared in the same way as in Example 168, using the appropriate starting materials.

| Example | Compound |
|---|---|
| 169 | 5-(4-Fluorophenyl)-1,3H-thieno[2,3-d]pyrimidine-2,4-dione |
| 170 | 6-Methyl-1,3H-thieno[2,3-d]pyrimidine-2,4-dione |
| 171 | 5-Benzo[1,3]dioxol-5-yl-1,3H-thieno[2,3-d]pyrimidine-2,4-dione |
| 172 | 5-(1,3-Benzodioxol-5-yl)-6-methyl-1,3H-thieno[2,3-d]pyrimidine-2,4-dione |
| 173 | 5-(4-Fluorophenyl)-6-methyl-1,3H-thieno[2,3-d]pyrimidine-2,4-dione |
| 174 | 6-Methyl-5-phenyl-1,3H-thieno[2,3-d]pyrimidine-2,4-dione |

### Example 175

### 2,4-Dichloro-5-phenyl-thieno[2,3-d]pyrimidine

A stirred mixture of 5-phenyl-1,3*H-*thieno[2,3*-d*]pyrimidin-2,4-dione (1.07g, 4.39 mmol) and phenylphosphonic dichloride (10ml) was heated at 150°C for 7 hours then allowed to stand at ambient temperature for 18 hours. The resulting dark solution was poured into ice-water and extracted with dichloromethane (3 x 150ml). The combined extracts were washed with saturated sodium hydrogen carbonate solution (150ml) and dried (MgSO₄). The solvent was removed *in vacuo* and the oily residue triturated with 40-60°C petrol to give 2,4*-*dichloro-5-phenyl-thieno[2,3-*d*]pyrimidine (0.82g) as a pale yellow solid which was used without further purification.

### Examples 176 to 180

The compounds set out below were prepared in the same way as in Example 175, using the appropriate starting materials.

| Example | Compound |
|---|---|
| 176 | 2,4-Dichloro-5-(4-fluoro-phenyl)-thieno[2,3-d]pyrimidine |
| 177 | 5-Benzo[1,3]dioxol-5-yl-2,4-dichloro-thieno[2,3-d]pyrimidine |
| 178 | 2,4-Dichloro-6-methyl-5-phenylthieno[2,3-d]pyrimidine |
| 179 | 2,4-Dichloro-5-(4-fluorophenyl)-6-methylthieno[2,3-d]pyrimidine |
| 180 | 5-(1,3-Benzodioxol-5-yl)-2,4-dichloro-6-methylthieno[2,3-d]pyrimidine |

### Example 181

### (2-Chloro-5-phenyl-thieno[2,3-d]pyrimidin-4-yl)-pyridin-2-ylmethyl-amine

A mixture of 2,4-dichloro-5-phenyl-thieno[2,3-*d*]pyrimidine (0.82g, 2.92 mmol), 2-(aminomethyl)pyridine (0.35g, 3.21 mmol), triethylamine (0.32g, 0.45ml, 3.21 mmol) and propan-2-ol (20 ml) was stirred at ambient temperature for 72 hours. Water (50ml) was added to the reaction mixture and the organic phase was extracted using dichloromethane (3 x 50ml). The combined extracts were washed with water and dried (MgSO₄). The solvent was removed *in vacuo* to give 2-chloro-*N*-(2-pyridyl)methyl-5-phenylthieno[2,3-*d*]pyrimidin-4-ylamine (1.0g) as a white solid which was used without further purification.

### Examples 182 to 187

The compounds set out below were prepared in the same way as in Example 181, using the appropriate starting materials.

| Example | Compound |
|---|---|
| 182 | (2-Chloro-5-phenyl-thieno[2,3-d]pyrimidin-4-yl)-furan-2-ylmethyl-amine; |
| 183 | [2-Chloro-5-(4-fluorophenyl)-thieno[2,3-d]pyrimidin-4-yl]-pyridin-2-ylmethyl-amine |
| 184 | (2-Chloro-5-phenyl-thieno[2,3-d]pyrimidin-4-yl)-(6-methyl-pyridin-2-yl methyl)-amine |
| 185 | [2-Chloro-5-(4-fluorophenyl)-thieno[2,3-d]pyrimidin-4-yl]-(6-methyl-pyridin-2-ylmethyl)-amine |
| 186 | (5-Benzo[1,3]dioxol-5-yl-2-chloro-thieno[2,3-d]pyrimidin-4-yl)-(6-meth yl-pyridin-2-ylmethyl)-amine |
| 187 | (5-Benzo[1,3]dioxol-5-yl-2-chloro-thieno[2,3-d]pyrimidin-4-yl)-pyridin-2-ylmethyl-amine |

### Example 188

### N²-Cyclopropylmethyl-5-phenyl-N⁴-pyridin-2-ylmethyl-thieno[2,3-d]pyrimidine-2,4-diamine

In a 10 ml glass tube were placed 2-chloro-*N*-(2-pyridyl)methyl-5-phenylthieno[2,3-*d*]pyrimidin-4-ylamine (0.03g, 0.0852 mmol) and cyclopropylmethylamine (0.5ml). The vessel was sealed with a septum and placed in the microwave cavity. Microwave irradiation of 200 W was used, the temperature being ramped from room temperature to 200°C. Once 200°C was reached, the reaction mixture was held at this temperature for 40 minutes. After cooling to ambient temperature, water (4ml) was added and the mixture stirred for 2.5 hours. The resulting solid was collected by filtration, washed with water and dried under reduced pressure to give N²-Cyclopropylmethyl-5-phenyl-N⁴-pyridin-2-ylmethyl-thieno[2,3-d]pyrimidine-2,4-diamine (0.025g) as a yellow solid, m.p.t. 109-111°C.

### Examples 189 to 224

The compounds set out below were prepared in the same way as in Example 188, using the appropriate starting materials.

| Example | Compound |
|---|---|
| 189 | N²-(2-Methoxyethyl)-5-phenyl-N⁴-(pyridin-2-ylmethyl)thieno[2,3-d]pyrimidine-2,4-diamine |
| 190 | 2-{5-Phenyl-4-[(pyridin-2-ylmethyl)-amino]-thieno[2,3-d]pyrimidin-2-ylamino }-ethanol |
| 191 | (2-Methoxy-5-phenyl-thieno[2,3-d]pyrimidin-4-yl)-pyridin-2-ylmethyl-amine |
| 192 | 5-Phenyl-N-4-pyridin-2-ylmethyl-thieno[2,3-d]pyrimidine-2,4-diamine |
| 193 | N,N'-Bis(2-furylmethyl)-5-phenylthieno[2,3-d]pyrimidine-2,4-diamine |
| 194 | 5-(3,4-Dimethylphenyl)-N-(2-furylmethyl)thieno[2,3-d]pyrimidin-4-amine |
| 195 | (2-Benzyloxy-5-phenyl-thieno[2,3-d]pyrimidin-4-yl)-furan-2-ylmethyl-amine |
| 196 | N²-methyl-5-phenyl-N⁴-(pyridin-2-ylmethyl)thieno[2,3-d]pyrimidine-2,4-diamine |
| 197 | (2-Morpholin-4-yl-5-phenyl-thieno[2,3-d]pyrimidin-4-yl)-pyridin-2-ylmethyl-amine |
| 198 | N²,N²-Dimethyl-5-phenyl-N⁴-pyridin-2-ylmethyl-thieno[2,3-d]pyrimidine-2,4-diamine |
| 199 | 5-Phenyl-4-[(pyridin-2-ylmethyl)-amino]-thieno[2,3-d]pyrimidine-2-carbonitrile |
| 200 | 5-(4-Fluorophenyl)-N²,N²-dimethyl-N⁴-pyridin-2-ylmethyl-thieno[2,3-d]pyrimidine-2,4-diamine |
| 201 | 1-{5-(4-Fluorophenyl)-4-[(pyridin-2-ylmethyl)-amino]-thieno[2,3-d]pyrimidin-2-yl}-piperidine-4-carboxylic acid methyl ester |
| 202 | 3-{5-Phenyl-4-[(pyridin-2-ylmethyl)-amino]-thieno[2,3-d]pyrimidin-2-ylamino}-propionic acid ethyl ester |
| 203 | [2-(2-Methoxy-ethoxy)-5-phenyl-thieno[2,3-d]pyrimidin-4-yl]-pyridin-2-ylmethyl-amine |
| 204 | 5-(4-Fluorophenyl)-N²-methyl-N⁴-pyridin-2-ylmethyl-thieno[2,3-d]pyrimidine-2,4-diamine |
| 205 | 5-(4-Fluorophenyl)-N⁴-pyridin-2-ylmethyl-thieno[2,3-d]pyrimidine-2,4-diamine |
| 206 | 2-(1-{5-Phenyl-4-[(pyridin-2-ylmethyl)-amino]-thieno[2,3-d]pyrimidin-2-yl}-piperidin-4-yl)-ethanol |
| 207 | [5-(4-Fluorophenyl)-2-morpholin-4-yl-thieno[2,3-d]pyrimidin-4-yl]-pyridin-2-ylmethyl-amine |
| 208 | 2-((2-Hydroxy-ethyl)-{5-phenyl-4-[(pyridin-2-ylmethyl)-amino]-thieno[2,3-d]pyrimidin-2-yl}-amino)-ethanol |
| 209 | 5-(4-Fluorophenyl)-N²-(2-methoxy-ethyl)-N⁴-pyridin-2-ylmethyl-thieno[2,3-d]pyrimidine-2,4-diamine |
| 210 | 2-{5-Phenyl-4-[(pyridin-2-ylmethyl)-amino]-thieno[2,3-d]pyrimidin-2-ylamino}-propane-1,3-diol |
| 211 | [2-(2-Dimethylamino-ethoxy)-5-phenyl-thieno[2,3-d]pyrimidin-4-yl]-pyridin-2-ylmethyl-amine |
| 212 | 2-{5-(4-Fluorophenyl)-4-[(pyridin-2-ylmethyl)-amino]-thieno[2,3-d]pyrimidin-2-ylamino-ethanol |
| 213 | 2-{4-[(6-Methyl-pyridin-2-ylmethyl)-amino]-5-phenyl-thieno[2,3-d]pyrimidin-2-ylamino}-ethanol |
| 214 | 3-{5-Phenyl-4-[(pyridin-2-ylmethyl)-amino]-thieno[2,3-d]pyrimidin-2-ylamino}-propane-1,2-diol |
| 215 | [2-(4-Methyl-piperazin-1-yl)-5-phenyl-thieno[2,3-d]pyrimidin-4-yl]-pyridin-2-ylmethyl-amine |
| 216 | 2-{4-[(6-Methyl-pyridin-2-ylmethyl)-amino]-5-phenyl-thieno[2,3-d]pyrimidin-2-ylamino}-ethanol |
| 217 | 2-((2-Hydroxy-ethyl)-{4-[(6-methyl-pyridin-2-ylmethyl)-amino]-5-phenyl-thieno[2,3-d]pyrimidin-2-yl}-amino)-ethanol |
| 218 | 2-{5-(4-Fluorophenyl)-4-[(6-methyl-pyridin-2-ylmethyl)-amino]-thieno[2,3-d]pyrimidin-2-ylamino}-ethanol |
| 219 | 2-[{5-(4-Fluorophenyl)-4-[(pyridin-2-ylmethyl)-amino]-thieno[2,3-d]pyrimidin-2-yl}-(2-hydroxy-ethyl)-amino]-ethanol |
| 220 | 2-[{5-(4-Fluorophenyl)-4-[(6-methyl-pyridin-2-ylmethyl)-amino]-thieno[2,3-d]pyrimidin-2-yl}-(2-hydroxy-ethyl)-amino]-ethanol |
| 221 | 2-{5-Benzo[1,3]dioxol-5-yl-4-[(pyridin-2-ylmethyl)-amino]-thieno[2,3-d]pyrimidin-2-ylamino}-ethanol |
| 222 | 2-{5-Benzo[1,3]dioxol-5-yl-4-[(6-methyl-pyridin-2-ylmethyl)-amino]-thieno[2,3-d]pyrimidin-2-ylamino}-ethanol |
| 223 | 2-[{5-Benzo[1,3]dioxol-5-yl-4-[(pyridin-2-ylmethyl)-amino]-thieno[2,3-d]pyrimidin-2-yl}-(2-hydroxy-ethyl)-amino]-ethanol |
| 224 | 2-[{5-Benzo[1,3]dioxol-5-yl-4-[(6-methyl-pyridin-2-ylmethyl)-amino]-thieno[2,3-d]pyrimidin-2-yl}-(2-hydroxy-ethyl)-amino]-ethanol |

### Example 225

### (4-Oxo-5-phenyl-3,4-dihydrothieno[2,3-d]pyrimidin-2-yl)acetic acid ethyl ester

Hydrogen chloride gas was bubbled through a stirred reaction mixture of 2-amino-4-phenylthiophene-3-carboxylic acid ethyl ester (4.94g, 0.02mol) in ethyl cyanoacetate (50ml) for 2 hours. A thick suspension formed initially which slowly dissolved on gentle warming. The mixture was allowed to stand at ambient temperature for 18 hours. Excess hydrogen chloride was removed by bubbling nitrogen through the reaction mixture and most of the excess ethyl cyanoacetate was distilled out at reduced pressure. The solid residue was recrystallised from ethanol to give (4-oxo-5-phenyl-3,4-dihydrothieno[2,3-*d*]pyrimidin-2-yl)acetic acid ethyl ester (3.64g), which was used without further purification.

### Examples 226 to 230

The compounds set out below were prepared in the same way as in Example 225, using the appropriate starting materials.

| Example | Compound |
|---|---|
| 226 | [5-(4-Fluorophenyl)-4-oxo-3,4-dihydro-thieno[2,3-d]pyrimidin-2-yl]-acetic acid ethyl ester |
| 227 | (5-Benzo[1,3]dioxol-5-yl-4-oxo-3,4-dihydro-thieno[2,3-d]pyrimidin-2-yl)-acetic acid ethyl ester |
| 228 | (6-Methyl-4-oxo-5-phenyl-3,4-dihydro-thieno[2,3-d]pyrimidin-2-yl)-acetic acid ethyl ester |
| 229 | [5-(4-Fluorophenyl)-6-methyl-4-oxo-3,4-dihydro-thieno[2,3-d]pyrimidin-2-yl]-acetic acid ethyl ester |
| 230 | (5-Benzo[1,3]dioxol-5-yl-6-methyl-4-oxo-3,4-dihydro-thieno[2,3-d]pyrimidin-2-yl)-acetic acid ethyl ester |

### Example 231

### (4-Chloro-5-phenylthieno[2,3-d]pyrimidin-2-yl)acetic acid ethyl ester

A stirred solution of (4-oxo-5-phenyl-3,4-dihydrothieno[2,3*-d*]pyrimidin-2-yl)acetic acid ethyl ester (1.0g, 3.185mmol), phosphoryl chloride (15ml) and *N*,*N*-dimethylaniline (4.8ml) was heated under reflux for 6 hours and then left to stand at ambient temperature for 18 hours. The excess phosphoryl chloride was removed *in vacuo* to give a dark residue, which was dissolved in dichloromethane (100ml) and then washed with water (2x50ml) followed by saturated sodium hydrogen carbonate solution (50ml). The organic layer was dried (MgSO₄) and the solvent was removed *in vacuo* to give the crude product. Purification by flash chromatography (silica) eluting with dichloromethane and 40°-60° petroleum ether (3:1) gave (4-chloro-5-phenylhieno[2,3*-d*]pyrimidin-2-yl)acetic acid ethyl ester as a pale-yellow solid (0.86g).

### Examples 232 to 236

The compounds set out below were prepared in the same way as in Example 231, using the appropriate starting materials.

| Example | Compound |
|---|---|
| 232 | [4-Chloro-5-(4-Fluorophenyl)-thieno[2,3-d]pyrimidin-2-yl]-acetic acid ethyl ester |
| 233 | (4-Chloro-5-Benzo[1,3]dioxol-5-yl-thieno[2,3-d]pyrimidin-2-yl)-acetic acid ethyl ester |
| 234 | (4-Chloro-6-methyl-5-phenyl-thieno[2,3-d]pyrimidin-2-yl)-acetic acid ethyl ester |
| 235 | [4-Chloro-5-(4-fluorophenyl)-6-methyl-thieno[2,3-d]pyrimidin-2-yl]-acetic acid ethyl ester |
| 236 | (5-Benzo[1,3]dioxol-5-yl-4-chloro-6-methyl-thieno[2,3-d]pyrimidin-2-yl)-acetic acid ethyl ester |

### Example 237

### {5-Phenyl-4-[(pyridin-2-ylmethyl)amino]thieno[2,3-d]pyrimidin-2-yl} acetic acid ethyl ester

A stirred mixture of (4-chloro-5-phenylthieno[2,3*-d*]pyrimidin-2-yl)acetic acid ethyl ester (0.36g, 1.08mmol), 2-aminomethylpyridine (0.12ml, 1.19mmol), triethylamine (0.17ml, 1.19mmol) and ethanol (8ml) was heated under reflux for 4 hours. The solution was then cooled to ambient temperature, poured into water (100ml) and extracted with dichloromethane (3x50ml). The combined organic extracts were dried (MgSO₄) and the solvent removed *in vacuo* to give the crude product. Purification by flash chromatography (silica) eluting with ethyl acetate and 40°-60° petroleum ether (1:2) gave {5-phenyl-4-[(pyridin-2-ylmethyl)amino]thieno[2,3*-d*]pyrimidin-2-yl}acetic acid ethyl ester as a colourless gum (0.41 g).

### Examples 238 to 249

The compounds set out below were prepared in the same way as in Example 237, using the appropriate starting materials.

| Example | Compound |
|---|---|
| 238 | {4-[(Furan-2-ylmethyl)-amino]-5-phenyl-thieno[2,3-d]pyrimidin-2-yl}-acetic acid ethyl ester |
| 239 | {5-(4-Fluorophenyl)-4-[(pyridin-2-ylmethyl)-amino]-thieno[2,3-d]pyrimidin-2-yl}-acetic acid ethyl ester |
| 240 | {5-Benzo[1,3]dioxol-5-yl-4-[(pyridin-2-ylmethyl)-amino]-thieno[2,3-d]pyrimidin-2-yl}-acetic acid ethyl ester |
| 241 | {5-(4-Fluorophenyl)-4-[(6-methyl-pyridin-2-ylmethyl)-amino]-thieno[2,3-d]pyrimidin-2-yl}-acetic acid ethyl ester |
| 242 | {5-Benzo[1,3]dioxol-5-yl-4-[(6-methyl-pyridin-2-ylmethyl)-amino]-thieno[2,3-d]pyrimidin-2-yl}-acetic acidethyl ester |
| 243 | {6-Methyl-4-[(6-methyl-pyridin-2-ylmethyl)-amino]-5-phenyl-thieno[2,3-d]pyrimidin-2-yl}-aceticacidethyl ester |
| 244 | {5-(4-Fluorophenyl)-6-methyl-4-[(6-methyl-pyridin-2-ylmethyl)-amino]-thieno[2,3-d]pyrimidin-2-yl}-acetic acid ethyl ester |
| 245 | {5-Benzo[1,3]dioxol-5-yl-6-methyl-4-[(6-methyl-pyridin-2-ylmethyl)-amino]-thieno[2,3-d]pyrimidin-2-yl}-acetic acid ethyl ester |
| 246 | {6-Methyl-5-phenyl-4-[(pyridin-2-ylmethyl)-amino]-thieno[2,3-d]pyrimidin-2-yl}-acetic acid ethyl ester |
| 247 | {5-(4-Fluorophenyl)-6-methyl-4-[(pyridin-2-ylmethyl)-amino]-thieno[2,3-d]pyrimidin-2-yl}-acetic acid ethyl ester |
| 248 | {5-Benzo[1,3]dioxol-5-yl-6-methyl-4-[(pyridin-2-ylmethyl)-amino]-thieno[2,3-d]pyrimidin-2-yl}-acetic acid ethyl ester |
| 249 | 4-[(6-Methyl-pyridin-2-ylmethyl)-amino]-5-phenyl-thieno[2,3-d]pyrimidine-2-carboxylic acid ethyl ester |

### Example 250

### 2-{5-Phenyl-4-[(pyridin-2-ylmethyl)amino]thieno[2,3-d]pyrimidin-2-yl}ethanol

A stirred solution of {5-phenyl-4-[(pyridin-2-ylmethyl)amino]thieno[2,3-d]pyrimidin-2-yl}acetic acid ethyl ester (0.10g, 0.248mmol) in anhydrous tetrahydrofuran (1ml) was cooled in an ice-bath and treated, under a nitrogen atmosphere, with diisobutylaluminium hydride (1M solution in hexane, 1.04ml, 1.04mmol) over about 15 minutes. The reaction mixture was allowed to warm up and left to stir at ambient temperature for 3 hours. The resultant mixture was then cooled in an ice-bath and quenched by the slow addition of methanol (0.5ml) followed by water (1 ml). The mixture was then diluted with 2M sodium hydroxide solution (10ml) and extracted with ethyl acetate (3x20ml). The combined organic extracts were dried (MgSO₄) and the solvent removed *in vacuo to* give the crude product. Purification by flash chromatography (silica) eluting with ethyl acetate and 40°-60° petroleum ether (4:1) gave 2-{5-phenyl-4-[(pyridin-2-ylmethyl) amino]thieno[2,3*-d*]pyrimidin-2-yl}ethanol as an off-white solid (0.05g), m.p. 90°-92°C.

### Examples 251 to 262

The compounds set out below were prepared in the same way as in Example 250, using the appropriate starting materials.

| Example | Compound |
|---|---|
| 251 | 2-{4-[(Furan-2-ylmethyl)-amino]-5-phenyl-thieno[2,3-d]pyrimidin-2-yl}-ethanol |
| 252 | 2-{4-[(6-Methyl-pyridin-2-ylmethyl)-amino]-5-phenyl-thieno[2,3-d]pyrimidin-2-yl}-ethanol |
| 253 | 2-{5-(4-Fluorophenyl)-4-[(pyridin-2-ylmethyl)-amino]-thieno[2,3-d]pyrimidin-2-yl}-ethanol |
| 254 | 2-{5-(4-Fluorophenyl)-4-[(6-methyl-pyridin-2-ylmethyl)-amino]-thieno[2,3-d]pyrimidin-2-yl}-ethanol |
| 255 | 2-{5-Benzo[1,3]dioxol-5-yl-4-[(pyridin-2-ylmethyl)-amino]-thieno[2,3-d]pyrimidin-2-yl}-ethanol |
| 256 | 2-{5-Benzo[1,3]dioxol-5-yl-4-[(6-methyl-pyridin-2-ylmethyl)-amino]-thieno[2,3-d]pyrimidin-2-yl}-ethanol |
| 25 | 2-{6-Methyl-5-phenyl-4-[(pyridin-2-ylmethyl)-amino]-thieno[2,3-d]pyrimidin-2-yl}-ethanol |
| 258 | 2-{6-Methyl-4-[(6-methyl-pyridin-2-ylmethyl)-amino]-5-phenyl-thieno[2,3-d]pyrimidin-2-yl}-ethanol |
| 259 | 2-{5-(4-Fluorophenyl)-6-methyl-4-[(pyridin-2-ylmethyl)-amino]-thieno[2,3-d]pyrimidin-2-yl}-ethanol |
| 260 | 2-{5-(4-Fluorophenyl)-6-methyl-4-[(6-methyl-pyridin-2-ylmethyl)-amino]-thieno[2,3-d]pyrimidin-2-yl}-ethanol |
| 261 | 2-{5-Benzo[1,3]dioxol-5-yl-6-methyl-4-[(pyridin-2-ylmethyl)-amino]-thieno[2,3-d]pyrimidin-2-yl}-ethanol |
| 262 | 2-{5-Benzo[1,3]dioxol-5-yl-6-methyl-4-[(6-methyl-pyridin-2-ylmethyl)-amino]-thieno[2,3-d]pyrimidin-2-yl}-ethanol |

### Example 263

### 2-{5-Phenyl-4-[(pyridin-2-ylmethyl)amino]thieno[2,3-d]pyrimidin-2-yl}malonic acid diethyl ester

Sodium hydride (60% dispersion in oil, 64mg, 1.59mmol) was treated with a solution of {5-phenyl-4-[(pyridin-2-ylmethyl)amino]thieno[2,3*-d*]pyrimidin-2-yl}acetic acid ethyl ester (0.43g, 1.06mmol) in diethyl carbonate (2.5ml). The resulting suspension was stirred at ambient temperature for 5 hours and left to stand for 18 hours. The mixture was then quenched with aqueous ammonium chloride solution (50ml) and extracted with diethyl ether (3x50ml). The combined organic extracts were dried (MgSO₄) and the solvent removed *in vacuo* to give the crude product. Purification by flash chromatography (silica) eluting with dichloromethane followed by dichloromethane and ethyl acetate (5:1) gave 2-{5-phenyl-4-[(pyridin-2-ylmethyl)amino]thieno[2,3*-d*]pyrimidin-2-yl}malonic acid diethyl ester as a colourless gum (0.32g).

### Examples 264 to 274

The compounds set out below were prepared in the same way as in Example 263, using the appropriate starting materials.

| Example | Compound |
|---|---|
| 264 | 2-{5-(4-Fluorophenyl)-4-[(pyridin-2-ylmethyl)-amino]-thieno[2,3-d]pyrimidin-2-yl}-malonic acid diethyl ester |
| 265 | 2-{5-Benzo[1,3]dioxol-5-yl-4-[(pyridin-2-ylmethyl)-amino]-thieno[2,3-d]pyrimidin-2-yl}-malonic acid diethyl ester |
| 266 | 2-{5-(4-Fluorophenyl)-4-[(6-methyl-pyridin-2-ylmethyl)-amino]-thieno[2,3*-d*]pyrimidin-2-yl}-malonic acid diethyl ester |
| 267 | 2-{4-[(6-Methyl-pyridin-2-ylmethyl)-amino]-5-phenyl-thieno[2,3d]pyrimidin-2-yl}-malonic acid diethyl ester |
| 268 | 2-{5-Benzo[1,3]dioxol-5-yl-4-[(6-methyl-pyridin-2-ylmethyl)-amino]-thieno[2,3-d]pyrimidin-2-yl}-malonic acid diethyl ester |
| 269 | 2-{6-Methyl-5-phenyl-4-[(pyridin-2-ylmethyl)-amino]-thieno[2,3d]pyrimidin-2-yl}-malonic acid diethyl ester |
| 270 | 2-{5-(4-Fluorophenyl)-6-methyl-4-[(pyridin-2-ylmethyl)-amino]-thieno[2,3-d]pyrimidin-2-yl}-malonic acid diethyl ester |
| 271 | 2-{5-Benzo[1,3]dioxol-5-yl-6-methyl-4-[(pyridin-2-ylmethyl)-amino]-thieno[2,3-d]pyrimidin-2-yl}-malonic acid diethyl ester |
| 272 | 2-{6-Methyl-4-[(6-methyl-pyridin-2-ylmethyl)-amino]-5-phenylthieno[2,3-d]pyrimidin-2-yl}-malonic acid diethyl ester |
| 273 | 2-{5-(4-Fluorophenyl)-6-methyl-4-[(6-methyl-pyridin-2-ylmethyl)-amino]-thieno[2,3-d]pyrimidin-2-yl}-malonic acid diethyl ester |
| 274 | 2-{5-Benzo[1,3]dioxol-5-yl-6-methyl-4-[(6-methyl-pyridin-2-ylmethyl)-amino]-thieno[2,3-d]pyrimidin-2-yl}-malonic acid diethyl ester |

### Example 275

### 2-{5-Phenyl-4-[(pyridin-2-ylmethyl)-amino]-thieno[2,3-d]pyrimidin-2-yl}-propane-1,3-diol

A stirred solution of 2-{5-phenyl-4-[(pyridin-2-ylmethyl)amino]thieno[2,3*-d*]pyrimidin-2-yl}malonic acid diethyl ester (0.32g, 0.672mmol) in anhydrous tetrahydrofuran (10ml) was cooled in an ice-bath and treated, under a nitrogen atmosphere, with diisobutylaluminium hydride (1 M solution in hexane, 5.52ml), 5.52mmol) over 15 minutes. The reaction mixture was allowed to warm up and left to stir at ambient temperature for 3 hours. The resultant mixture was then cooled in an ice-bath and quenched by the slow addition of methanol (5ml) followed by water (10ml). The mixture was then diluted with 2M sodium hydroxide solution (50ml) and extracted with ethyl acetate (3x50ml). The combined organic extracts were dried (MgSO₄) and the solvent removed *in vacuo* to give the crude product. Purification by flash chromatography (silica) eluting with 5% methanol in dichloromethane gave 2-{5-phenyl-4-[(pyridin-2-ylmethyl)amino]thieno [2,3*-d*]pyrimidin-2-yl}propane-1,3-diol as a pale yellow solid (0.08g), m.p. 137°-139°C.

### Examples 276 to 286

The compounds set out below were prepared in the same way as in Example 275, using the appropriate starting materials.

| Example | Compound |
|---|---|
| 276 | 2-{4-[(6-Methyl-pyridin-2-ylmethyl)-amino]-5-phenyl-thieno[2,3-d]pyrimidin-2-yl}-propane-1,3-diol |
| 277 | 2-{5-(4-Fluorophenyl)-4-[(pyridin-2-ylmethyl)-amino]-thieno[2,3-d]pyrimidin-2-yl}-propane-1,3-diol |
| 278 | 2-{5-(4-Fluorophenyl)-4-[(6-methyl-pyidin-2-ylmethyl)-amino]-thieno[2,3-d]pyrimidin-2-yl}-propane-1,3-diol |
| 279 | 2-{5-Benzo[1,3]dioxol-5-yl-4-[(pyridin-2-ylmethyl)-amino]-thieno[2,3-d]pyrimidin-2-yl}-propane-1,3-diol |
| 280 | 2-{5-Benzo[1,3]dioxol-5-yl-4-[(6-methyl-pyridin-2-ylmethyl)-amino]-thieno[2,3-d]pyrimidin-2-yl}-propane-1,3-diol |
| 281 | 2-{6-Methyl-5-phenyl-4-[(pyridin-2-ylmethyl)-amino]-thieno[2,3-d]pyrimidin-2-yl}-propane-1,3-diol |
| 282 | 2-{6-Methyl-4-[(6-methyl-pyridin-2-ylmethyl)-amino]-5-phenyl-thieno[2,3-d]pyrimidin-2-yl}-propane-1,3-diol |
| 283 | 2-{5-(4-Fluorophenyl)-6-methyl-4-[(pyridin-2-ylmethyl)-amino]-thieno[2,3-d]pyrimidin-2-yl}-propane-1,3-diol |
| 284 | 2-{5-(4-Fluorophenyl)-6-methyl-4-[(6-methyl-pyridin-2-ylmethyl)-amino]thieno[2,3-d]pyrimidin-2-yl}-propane-1,3-diol |
| 285 | 2-{5-Benzo[1,3]dioxol-5-yl-6-methyl-4-[(pyridin-2-ylmethyl)-amino]-thieno[2,3-d]pyrimidin-2-yl}-propane-1,3-diol |
| 286 | 2-{5-Benzo[1,3]dioxol-5-yl-6-methyl-4-[(6-methyl-pyridin-2-ylmethyl)-amino]-thieno[2,3-d]pyrimidin-2-yl}-propane-1,3-diol |

### Example 287

### N-Methyl-2-{5-phenyl-4-[{pyridin-2-ylmethyl)amino]thieno[2,3-d]pyrimidin-2-yl}acetamide

In a 10ml glass tube were placed {5-phenyl-4-[(pyridin-2-ylmethyl)amino]thieno[2,3*-d*]pyrimidin-2-yl}acetic acid ethyl ester (35mg, 0.088mmol) and a saturated solution of methylamine in ethanol (2.0ml). The tube was sealed with a septum and placed in the microwave cavity. Microwave irradiation of 200W was used, the temperature being ramped from room temperature to 100°C. Once 100°C was reached, the reaction mixture was held at this temperature for 30 minutes. The temperature was then ramped up to 150°C and the reaction mixture was held at this temperature for a further 30 minutes. The resultant mixture was diluted with water (50ml) and extracted with dichloromethane (3x50ml). The combined organic extracts were dried (MgSO₄) and the solvent removed *in vacuo* to give a residue which was purified by flash chromatography (silica) eluting with 5% methanol in dichloromethane to give *N*-methyl-2-{5-phenyl-4-[ {pyridin-2-ylmethyl)amino]thieno[2,3*-d*]pyrimidin-2-yl}acetamide (2mg).

### Examples 288 to 298

The compounds set out below were prepared in the same way as in Example 287, using the appropriate starting materials.

| Example | Compound |
|---|---|
| 288 | N-Methyl-2-{4-[(6-methyl-pyridin-2-ylmethyl)-amino]-5-phenyl-thieno[2,3-d]pyrimidin-2-yl}-acetamide |
| 289 | 2-{5-(4-Fluorophenyl)-4-[(pyridin-2-ylmethyl)-amino]-thieno[2,3-d]pyrimidin-2-yl}-N-methyl-acetamide |
| 290 | 2-{5-(4-Fluorophenyl)-4-[(6-methyl-pyridin-2-ylmethyl)-amino]-thieno[2,3-d]pyrimidin-2-yl}-N-methyl-acetamide |
| 291 | 2-{5-Benzo[1,3]dioxol-5-yl-4-[(pyridin-2-ylmethyl)-amino]-thieno[2,3-d]pyrimidin-2-yl}-N-methyl-acetamide |
| 292 | 2-{5-Benzo[1,3]dioxol-5-yl-4-[(6-methyl-pyridin-2-ylmethyl)-amino]-thieno[2,3-d]pyrimidin-2-yl}-N-methyl-acetamide |
| 293 | N-Methyl-2-{6-methyl-5-phenyl-4-[(pyridin-2-ylmethyl)-amino]-thieno[2,3-d]pyrimidin-2-yl}-acetamide |
| 294 | N-Methyl-2-{6-methyl-4-[(6-methyl-pyridin-2-ylmethyl)-amino]-5-phenyl-thieno[2,3-d]pyrimidin-2-yl}-acetamide |
| 295 | 2-{5-(4-Fluorophenyl)-6-methyl-4-[(pyridin-2-ylmethyl)-amino]-thieno[2,3-d]pyrimidin-2-yl}-N-methyl-acetamide |
| 296 | 2-{5-(4-Fluorophenyl)-6-methyl-4-[(6-methyl-pyridin-2-ylmethyl)-amino]-thieno[2,3-d]pyrimidin-2-yl}-N-methyl-acetamide |
| 297 | 2-{5-Benzo[1,3]dioxol-5-yl-6-methyl-4-[(pyridin-2-ylmethyl)-amino]-thieno[2,3-d]pyrimidin-2-yl}-N-methyl-acetamide |
| 298 | 2-{5-Benzo[1,3]dioxol-5-yl-6-methyl-4-[(6-methyl-pyridin-2-ylmethyl)-amino]-thieno[2,3-d]pyrimidin-2-yl}-N-methyl-acetamide |

### Example 299

### 2-Chloromethyl-5-phenyl-3H-thieno[2,3-d]pyrimidin-4-one

Hydrogen chloride gas was bubbled through a stirred solution of 2-amino-4-phenylthiophene-3-carboxylic acid ethyl ester (4.94g, 0.02mol) and chloroacetonitrile (1.4ml), 0.022mol) in anhydrous 1,4-dioxane (60ml) for about 4 hours. A thick suspension formed initially which slowly dissolved. The mixture was stirred at ambient temperature for 18 hours before being poured into water (250ml) and basified (pH 8) by the addition of sodium hydrogen carbonate. The supernatant was then decanted to leave a gummy solid which was triturated with aqueous ethanol to give 2-chloromethyl-5-phenyl-3H-thieno[2,3-*d*]pyrimidin-4-one as a yellow solid (3.70g), which was used without further purification.

### Example 300

### 4-Chloro-2-chloromethyl-5-phenylthieno[2,3-d]pyrimidine

A stirred suspension of 2-chloromethyl-5-phenyl-3H-thieno[2,3*-d*]pyrimidin-4-one (1.5g, 5.42mmol) and phosphoryl chloride (23ml) was heated under reflux for 7 hours and then left to stand at ambient temperature for 18 hours. The excess phosphoryl chloride was removed *in vacuo* to give a dark residue, which was dissolved in dichloromethane (100ml) and then washed with water (2x100ml) followed by saturated sodium hydrogen carbonate solution (1 00ml).The organic extract was dried (MgSO₄) and the solvent was removed *in vacuo* to give the crude product. Purification by flash chromatography (silica) eluting with dichloromethane gave 4-chloro-2-chloromethyl-5-phenylthieno[2,3*-d*]pyrimidine as a pale-yellow solid (1.42g).

### Example 301

### (2-Chloromethyl-5-phenylthieno[2,3-d]pyrimidln-4-yl)pyridin-2-ylmethylamine

A reaction mixture of 4-chloro-2-chloromethyl-5-phenylthieno[2,3*-d*]pyrimidine (0.50g, 1.69mmol), 2-aminomethylpyridine (0.17ml, 1.69mmol), triethylamine (0.26ml, 1.86mmol) and propan-2-ol (15ml) was stirred at ambient temperature for 4 days. The solution was then poured into water (150ml) and extracted with ethyl acetate (3x75ml).

The combined organic extracts were dried (MgSO₄) and the solvent removed *in vacuo* to give the crude product. Purification by flash chromatography (silica) eluting with ethyl acetate and 40°-60° petroleum ether (1:4) gave (2-chloromethyl-5-phenylthieno[2,3*-d*]pyrimidin-4-yl)pyridin-2-ylmethylamine as a yellow solid (0.17g), m.p. 97°-99°C.

### Example 302

### (2-Dimethylaminomethyl-5-phenylthieno[2,3-d]pyrimidin-4-yl)pyridin-2-ylmethylamine

In a 10ml glass tube were placed (2-chloromethyl-5-phenylthieno[2,3*-d*]pyrimidin-4-yl)pyridin-2-ylmethylamine (50mg, 0.136mmol) and a saturated solution of dimethylamine in ethanol (2.0ml). The tube was sealed with a septum and placed in the microwave cavity. Microwave irradiation of 200W was used, the temperature being ramped from room temperature to 150°C. Once 150°C was reached, the reaction mixture was held at this temperature for 10 minutes. The resultant mixture was diluted with water (50ml) and extracted with dichloromethane (3x50ml). The combined organic extracts were dried (MgSO₄) and the solvent removed *in vacuo* to give the crude product. Purification by flash chromatography (silica) eluting with 4% triethylamine in ethyl acetate gave (2-dimethylaminomethyl-5-phenylthieno[2,3*-d*]pyrimidin-4-yl)pyridin-2-ylmethylamine as a yellow gum (36mg).

### Examples 303 to 304

The compounds set out below were prepared in the same way as in Example 302, using the appropriate starting materials.

| Example | Compound |
|---|---|
| 303 | (2-Morpholin-4-ylmethyl-5-phenyl-thieno[2,3-d]pyrimidin-4-yl)-pyridin-2-ylmethyl-amine |
| 304 | (2-Methylaminomethyl-5-phenyl-thieno[2,3-d]pyrimidin-4-yl)-pyridin-2-ylmethyl-amine |

### Example 305

### (2-Methoxymethyl-5-phenylthien[2,3-d]primidin-4-yl)pyridin-2-ylmethylamine

In a 10ml glass tube was placed anhydrous methanol (1ml). This was cooled in an ice-bath and treated with sodium hydride (60% dispersion in oil, 6mg, 0.147mmol). After stirring for about 10 minutes, (2-chloromethyl-5-phenylthieno[2,3*-d*]pyrimidin-4-yl)pyridin-2-ylmethylamine (36mg, 0.098mmol) was added. The tube was sealed with a septum and placed in the microwave cavity. Microwave irradiation of 200W was used, the temperature being ramped from room temperature to 150°C. Once 150°C was reached, the reaction mixture was held at this temperature for 10 minutes. The resultant mixture was diluted with water (50ml) and extracted with dichloromethane (3x50ml). The combined organic extracts were dried (MgSO₄) and the solvent removed *in vacuo* to give the crude product. Purification by flash chromatography (silica) eluting with ethyl acetate and 40°-60° petroleum ether (3:2) gave (2-methoxymethyl-6-phenylthien[2,3*-d*] primidin-4-yl)pyidin-2-ylmethylamine as a pale brown gum (1mg).

### Example 306

### 6-Bromo-3H-thieno[2,3-d]pyrimidin-4-one

A suspension of 3*H-*thieno[2,3*-d*]pyrimidin-4-one (7.68g, 0.056mol) in glacial acetic acid (75ml) was treated with bromine (7.5ml) and stirred at ambient temperature for 4 hours. The resulting solid was collected by filtration, washed with water and dried *in vacuo* to give 6-bromo-3*H-*thieno[2,3*-d*]pyrimidin-4-one as a light brown solid (11.64g), which was used without further purification.

### Example 307

The compound set out below was prepared in the same way as in Example 306 , using the appropriate starting materials.

| Example | Compound |
|---|---|
| 307 | 6-Bromo-5-phenylthieno[2,3-d]pyrimidin-4(3H)-one |

### Example 308

### 6-Bromo-4-chlorothieno[2,3-d]pyrimidine

6-Bromo-3*H-*thieno[2,3*-d*]pyrimidin-4-one (11.64g, 0.05ml) was added portion-wise to phosphoryl chloride (220ml) and the resulting mixture was heated under reflux for 6 hours. The excess phosphoryl chloride was then removed *in vacuo.* The resulting residue was dissolved in dichloromethane (250ml) and washed with water (2x100ml), followed by saturated sodium hydrogen carbonate solution (100ml). The organic layer was then dried (MgSO₄) and the solvent was removed *in vacuo* to give 6-bromo-4-chlorothieno[2,3*-d*]pyrimidine (9.06g) as a yellow solid, which was used without further purification.

### Example 309

The compound set out below was prepared in the same way as in Example 308, using the appropriate starting materials.

| Example | Compound |
|---|---|
| 309 | 6-Bromo-4-chloro-5-phenylthieno[2,3-d]pyrimidin-4(3H)-one |

### Example 310

### 5-Bromo-4-chlorothieno[2,3-d]pyrimidine

A stirred solution of 6-bromo-4-chlorothieno[2,3*-d*]pyrimidine (4.0g, 0.016mol) in anhydrous tetrahydrofuran (100ml) was cooled in a dry-ice/acetone bath and treated, under a nitrogen atmosphere, with lithium diisopropylamide (1.8M solution in tetrahydrofuran, 9.0ml, 0.016mol) over about 20 minutes. The resultant dark solution was stirred in the cold for 1 hour and then treated with a mixture of water (5ml) and tetrahydrofuran (20ml) over about 20 minutes. The mixture was then allowed to warm up to about 0°C before being poured into water (250ml) and extracted with dichloromethane (3x100ml). The combined organic extracts were dried (MgSO₄) and the solvent removed *in vacuo* to give the crude product. Purification by flash chromatography (silica) eluting with dichloromethane gave 5-bromo-4-chlorothieno[2,3*-d*]pyrimidine as a pale brown solid (3.8g).

### Example 311

### (5-Bromothieno[2,3-d]pyrimidin-4-yl)pyridin-2-ylmethylamine

A stirred mixture of 5-bromo-4-chlorothieno[2,3*-d*]pyrimidine (3.8g, 15.2mmol), ethanol (250ml), triethylamine (2.32ml, 16.7mmol) and 2-aminomethylpyridine (1.72ml, 16.7mmol) was heated under reflux for 2.5 hours. The solution was then cooled to ambient temperature, poured into water (300ml) and extracted with dichloromethane (3x150ml). The combined organic extracts were dried (MgSO₄) and the solvent removed *in vacuo* to give the crude product. Purification by flash chromatography (silica) eluting with ethyl acetate and 40°-60° petroleum ether (1:1) gave (5-bromothieno[2,3*-d*] pyrimidin-4-yl)pyridin-2-ylmethylamine as a yellow solid (3.12g), m.pt. 127°-129°C.

### Example 312

### [5-(1-Methyl-1H-indol-5-yl)thieno[2,3-d]pyrimidin-4-yl]pyridin-2-ylmethylamine

In a 10ml glass tube were placed 5-bromothieno[2,3-d]pyrimidin-4-yl)pyridin-2-ylmethylamine (47mg, 0.146mmol), N-methylindole-5-boronic acid (51mg, 0.294mmol), polymer-bound triphenylphosphine-Pd(0) (Argonaut PS-PPh₃-Pd, 0.13mmol/g,146mg, 0.0146mmol), sodium carbonate (46mg, 0.440mmol), dimethoxyethane (0.75ml), ethanol (0.75ml) and water (0.5ml). The tube was sealed with a septum and placed in the microwave cavity. Microwave irradiation of 200W was used, the temperature being ramped from room temperature to 150°C. Once 150°C was reached, the reaction mixture was held at this temperature for 1 hour. The resultant mixture was filtered through Kieselguhr, the filtered solid being washed through with water and dichloromethane. The filtrate was then extracted with dichloromethane (3x50ml) and the organic extracts dried (MgSO₄). The solvent was removed *in vacuo* to give the crude product. Purification by flash chromatography (silica) eluting with ethyl acetate and 40°-60° petroleum ether (2:1) gave [5-(1-methyl-1*H-*indol-5-yl)thieno[2,3*-d*]pyrimidin-4-yl]pyridin-2-ylmethylamine as a yellow solid (25mg), m.pt. 184-185°C.

### Examples 313 to 333

The compounds set out below were prepared in the same way as in Example 312, using the appropriate starting materials.

| Example | Compound |
|---|---|
| 313 | Pyridin-2-ylmethyl-[5-(4-trifluoromethyl-phenyl)-thieno[2,3-d]pyrimidin-4-yl]-amine |
| 314 | (5-Benzo[1,3]dioxol-5-yl-thieno[2,3-d]pyrimidin-4-yl)-pyridin-2-ylmethyl-amine |
| 315 | [5-(4-Dimethylamino-phenyl)-thieno[2,3-d]pyrimidin-4-yl]-pyridin-2-ylmethyl-amine |
| 316 | [5-(3,4-Dimethyl-phenyl)-thieno[2,3-d]pyrimidin-4-yl]-pyridin-2-ylmethyl-amine |
| 317 | Pyridin-2-ylmethyl-[5-(4-trifluoromethoxy-phenyl)-thieno[2,3-d]pyrimidin-4-yl]-amine |
| 318 | Pyridin-2-ylmethyl-(5-p-tolyl-thieno[2,3-d]pyrimidin-4-yl)-amine |
| 319 | (5-Benzo[1,3]dioxol-5-yl-thieno[2,3-d]pyrimidin-4-yl)-pyridin-2-ylmethyl-amine |
| 320 | [5-(2,3-Dihydro-benzo[1,4]dioxin-6-yl)-thieno[2,3-d]pyrimidin-4-yl]-pyridin-2-ylmethyl-amine |
| 321 | [5-(3-Chlorophenyl)-thieno[2,3-d]pyrimidin-4-yl]-pyridin-2-ylmethyl-amine |
| 322 | [5-(3-Methoxyphenyl)-thieno[2,3-d]pyrimidin-4-yl]-pyridin-2-ylmethyl-amine |
| 323 | [5-(1H-Indol-6-yl)-thieno[2,3-d]pyrimidin-4-yl]-pyridin-2-ylmethyl-amine |
| 324 | [5-(4-Methoxymethoxy-phenyl)-thieno[2,3-d]pyrimidin-4-yl]-pyridin-2-ylmethyl-amine |
| 325 | [5-(4-Chlorophenyl)-thieno[2,3-d]pyrimidin-4-yl]-pyridin-2-ylmethyl-amine |
| 326 | [5-(2,2-Difluoro-benzo[1,3]dioxol-5-yl)-thieno[2,3-d]pyrimidin-4-yl]-pyridin-2-ylmethyl-amine |
| 327 | 4-{4-[(Pyridin-2-ylmethyl)-amino]-thieno[2,3-d]pyrimidin-5-yl}-benzoic acid methyl ester |
| 328 | [5-(6-Methoxy-pyridin-3-yl)-thieno[2,3-d]pyrimidin-4-yl]-pyridin-2-ylmethyl-amine |
| 329 | [5-(2,4-Dichloro-phenyl)-thieno[2,3-d]pyrimidin-4-yl]-pyridin-2-ylmethyl-amine |
| 330 | [5-(4-Chloro-3-trifluoromethyl-phenyl)-thieno[2,3-d]pyrimidin-4-yl]-pyridin-2-ylmethyl-amine |
| 331 | [5-(3-Fluorophenyl)-thieno[2,3-d]pyrimidin-4-yl]-pyridin-2-ylmethyl-amine |
| 332 | [5-(4-Morpholin-4-yl-phenyl)-thieno[2,3-d]pyrimidin-4-yl]-pyridin-2-ylmethyl-amine |
| 333 | [5-(3,4-Difluoro-phenyl)-thieno[2,3-d]pyrimidin-4-yl]-pyridin-2-ylmethyl-amine |

### Example 334

### 5-Bromo-6-methyl-1H-thieno[2,3-d]pyrimidine-2,4-dione

A brown glass round-bottom flask was charged with 6-methyl-1*H-*thieno[2,3*-d*]pyrimidine-2,4-dione (2.70g, 14.8mmol) and glacial acetic acid (30ml). Bromine (2.70ml) was added and the mixture stirred at ambient temperature for 4 hours. The resultant mixture was diluted with water (30ml) and the solid was collected by filtration, washed thoroughly with water and then dried *in vacuo* to give 5-bromo-6-methyl-1*H-*thieno[2,3*-d*]pyrimidine-2,4-dione as a brown solid (2.43g), which was used without further purification.

### Example 335

### 5-Bromo-2,4-dichloro-6-methylthleno[2,3-d]pyrimidine

A stirred mixture of 5-bromo-6-methyl-1*H-*thieno[2,3*-d*]pyrimidine-2,4-dione (2.43g, 9.3mmol) and phenylphosphonic dichloride (15ml) was heated at a temperature of 150°C for 6 hours in a vessel protected by a calcium chloride drying tube. The reaction mixture was allowed to cool to ambient temperature and poured into ice-water (250ml). After stirring for 45 minutes, the resulting mixture was extracted with dichloromethane (3x100ml). The combined organic extracts were washed with water (100ml) followed by saturated sodium hydrogen carbonate solution (1 00ml) and dried (MgSO₄). The solvent was removed *in vacuo* to give the crude product which was purified by flash chromatography (silica) eluting with dichloromethane giving 2.02g of 5-bromo-2,4-dichloro-6-methylthieno[2,3*-d*]pyrimidine as a yellow solid.

### Example 336

### 5-Bromo-2-chloro-6-methylthieno[2,3-d]pyrimidin-4-yl)pyridin-2-ylmethylamine

A stirred mixture of 5-bromo-2,4-dichloro-6-methylthieno[2,3*-d*]pyrimidine (1.0g, 3.35mmol), ethanol (40ml), 2-aminomethylpyridine (0.40ml), 3.69mmol) and triethylamine (0.50ml), 3.69mmol) was heated at a temperature of 60°C for 1 hour. The reaction mixture was then cooled to ambient temperature, diluted with water (100ml) and extracted with dichloromethane (3x50ml). The combined organic extracts were dried (MgSO₄) and the solvent removed *in vacuo* to give a residue. Purification by flash chromatography (silica) eluting with dichloromethane followed by 5% ethyl acetate in dichloromethane gave (5-bromo-2-chloro-6-methylthieno[2,3*-d*]pyrimidin-4-yl)pyridin-2-ylmethylamine as a white solid (0.99g).

### Example 337

The compound set out below was prepared in the same way as in Example 336, using the appropriate starting materials.

| Example | Compound |
|---|---|
| 337 | 5-Bromo-2-chloro-6-methyl-thieno[2,3-d]pyrimidin-4-yl)-(6-methyl-pyridin-2-ylmethyl)-amine |

### Example 338

### 2-[{5-Bromo-6-methyl-4-[(pyridin-2-ylmethyl)amino]thieno[2,3-d]pyrimidin-2-yl}-(2-hydroxyethyl)amino]ethanol

In a 10ml glass tube were placed 5-bromo-2-chloro-6-methylthieno[2,3*-d*]pyrimidin-4-yl)pyridin-2-ylmethylamine (0.1 0g, 0.27mmol) and diethanolamine (0.80ml). The tube was sealed with a septum and placed in the microwave cavity. Microwave irradiation of 200W was used, the temperature being ramped from room temperature to 200°C. Once 200°C was reached, the reaction mixture was held at this temperature for 30 minutes. Aftercooling to ambient temperature, water (50 ml) was added and the mixture extracted with dichloromethane (3x50 ml). The combined organic extracts were dried (MgSO₄) and the solvent removed *in vacuo* to give the crude product which was recrystallised from dichloromethane to give 2-[{5-bromo-6-methyl-4-[(pyridin-2-ylmethyl)amino]thieno [2,3*-d*]pyrimidin-2-yl}-(2-hydroxyethyl)amino]ethanol as a white solid (0.71g), m.pt. 152°C.

### Examples 339 to 341

The compounds set out below were prepared in the same way as in Example 338, using the appropriate starting materials.

| Example | Compound |
|---|---|
| 339 | 2-{5-Bromo-6-methyl-4-[(6-methyl-pyridin-2-ylmethyl)-amino]-thieno[2,3-d]pyrimidin-2-ylamino}-ethanol |
| 340 | 2-[{5-Bromo-6-methyl-4-[(6-methyl-pyridin-2-ylmethyl)-amino]thieno[2,3-d]pyrimidin-2-yl}-(2-hydroxy-ethyl)-amino]-ethanol |
| 341 | 2-({5-Bromo-6-methyl-4-[(pyridin-2-ylmethyl)-amino]-thieno[2,3d]pyrimidin-2-yl}amino)-ethanol |

### Example 342

### 2-((2-hydroxyethyl)-{6-methyl-5-phenyl-4-[(pyridin-2-ylmethyl)amino]thieno[2,3-d]pyrimidin-2-yl}amino)ethanol

In a 10ml glass tube were placed 2-[{5-bromo-6-methyl-4-[(pyridin-2-ylmethyl)amino]thieno[2,3-*d*]pyrimidin-2-yl}-(2-hydroxyethyl)amino]ethanol (32mg, 0.073mmol), phenylboronic acid (20mg, 0.166mmol), sodium carbonate (26mg, 0.249mmol), triphenylphosphine (7mg, 0.025mmol), palladium(II)acetate (2mg, 0.008mmol), dimethoxyethane (0.75ml) and water (0.25ml). The tube was sealed with a septum and placed in the microwave cavity. Microwave irradiation of 200W was used, the temperature being ramped from room temperature to 150°C. Once 150°C was reached, the reaction mixture was held at this temperature for 1 hour. The resultant mixture was diluted with water (50ml), extracted into dichloromethane (3x50ml) and the combined organic extracts were dried (MgSO₄). The solvent was removed *in vacuo* to give the crude product which was purified by flash chromatography (silica) eluting with ethyl acetate followed by 5% methanol in ethyl acetate to give 2-((2-hydroxyethyl)-{6-methl-5-phenyl-4-[(pyridin-2-ylmethyl)amino]thieno[2,3-d] pyrimidin-2-yl}amino)ethanol as a brown gum (7mg).

### Examples 343 to 353

The compounds set out below were prepared in the same way as in Example 342, using the appropriate starting materials.

| Example | Compound |
|---|---|
| 343 | 2-[{5-Benzo[1,3]dioxol-5-yl-6-methyl-4-[(pyridin-2-ylmethyl)-amino]-thieno[2,3-*d*]pyrimidin-2-yl}-(2-hydroxy-ethyl)-amino]-ethanol |
| 344 | 2-[{5-(4-Fluorophenyl)-6-methyl-4-[(pyridin-2-ylmethyl)-amino]-thieno[2,3-d]pyrimidin-2-yl}-(2-hydroxy-ethyl)-amino]-ethanol |
| 345 | 2-{6-Methyl-5-phenyl-4-[(pyridin-2-ylmethyl)-amino]-thieno[2,3-d]pyrimidin-2-ylamino}-ethanol |
| 346 | 2-{6-Methyl-4-[(6-methyl-pyridin-2-ylmethyl)-amino]-5-phenyl-thieno[2,3-d]pyrimidin-2-ylamino}-ethanol |
| 347 | 2-((2-Hydroxy-ethyl)-{6-methyl-4-[(6-methyl-pyridin-2-ylmethyl)-amino]-5-phenyl-thieno[2,3-*d*] pyrimidin-2-yl}-amino)-ethanol |
| 348 | 2-{5-(4-Fluorophenyl)-6-methyl-4-[(pyridin-2-ylmethyl)-amino]-thieno[2,3-d]pyrimidin-2-ylamino}-ethanol |
| 349 | 2-{5-(4-Fluorophenyl)-6-methyl-4-[(6-methyl-pyridin-2-ylmethyl)-amino]-thieno[2,3-d]pyrimidin-2-ylamino}-ethanol |
| 350 | 2-[{5-(4-Fluorophenyl)-6-methyl-4-[(6-methyl-pyridin-2-ylmethyl)-amino]-thieno[2,3-d]pyrimidin-2-yl}-(2-hydroxy-ethyl)-amino]-ethanol |
| 351 | 2-{5-Benzo[1,3]dioxol-5-yl-6-methyl-4-[(pyridin-2-ylmethyl)-amino]-thieno[2,3-d]pyrimidin-2-ylamino}-ethanol |
| 352 | 2-{5-Benzo[1,3]dioxol-5-yl-6-methyl-4-[(6-methyl-pyridin-2-ylmethyl)-amino]-thieno[2,3-d]pyrimidin-2-ylamino}-ethanol |
| 353 | 2-[{5-Benzo[1,3]dioxol-5-yl-6-methyl-4-[(6-methyl-pyridin-2-ylmethyl)-amino]-thieno[2,3-d]pyrimidin-2-yl}-(2-hydroxy-ethyl)-amino]-ethanol |

### Example 354

Analytical Data for compounds representative of the above examples are shown in the table below.

| Example | NMR Spectrum ¹H (400 MHz; CDCl₃; Me₄Si), | Mass Spectrum (m/z) (APCI) |
|---|---|---|
| 101 | 2.65 (3H, s), 4.72 (2H, d), 6.5 (1H, br s), 6.98 (1H, s), 7.12 (1H, m), 7.19 (1H, d), 7.48 (5H, s), 7.60 (1H, t), 8.25 (1H, d). | 333 (100%, [M+H]+) |
| 83 | 4.60 (2H, d), 5.27 (1H, brs), 6.07 (1H, d), 6.27 (1H, d), 7.10 (1H, s), 7.29 (1H, s), 7.43 (5H, m), 8.55 (1H, s). | 308 (100%, [M+H]+) |
| 105 | 4.73 (2H, s), 6.60 (1H, br s), 7.09 (1H, s), 7.12 (1H, m), 7.17 (1H, d), 7.51 (5H, m), 7.59 (1H, t), 8.25 (1H, d), 8.55 (1H, s). | 319 (100%, [M+H]+) |
| 106 | 5.55 (2H, s), 6.62 (1H, d), 7.15 (1H, m), 7.29 (1H, s), 7.36 (3H, m), 7.50 (3H, m), 8.55 (1H, m), 8.71 (1H, s). | 320 (100%, [M+H]+) |
| 190 | 3.60 (2H, m), 3.81 (2H, m), 4.42 (1H, br s), 4.61 (2H, d), 5.27 (1H, m), 6.31 (1H, br s), 6.65 (1H, s), 7.15 (2H, m), 7.46 (5H, s), 7.60 (1H, t), 8.25 (1H, d) | 378 (100%, [M+H]+) |
| 189 | 3.38 (3H, s), 3.55 (2H, m), 3.65 (2H, m), 4.65 (2H, d), 5.19 (1H, m), 6.10 0 (1H, br s), 6.61 (1H, s), 7.11 (2H, m), 7.45 (5H, m), 7.60 (1H, t), 8.30 (1H, d). | 393 (100%, [M+2H]+) |
| 191 | 4.02 (3H, s), 4.70 (2H, d), 6.60 (1H, br s), 6.85 (1H, s), 7.13 (2H, m), 7.48 (5H, s), 7.58 (1H, t), 8.24 (1H, d). | 349 (100%, [M+H]+) |
| 196 | 3.00 (3H, d), 4.67 (2H, d), 4.82 (1H, br s), 6.07 (1H, br s), 6.60 (1H, s), 7.12 (2H, m), 7.43 (5H, m), 7.60 (1H, t), 8.31 (1H, d). | 348 (100%, [M+H]+) |
| 197 | 3.72 (4H, m), 3.80 (4H, m), 4.62 (2H, d), 6.04 (1H, br s), 6.62 (1H, s), 7.13 (2H, m), 7.45 (5H, m), 7.59 (1H, t), 8.30 (1H, d). | 404 (100%, [M+H]+) |
| 198 | 3.17 (6H, s), 4.68 (2H, d), 5.90 (1H br s), 6.59 (1H, s), 7.12 (1H, m), 7.18 (1H, d), 7.42 (5H, m), 7.58 (1H, t), 8.32 (1H, d). | 363 (100%, [M+2H]+) |
| 123 | 4.70 (2H, d), 6.65 (1H, br s), 7.09 (1H, s), 7.18 (4H, m), 7.48 (2H, m), 7.60 (1H, t), 8.25 (1H, d), 8.56 (1H, s). | 337 (100%, [M+H]+) |
| 138 | 3.90 (3H, s), 4.73 (2H,d), 6.64 (1H, br s), 7.04 (3H, m), 7.18 (2H, m), 7.40 (2H, m), 7.60 (1H, t), 8.28 (1H, d), 8.52 (1H, s). | 350 (100%, [M+2H]+) |
| 84 | 0.07 (2H, m), 0.38 (2H, m), 0.82 (1H, m), 3.30 (2H, m), 5.41 (1H, br s), 7.18 (1H, s), 7.50 (5H, m), 8.56 (1H, s). | 282 (100%, [M+H]+) |
| 135 | 4.61 (2H, d), 5.18 (1H, br s), 6.10 (1H, s), 6.28 (1H, s), 7.06 (1H, s), 7.32 (3H, m), 7.40 (2H, m), 8.51 (1H, s). | 341 (100%, [M]+) |
| 109 | 5.41 (2H, s), 6.28 (1H, d), 6.31 (1H, d), 7.26 (1H, s), 7.28 (3H, m), 7.40 (3H, m), 8.69 (1H, s). | 309 (100%, [M+H]+) |
| 108 | 2.20 (3H, s), 4.55 (2H, d), 5.26 (1H, br s), 5.82 (1H, d), 5.92 (1H, d), 7.07 (1H, s), 7.41 (5H, s), 8.52 (1H, s). | 322 (100%, [M+H]+) |
| 133 | 2.25 (3H, s), 2.30 (3H, s), 4.65 (2H, br s), 5.41 (1H, br s), 6.08 (1H, d), 6.25 (1H, d), 7.02 (1H, s), 7.15 (3H, m), 7.28 (1H, d), 8.50 (1H, s) | 336 (100%, [M+H]+) |
| 149 | 1.40 (3H, d), 5.37 (1H, m), 6.60 (1H, d), 7.06 (1H, s), 7.10 (2H, m), 7.48 (5H, m), 7.60 (1H, t), 8.22 (1 H, d), 8.50 (1H, s). | 334 (100%, [M+2H]+) |
| 150 | 2.63 (3H, s), 4.62 (2H, d), 5.20 (1H, brs), 6.06 (1H, d), 6.27 (1H, d), 6.98 (1H, s), 7.28 (1H, d), 7.40 (5H, m) | 322 (100%, [M+H]+) |
| 152 | 1.35 (3H, t), 2.90 (2H, q), 4.75 (2H, d), 6.35 (1H, br s), 7.00 (1H, s), 7.10 (1H, m), 7.20 (1H, d), 7.50 (5H, s), 7.60 (1H, t), 8.25 (1H, d) | 347 (100%, [M+H]+) |
| 312 | 3.85 (3H, s), 4.65 (2H, d), 6.45 (1H, br s), 6.55 (1H, d), 7.00 (1H, m), 7.05 (1H, s), 7.10 (1H, d), 7.15 (1H, d), 7.30 (1H, m), 7.40 (1H, d), 7.50 (1H, t), 7.75 (1H, s), 7.80 (1H, m), 8.50 (1H, s) | 372 (100%, [M+H]+) |
| 313 | 4.75 (2H, d), 6.75 (1H, br s), 7.15 (3H, m), 7.20 (1H, m), 7.60 (3H, m), 7.80 (1H, d), 8.15 (1H, d), 8.60 (1H, s) | 387 (100%, [M+H]+) |
| 314 | 4.75 (2H, d), 6.05 (2H, s), 6.75 (1H, br s), 6.95 (3H, m), 7.05 (1H, s), 7.20 (2H, m), 7.60 (1H, t), 8.35 (1H, d), 8.50 (1H, s) | 363 (100%, [M+H]+) |
| 237 | 1.25 (3H, t), 3.90 (2H, s), 4.20 (2H, q), 4.70 (2H, d), 6.50 (1H, br s), 7.05 (1H, s), 7.10 (1H, m), 7.20 (1H, dd), 7.50 (5H, m), 7.60 (1H, t), 8.25 (1H, d) | 405 (100%, [M+H]+) |
| 275 | 3.20 (1H, m), 4.05 (4H, m), 4.40 (2H, br s), 4.65 (2H, d), 6.75 (1H, br s), 7.05 (1H, s), 7.15 (2H, m), 7.50 (5H, m), 7.60 (1H, m), 8.25 (1H, d) | 393 (100%, [M+H]+) |
| 250 | 3.10 (2H, t), 4.05 (2H, m), 4.65 (2H, d), 4.85 (1H, br s), 6.65 (1H, br s), 7.05 (1H, s), 7.15 (2H, m), 7.50 (5H, m), 7.60 (1H, m), 8.25 (1H, d) | 363 (100%, [M+H]+) |
| 287 | 2.80 (3H, d), 3.85 (2H, s), 4.70 (2H, d), 6.55 (1H, br s), 7.05 (1H, s), 7.15 (2H, m), 7.50 (6H, m), 7.60 (1H, m), 8.30 (1H, d) | 390 (100%, [M+H]+) |
| 302 | 2.40 (6H, s), 3.70 (2H, s), 4.75 (2H, d), 6.45 (1H, br s), 7.00 (1H, s), 7.10 (1H, m), 7.20 (1H, d), 7.50 (5H, br s), 7.60 (1H, t), 8.25 (1H, d) | 376 (100%, [M+H]+) |
| 303 | 2.65 (4H, m), 3.80 (6H, m), 4.75 (2H, d), 6.40 (1H, br s), 7.05 (1H, s), 7.10 (1H, m), 7.20 (1H, dd), 7.50 (5H, br s), 7.60 (1H, m), 8.30 (1H, d) | 418 (100%, [M+H]+) |
| 304 | 2.50 (3H, s), 3.90 (2H, s), 4.75 (2H, d), 6.50 (1H, br s), 7.05 (1H, s), 7.15 (1H, m), 7.20 (1H, d), 7.50 (6H, br s), 7.60 (1H, t), 8.30 (1H, d) | 362 (100%, [M+H]+) |
| 305 | 3.55 (3H, s), 4.60 (2H, s), 4.75 (2H, d), 6.55 (1H, br s), 7.05 (1H, s), 7.10 (1H, m), 7.20 (1H, d), 7.50 (5H, br s), 7.60 (1H, t), 8.25 (1H, d) | 363 (100%, [M+H]+) |
| 157 | 2.30 (3H, s), 4.65 (2H, d), 6.30 (1H, br s), 7.10 (2H, m), 7.40 (2H, m), 7.50 (3H, m), 7.60 (1H, t), 8.20 (1H, d), 8.50 (1H, s) | 333 (100%, [M+H]+) |
| 342 | 2.20 (3H, s), 3.80 (4H, m), 3.85 (4H, m), 4.50 (2H, d), 4.75 (2H, br s), 6.05 (1H, br s), 7.10 (2H, m), 7.35 (2H, m), 7.50 (3H, m), 7.55 (1H, m), 8.25 (1H, d) | 436 (100%, [M+H]+) |
| 343 | 2.20 (3H,s), 3.80 (4H, m), 3.90 (4H, m), 4.55 (2H, d), 4.75 (2H, br s), 6.00 (2H, s), 6.30 (1H, br s), 6.80 (2H, m), 6.95 (1H, d), 7.15 (2H, m), 7.60 (1H, t), 8.35 (1H, d) | 480 (100%, [M+H]+) |
| 344 | 2.20 (3H, s), 3.80 (4H, m), 3.90 (4H, m), 4.50 (2H, d), 4.75 (2H, br s), 6.20 (1H, br s), 7.15 (4H, m), 7.30 (2H, m), 7.55 (1H, t), 8.25 (1H, d) | 454 (100%, [M+H]+) |
| 148 | 1.30 (3H, s), 1.35 (3H, s), 3.10 (1H, q), 4.75 (2H, d), 6.25 (1H, br s), 7.00 (1H, s), 7.10 (1H, m), 7.20 (1H, d), 7.50 (5H, s), 7.60 (1H, t), 8.30 (1H, d) | 361 (100%, [M+H]+) |
| 212 | 3.62 (2H, m), 3.85 (2H, m), 4.37 (1H, br s), 4.62 (2H, d), 5.31 (1H, br t), 6.40 (1H, br s), 6.60 (1H, s), 7.16 (4H, m), 7.45 (2H, m), 7.62 (1H, m), 8.28 (1H, m). | 396 (100%, [M+H]+) |
| 208 | 3.80 (4H, m), 3.88 (4H, m), 4.50 (2H, br s), 4.60 (2H, d), 6.35 (1H, br s), 6.63 (1H, s), 7.14 (2H, m), 7.45 (5H, s), 7.59 (1H, m), 8.27 (1H, d). | 422 (100%, [M+H]+) |
| 318 | 2.48 (3H, s), 4.73 (2H, d), 6.55 (1H, br s), 7.05 (1H, s), 7.16 (2H, m), 7.28 (2H, m), 7.37 (2H, m), 7.60 (1H, m), 8.26 (1H, m), 8.53 (1H, s). | 333 (100%, [M+H]+) |
| 213 | 2.33 (3H, s), 3.62 (2H, m), 3.83 (2H, m), 4.47 (1H, br s), 4.62 (2H, d), 5.42 (1H, br t), 6.11 (1H, br s), 6.61 (1H, s), 6.95 (2H, m), 7.44 (6H, m). | 392 (100%, [M+H]+) |
| 217 | 2.35 (3H, s), 3.79 (4H, m), 3.87 (4H, m), 4.59 (2H, d), 4.62 (2H, br s), 6.14 (1H, br s), 6.61 (1H, s), 6.96 (2H, m), 7.45 (6H, m). | 436 (100%, [M+H]+) |

### Example 355

### Bioassays-Kv1.5 Rb⁺ Efflux Assay

The pore of a potassium channel is permeable to other monovalent cations such as Rb⁺ and Tl⁺. Analysis of cellular efflux of potassium channel permeable ions enables potassium channel activity to be monitored directly. Cells stably transfected with cDNA for human Kv1.5 (in pEF6::VA-His-TOPO) were grown in Dulbecco's Modified Eagle media (DMEM) alpha supplemented with 10% Fetal Calf Serum (FCS), 20 µl/ml penicillin (5000U/ml) streptomycin (5000µg/ml), 10µl/ml [100x] glutamine, and blasticidin (7.5µg/ml). Cells were dispensed into 96 well cellstar TC plates and allowed to grow until a confluent monolayer was visible. On the morning of the assay run, cold media was aspirated using the TECAN plate washer, and a 50µl media spike containing 2µCi/ml (37kBq/ml) ⁸⁶Rb⁺ added to each well containing cells using the Shallow-Well Matrix Platemate. Plates were placed in a incubator at 37°C for a minimum of 3 hours. Unloaded ⁸⁶Rb⁺ in the "hot" media was aspirated and each well washed 4x250µl with Earls Balanced Salt Solution (EBSS) which contained 5mM KCI, 140mM NaCl, 2mM CaCl₂, 1mM MgSO₄, 10mM HEPES, and 5mM glucose, pH 7.4, 290-300 mOsm. These cells were then pre-incubated with 50µl of EBSS +/- test compounds for 10 minutes at room temperature. After the 10 minute incubation, 50µl of modified EBSS which contained 145mM KCl, 2mM CaCl₂, 1mM MgSO₄, 10mM HEPES, 5mM glucose, pH 7.4, 290-300 mOsm, was added, and the cells were incubated for a further 10 minutes at room temperature. The high KCI EBSS was used to depolarise cells to a membrane potential that would activate Kv1.5 channels. Afterthe final incubation 80µl/1 00µl of the reaction from each well was transferred to equivalent wells in a Packard "Optiplate" white 96 well plate and counted in a Packard TopCount liquid scintillation counter by Cerenkov emission. Percentage inhibition ⁸⁶Rb⁺ efflux through Kv1.5 was calculated by normalisation to 2.5mM 4 aminopyridine control block of Kv1.5. Alternatively cells were loaded with ⁸⁵Rb+ and quantified by atomic absorption spectroscopy. IC₅₀ determinations were derived from 10 point concentration response curves n=2 using the method described above. Data was fitted as variable slope sigmoidal fit using Graphpad Prism (V3.02) software.

| Example | Compound | IC₅₀ (µM) |
|---|---|---|
| 83 | Furan-2-ylmethyl-(5-phenyl-thieno[2,3-d]pyrimidin-4-yl)-amine | 0.07 |
| 102 | (1-Ethyl-pyrrolidin-2-ylmethyl)-(5-phenyl-thieno[2,3-d]pyrimidin-4-yl)-amine | >30 |
| 103 | N,N-Dimethyl-2-(5-phenyl-thieno[2,3-d]pyrimidin-4-ylamino)-acetamide | >30 |
| 104 | (3-Imidazol-1-yl-propyl)-(5-phenyl-thieno[2,3-d]pyrimidin-4-yl)-amine | >30 |
| 105 | (5-Phenyl-thieno[2,3-d]pyrimidin-4-yl)-pyridin-2-ylmethyl-amine | 0.28 |
| 106 | 5-Phenyl-4-(pyridin-2-ylmethoxy)-thieno[2,3-d]pyrimidine | 0.33 |
| 84 | Cyclopropylmethyl-(5-phenyl-thieno[2,3-d]pyrimidin-4-yl)-amine | 0.80 |
| 107 | Furan-3-ylmethyl-(5-phenyl-thieno[2,3-d]pyrimidin-4-yl)-amine | 1.05 |
| 108 | (5-Methyl-furan-2-ylmethyl)-(5-phenyl-thieno[2,3-d]pyrimidin-4-yl)-amine | 0.41 |
| 109 | 4-(Furan-2-ylmethoxy)-5-phenyl-thieno[2,3-d]pyrimidine | 0.8 |
| 110 | (1-Methyl 1H-pyrrol-2-ylmethyl)-(5-phenyl-thieno[2,3-d]pyrimidin-4-yl)-amine | 3.90 |
| 85 | (5-Phenyl-thieno[2,3-d]pyrimidin-4-yl)-thiophen-2-ylmethyl-amine | 4.21 |
| 86 | Benzyl-(5-phenyl-thieno[2,3-d]pyrimidin-4-yl)-amine | 4.36 |
| 111 | (5-Phenyl-thieno[2,3-d]pyrimidin-4-yl)-(2-pyridin-2-yl-ethyl)-amine | 4.49 |
| 112 | (5-Phenyl-thieno[2,3-d]pyrimidin-4-ylamino)-acetic acid methyl ester | 5.03 |
| 87 | Allyl-(5-phenyl-thieno[2,3-d]pyrimidin-4-yl)-amine | 5.14 |
| 113 | (2-Methoxy-ethyl)-(5-phenyl-thieno[2,3-d]pyrimidin-4-yl)-amine | 5.27 |
| 114 | (5-Phenyl-thieno[2,3-d]pyrimidin-4-yl)-(tetrahydro-furan-2-ylmethyl)-amine | 5.18 |
| 88 | Furan-2-ylmethyl-methyl-(5-phenyl-thieno[2,3-d]pyrimidin-4-yl)-amine | 6.82 |
| 115 | Thiophen-2-ylmethyl-(5-thiophen-2-yl-thieno[2,3-d]pyrimidin-4-yl)-amine | 7.07 |
| 116 | [1,3]Dioxolan-2-ylmethyl-(5-phenyl-thieno[2,3-d]pyrimidin-4- yl)-amine | 6.16 |
| 89 | Cyclohexylmethyl-(5-phenyl-thieno[2,3-d]pyrimidin-4-yl)-amine | 8.10 |
| 117 | 4-(Furan-2-ylmethylsulfanyl)-5-phenyl-thieno[2,3-d]pyrimidine | 9.37 |
| 118 | (5-Phenyl-thieno[2,3-d]pyrimidin-4-yl)-(2-thlophen-2-yl-ethyl)-amine | 8.56 |
| 90 | Phenethyl-(5-phenyl-thieno[2,3-d]pyrimidin-4-yl)-amine | 10.09 |
| 91 | Cyclohexyl-(5-phenyl-thieno[2,3-d]pyrimidin-4-yl)-amine | >30 |
| 119 | 4-Benzyloxy-5-phenyl-thieno[2,3-d]pyrimidine | 5.79 |
| 120 | 5-Phenyl-4-(pyridin-2-ylmethylsulfanyl)-thieno[2,3-d]pyrimidine | 12.73 |
| 121 | [5-(5-Methyl-pyridin-2-yl)-thieno[2,3-d]pyrimidin-4-yl]-pyridin-2-ylmethyl-amine | 2.09 |
| 122 | Furan-2-ylmethyl-[5-(5-methyl-pyridin-2-yl)-thieno[2,3-d]pyrimidin-4-yl]-amine | 3.53 |
| 123 | [5-(4-Fluorophenyl)-thieno[2,3-d]pyrimidin-4-yl]-pyridin-2-ylmethyl-amine | 0.05 |
| 124 | [5-(4-Fluorophenyl)-thieno[2,3-d]pyrimidin-4-yl]-furan-2-ylmethyl-amine | 1.16 |
| 92 | Furan-2-ylmethyl-(5-thiophen-2-yl-thieno[2,3-d]pyrimidin-4-yl)-amine | 1.91 |
| 93 | (4-Nitrobenzyl)-(5-thiophen-2-yl-thieno[2,3-d]pyrimidin-4-yl)-amine | >30 |
| 94 | (5-Thiophen-2-yl-thieno[2,3-d]pyrimidin-4-yl)-(3,4,5-timethoxy-benzyl)-amine | >30 |
| 95 | Cyclopropylmethyl-(5-thiophen-2-yl-thieno[2,3-d]pyrimidin-4-yl)-amine | 4.51 |
| 96 | Isobutyl-(5-thiophen-2-yl-thieno[2,3-d]pyimidin-4-yl)-amine | 6.32 |
| 97 | Benzyl-(5-thiophen-2-yl-thieno[2,3-d]pyrimidln-4-yl)-amine | 6.53 |
| 98 | Thiophen-2-ylmethyl-(5-thiophen-2-yl-thieno[2,3-d]pyrimidin-4-yl)-amine | 6.95 |
| 99 | Allyl-(5-thiophen-2-yl-thieno[2,3-d]pyrimidin-4-yl)-amine | 7.53 |
| 100 | Furan-2-ylmethyl-methyl-(5-thiophen-2-yl-thieno[2,3-d]pyrimidin-4-yl)-amine | 9.71 |
| 125 | [2-(4-Chloro-phenyl)-ethyl]-(5-methyl-thieno[2,3-d]pyrimidin-4-yl)-amine | 6.91 |
| 126 | Furan-2-ylmethyl-(5-p-tolyl-thieno[2,3-d]pyrimidin-4-yl)-amine | 0.47 |
| 127 | [2-(3,4-Dimethoxy-phenyl)-ethyl]-(5-p-tolyl-thieno[2,3-d]pyrimidin-4-yl)-amine | 5.3 |
| 128 | 1-[2-(5-p-Tolyl-thieno[2,3-d]pyrimidin-4-ylamino)-ethyl]-imidazolidin-2-one | >30 |
| 129 | 4-(Naphthalen-2-yloxy)-5-p-tolyl-thieno[2,3-d]pyrimidine | >30 |
| 130 | Phenethyl-(5-p-tolyl-thieno[2,3-d]pyrimidin-4-yl)-amine | 8.05 |
| 131 | 4-(5-p-Tolyl-thieno[2,3-d]pyrimidin-4-yloxy)-benzoic acid methyl ester | >30 |
| 132 | [2-(2,3-Dihydro-benzo[1,4]dioxin-6-yl)-ethyl]-[5-(3,4-dimethyl-phenyl)-thieno[2,3-d] pyrimidin-4-yl]-amine | 4.58 |
| 133 | [5-(3,4-Dimethyl-phenyl)-thieno[2,3-d]pyrimidin-4-yl]-furan-2-ylmethyl-amine | 0.67 |
| 135 | [5-(4-Chlorophenyl)-thieno[2,3-d]pyrimidin-4-yl]-furan-2-ylmethyl-amine | 0.93 |
| 136 | [5-(4-Chlorophenyl)-thieno[2,3-d]pyrimidin-4-yl]-phenethyl-amine | 16.8 |
| 137 | [5-(4-Bromophenyl)-thieno[2,3-d]pyrimidin-4-yl]-[2-(2,3-dihydro-benzo[1,4]dioxin-6-yl)-ethyl]-amine | 10.68 |
| 138 | [5-(4-Methoxy-phenyl)-thieno[2,3-d]pyrimidin-4-yl]-pyridin-2-ylmethyl-amine | 0.22 |
| 139 | Furan-2-ylmethyl-[5-(4-methoxy-phenyl)-thieno[2,3-d]pyrimidin-4-yl]-amine | 1.68 |
| 140 | (6-Ethyl-5-phenyl-thieno[2,3-d]pyrimidin-4-yl)-furan-2-ylmethyl-amine | 2.71 |
| 141 | (6-Ethyl-5-furan-3-yl-thieno[2,3-d]pyrimidin-4-yl)-furan-2-ylmethyl-amine | 2.15 |
| 142 | Pyridin-2-ylmethyl-(5-o-tolyl-thieno[2,3-d]pyfimidin-4-yl)-amine | 1.28 |
| 143 | Furan-2-ylmethyl-(5-o-tolyl-thieno[2,3-d]pyrimidin-4-yl)-amine | 1.32 |
| 144 | [5-(4-tert-Butyl-phenyl)-thieno[2,3-d]pyritnidan-4-yl]-pyridin-2-ylmethyl-amine | 1.32 |
| 145 | [5-(4-tert-Butyl-phenyl)-thieno[2,3-d]pyrimidin-4-yl]-furan-2-ylmethyl-amine | 3.05 |
| 146 | (5-Cyclohexyl-thieno[2,3-d]pyrimidin-4-yl)-pyridin-2-ylmethyl-amine | 1.68 |
| 147 | (5-Cyclohexyl-thieno[2,3-d]pyrimidin-4-yl)-furan-2-ylmethyl-amine | 2.52 |
| 148 | (2-Isopropyl-5-phenyl-thieno[2,3-d]pyrimidin-4-yl)-pyridin-2-ylmethyl-amine | 1.51 |
| 149 | (5-Phenyl-thieno[2,3-d]pyrimidin-4-yl)-(1-pyridin-2-yl-ethyl)-amine | 0.37 |
| 150 | Furan-2-ylmethyl-(2-methyl-5-phenyl-thieno[2,3-d]pyrimidin-4-yl)-amine | 0.14 |
| 151 | (5-Phenyl-2-trifluoromethyl-thieno[2,3-d]pyrimidin-4-yl)-pyridin-2-ylmethyl-amine | 1.68 |
| 181 | (2-Chloro-5-phenyl-thieno[2,3-d]pyrimidin-4-yl)-pyridin-2-ylmethyl-amine | 4.13 |
| 182 | (2-Chloro-5-phenyl-thieno[2,3-d]pyrimidin-4-yl)-furan-2-ylmethyl-amine | 1.04 |
| 188 | N²-Cyclopropylmethyl-5-phenyl-N⁴-pyridin-2-ylmethyl-thieno[2,3-d]pyrimidine-2,4-diamine | 2.52 |
| 189 | N²-(2-Methoxy-ethyl)-5-phenyl-N⁴-pyridin-2-ylmethyl-thieno[2,3-d]pyrimidine-2,4-diamine | 0.28 |
| 190 | 2-{5-Phenyl-4-[(pyridin-2-ylmethyl)-amino]-thieno[2,3-d]pyrimidin-2-ylamino} -ethanol | 0.26 |
| 191 | (2-Metlloxy-5-phenyl-thieno[2,3-d]pyrinlidin-4-yl)-pyridin-2-ylmethyl-amine | 0.47 |
| 192 | 5-Phenyl-N⁴-pyridin-2-ylmethyl-thieno[2,3-d]pyrimidine-2,4-diamine | 2.18 |
| 193 | N²,N⁴-Bis-furan-2-ylmethyl-5-phenyl-thieno[2,3- d]pyrimidine-2,4-diamine | 2.5 |
| 194 | N⁴-Furan-2-ylmethyl-5-phenyl-N²-pyridin-2-ylmethyl-thieno[2,3-d]pyimidine-2,4-diamine | 4.16 |
| 195 | (2-Benzyloxy-5-phenyl-thieno[2,3-d]pyrimidin-4-yl)-furan-2-ylmethyl-amine | 6.39 |
| 196 | N²-Methyl-5-phenyl-N⁴-pyridin-2-ylmethyl-thieno[2,3-d]pyrimidine-2,4-diamine | 0.04 |
| 197 | (2-Morpholin-4-yl-5-phenyl-thieno[2,3-d]pyrimidin-4-yl)-pyridin-2-ylmethyl-amine | 0.15 |
| 198 | N²,N²-Dimethyl-5-phenyl-N⁴-pyridin-2-ylmethyl-thieno[2,3-d]pyrimidine-2,4-diamine | 0.57 |
| 152 | (2-Ethyl-5-phenyl-thieno[2,3-d]pyrimidin-4-yl)-pyridin-2-ylmethyl-amine | 0.39 |
| 153 | (2-Ethyl-5-phenyl-thieno[2,3-d]pyrimidin-4-yl)-furan-2-ylmethyl-amine | 3.63 |
| 154 | [5-(4-Fluorophenyl)-2-methyl-thieno[2,3-d]pyrimidin-4-yl]-pyridin-2-ylmethyl-amine | 1.62 |
| 157 | (6-Methyl-5-phenyl-thieno[2,3-d]pyrimidin-4-yl)-pyridin-2-ylmethyl-amine | 0.24 |
| 158 | (5-Phenyl-thieno[2,3-d]pyrimidin-4-yl)-(1,2,3,4-tetrahydro-naphthalen-1-yl)-amine | 17.70 |
| 159 | 6-Methyl-5-phenyl-4-piperidin-1-yl-thieno[2,3-d]pyrimidine | 13.36 |
| 161 | (5-Phenyl-thieno[2,3-d]pyrimidin-4-yl)-(2-thiophen-2-yl-thiazol-4-ylmethyl)-amine | 25.46 |
| 162 | (2-Phenyl-thiazol-4-ylmethyl)-(5-phenyl-thieno[2,3-d]pyrimidin-4-yl)-amine | 19.72 |
| 163 | Phenethyl-(5-thiophen-2-yl-thieno[2,3-d]pyrimidin-4-yl)-amine | 14.32 |
| 164 | (6-Bromo-5-phenyl-thieno[2,3-d]pyrimidin-4-yl)-pyridin-2-ylmethyl-amine | 0.53 |
| 165 | (6-Bromo-5-phenyl-thieno[2,3-d]pyrimidin-4-yl)-furan-2-ylmethyl-amine | 1.6 6 |
| 199 | 5-Phenyl-4-[(pyridin-2-ylmethyl)-amino]-thieno[2,3-d]pyrimidine-2-carbonitrile | 6.61 |
| 200 | 5-(4-Fluorophenyl)-N²,N²-dimethyl-N⁴-pyridin-2-ylmethyl-thieno[2,3-d]pyrimidine-2,4-diamine | 71.77 |
| 201 | 1-{5-(4-Fluorophenyl)-4-[(pyridin-2-ylmethyl)-amino]-thieno [2,3-d]pyrimidin-2-yl}-piperidine-4-carboxylic acid methyl ester | 15.87 |
| 202 | 3-{5-Phenyl-4-[(pyridin-2-ylmethyl)-amino]-thieno[2,3-d]pyrimidin-2-ylamino}-propionic acid ethyl ester | 2.7 |
| 203 | [2-(2-Methoxy-ethoxy)-5-phenyl-thieno[2,3-d]pyrimidin-4-yl]-pyridin-2-ylmethyl-amine | 0.48 |
| 204 | 5-(4-Fluorophenyl)-N²-methyl-N⁴-pyridin-2-ylmethyl-thieno[2,3-d]pyrimidine-2,4-diamine | 4.7 |
| 205 | 5-(4-Fluorophenyl)-N⁴-pyridin-2-ylmethyl-thieno[2,3-d]pyrimidine-2,4-diamine | 11.87 |
| 206 | 2-(1-(5-Phenyl-4-[(pyridin-2-ylmethyl)-amino]-thieno[2,3-d]pyrimidin-2-yl)-piperidin-4-yl)-ethanol | 3.41 |
| 207 | [5-(4-Fluorophenyl)-2-morpholin-4-yl-thieno[2,3-d]pyrimidin-4-yl]-py/din-2-ylmethyl-amine | 42.5 |
| 208 | 2-((2-Hydroxy-ethyl)-{5-phenyl-4-[(pyridin-2-ylmethyl)-amino]-thieno[2,3-d]pyrimidin-2-yl}-amino)-ethanol | 0.33 |
| 209 | 5-(4-Fluorophenyl)-N²-(2-methoxy-ethyl)-N⁴-pyridin-2-ylmethyl-thieno[2,3-d]pyrimidine-2,4-diamine | 1.83 |
| 210 | 2-{5-Phenyl-4-[(pyridin-2-ylmethyl)-amino]-thieno[2,3-d]pyrimidin-2-ylamino}-propane-1,3-diol | 0.74 |
| 211 | [2-(2-Dimethylamino-ethoxy)-5-phenyl-thieno[2,3-d]pyrimidin-4-yl]-pyridin-2-ylmethyl-amine | 5.37 |
| 212 | 2-{5-(4-Fluorophenyl)-4-[(pyridin-2-ylmethyl)-amino]-thieno[2,3-d]pyrimidin-2-ylamino}-ethanol | 7.63 |
| 237 | {5-Phenyl-4-[(pyridin-2-ylmethyl)amino]thieno[2,3-d]pyrimidin-2-yl }acetic acid ethyl ester | 0.54 |
| 238 | {4-[(Furan-2-ylmethyl)-amino]-5-phenyl-thieno[2,3-d]pyrimidin-2-yl}-acetic acid ethyl ester | 3.33 |
| 250 | 2-{5-Phenyl-4-[(pyridin-2-ylmethyl)amino]thieno[2,3-d]pyrimidin-2-yl}ethanol | 0.33 |
| 251 | 2-{4-[(Furan-2-ylmethyl)-amino]-5-phenyl-thieno[2,3-d]pyrimidin-2-yl}-ethanol | 0.08 |
| 275 | 2-{5-Phenyl-4-[(pyridin-2-ylmethyl)-amino]-thieno[2,3-d]pyrimidin-2-yl}-propane-1,3-diol | 1.53 |
| 313 | Pyridin-2-ylmethyl-[5-(4-trifluoromethyl-phenyl)-thieno[2,3-d]pyrimidin-4-yl]-amine | 2.90 |
| 314 | (5-Benzo[1,3]dioxol-5-yl-thieno[2,3-d]pyrimidin-4-yl)-pyridin-2-ylmethyl-amine | 0.01 |
| 320 | [5-(2,3-Dihydro-benzo[1,4]dioxin-6-yl)-thieno[2,3-d]pyrimidin-4-yl]-pyridin-2-ylmethyl-amine | 0.37 |
| 321 | [5-(3-Chlorophenyl)-thieno[2,3-d]pyrimidin-4-yl]-pyridin-2-ylmethyl-amine | 1.52 |
| 322 | [5-(3-Methoxyphenyl)-thieno[2,3-d]pyrimidin-4-yl]-pyridin-2-ylmethyl-amine | 0.26 |
| 323 | [5-(1 H-Indol-6-yl)-thieno[2,3-d]pyrimidin-4-yl]-pyridin-2-ylmethyl-amine | 0.04 |
| 325 | [5-(4-Chlorophenyl)-thieno[2,3-d]pyrimidin-4-yl]-pyridin-2-ylmethyl-amine | 2.54 |
| 326 | [5-(2,2-Difluoro-benzo[1,3]dioxol-5-yl)-thieno[2,3-d]pyrimidin-4-yl]-pyridin-2-ylmethyl-amine | 10.43 |
| 331 | [5-(3-Fluorophenyl)-thieno[2,3-d]pyrimidin-4-yl]-pyridin-2-ylmethyl-amine | 0.03 |
| 332 | [5-(4-Morpholin-4-yl-phenyl)-thieno[2,3-d]pyrimidln-4-yl]-pyridin-2-ylmethyl-amine | 47.43 |
| 333 | [5-(3,4-Difluorophenyl)-thieno[2,3-d]pyrimidin-4-yl]-pyridin-2-ylmethyl-amine | 10.36 |

### Example 356

### Kv1.5 Autopatch Electrophysiology Method

The external bathing solution contained (in mM): 150 NaCl, 10 KCl, 100 Potassium Gluconate, 3 MgCl₂, 1 CaCl₂, 10 HEPES, pH 7.4. Patch pipettes were filled with an electrode solution of composition (in mM): 160 KCI, 0.5 MgCl₂, 10 HEPES, 1 EGTA, pH 7.4 with KOH.

Compounds were dissolved in DMSO (100 %) and made up in the external bather at a concentration of 1 µM. All experiments were conducted at room temperature (22-24°C).

A cell suspension (10 ml), with a density of 100,000 cells/ml, was aliquoted into a 15ml centrifuge tube and transferred to an incubator (37°C, 5% CO₂) for approximately one hour before use. Following 60 min incubation, a tube was taken and centrifuged at 1000 rpm for 4 mins at room temperature. 9.5 ml supernatant was thence discarded, leaving a cell pellet at the bottom of the tube. The pellet was then resuspended using 100 µl of cold (4 °C), filtered (0.22 µm), 0.2 % BSA/bather solution (0.02g BSA/10ml bather). The bottom of the tube was manually agitated gently until the solution became cloudy with cells. The 100 µl cell resuspension solution was then stored on the bench at 4 °C (using a Peltier-based temperature control device) until used.

A length of capillary glass (1 B150F-4, WPI) was dipped into the cell suspension solution, such that - 3 cm column of fluid was taken up by capillary action. A Ag/AgCl wire was dropped into the non-dipped end of the capillary also. The outside of the solution-filled end of the capillary was then dried and the capillary was loaded into the AutoPatch™. Borosilicate glass patch pipettes (from 1.5mm OD, thin-walled filamented, GC150-TF capillary glass, Harvard) were pulled using a DMZ pipette puller (Zeitz Instruments), and were back-filled using the internal pipette solution, being careful that no bubbles remain at the tip or in the body of the pipette. Patch pipettes typically had resistances of 2.3-3.5 MΩ. Once filled, the pipette tip and a proportion of the shaft (-15 mm) were dipped into Sigmacote (Sigma). The recording pipette was then loaded into the AutoPatch™.

Automated patch-clamping was initiated by the operator, but thereafter AutoPatch.execontinued the experiment providing that pre-set conditions and criteria were satisfied.

Whole cell patch-clamp recordings were made using the AutoPatch™ rig, which incorporated an EPC9 amplifier (HEKA, Germany) under control of Pulse software (v8.54, HEKA, Germany), a motion controller with 2 translators (Newport, UK), valve controller (VF1) and a c-level suction device all at room temperature (22-24°C). This equipment was completely under the control of AutoPatch.exe and operator intervention was only made when there was a requirement to refill the drug reservoirs or to prevent the loss of a cell due to a technical error. Cells with an Rₛₑᵣᵢₑₛ greater than 18 MΩ were discounted from the experiment.

Qualification stages prior to perfusion and drug application ensured that the observed current met the criteria for the experiment. Only those cells with an I_{K} > 500 pA were used for experiments. Cells were continuously perfused with external solution at a flow rate of 1.8-2 ml/minute. The perfusion chamber had a working volume of 80-85µl and allowed for rapid exchange of drug solutions. Online analysis of the hKᵥ1.5 current during the application of compounds was performed by the AutoPatch™ software. Voltage-step protocols and analysis of data was performed as described for conventional electrophysiology.

Electrophysiology voltage-step protocols and analysis of data was performed as follows. Data was sampled at 5kHz, and filtered with a -3 dB bandwidth of 2.5kHz. Cells were held at a voltage of -80mV. Currents were evoked to a voltage step for 1000ms in duration at OmV every 5s. Currents were analysed using Pulsefit software (v8.54, HEKA, Germany), with the total charge measured during the whole of the voltage step. All other plots were produced using Igor Pro (WaveMetrics).

| Example | Compound | % Inhibition at 1 µM |
|---|---|---|
| 83 | Furan-2-ylmethyl-(5-phenyl-thieno[2,3-d]pyrimidin-4-yl)-amine | 21.9 |
| 103 | N,N-Dimethyl-2-(5-phenyl-thieno[2,3-d]pyrimidin-4-ylamino)-acetamide | 13.6 |
| 105 | (5-Phenyl-thieno[2,3-d]pyrimidin-4-yl)-pyridin-2-ylmethyl-amine | 63.0 |
| 106 | 5-Phenyl-4-(pyridin-2-ylmethoxy)-thieno[2,3-d]pyrimidine | 21.9 |
| 84 | Cyclopropylmethyl-(5-phenyl-thieno[2,3-d]pyrimidin-4-yl)-amine | 15.3 |
| 107 | Furan-3-ylmethyl-(5-phenyl-thieno[2,3-d]pyrimidin-4-yl)-amine | 25.3 |
| 108 | (5-Methyl-furan-2-ylmethyl)-(5-phenyl-thieno[2,3-d]pyrimidin-4-yl)-amine | 37.2 |
| 109 | 4-(Furan-2-ylmethoxy)-5-phenyl-thieno[2,3-d]pyrimidine | 52.3 |
| 110 | (1-Methyl-1H-pyrrol-2-ylmethyl)-(5-phenyl-thieno[2,3-d]pyrimidin-4-yl)-amine | 14.2 |
| 85 | (5-Phenyl-thieno[2,3-d]pyrimidin-4-yl)-thiophen-2-ylmethyl-amine | 24.2 |
| 86 | Benzyl-(5-phenyl-thieno[2,3-d]pyrimidin-4-yl)-amine | 46.0 |
| 111 | (5-Phenyl-thieno[2,3-d]pyrimidin-4-yl)-(2-pyridin-2-yl-ethyl)-amine | 40.0 |
| 112 | (5-Phenyl-thieno[2,3-d]pyrimidin-4-ylamino)-acetic acid methyl ester | 43.5 |
| 87 | Allyl-(5-phenyl-thieno[2,3-d]pyrimidin-4-yl)-amine | 3.4 |
| 111 | (2-Methoxy-ethyl)-(5-phenyl-thieno[2,3-d]pyrimidin-4-yl)-amine | 22.2 |
| 88 | Furan-2-ylmethyl-methyl-(5-phenyl-thieno[2,3-d]pyrimidin-4-yl)-amine | 26.4 |
| 115 | Thiophen-2-ylmethyl-(5-thiophen-2-yl-thieno[2,3-d]pyrimidin-4-yl)-amine | 45.9 |
| 89 | Cyclohexylmethyl-(5-phenyl-thieno[2,3-d]pyrimidin-4-yl)-amine | 73.4 |
| 117 | 4-(Furan-2-ylmethylsulfanyl)-5-phenyl-thieno[2,3-d]pyrimidine | 43.6 |
| 118 | (5-Phenyl-thieno[2,3-d]pyrimidin-4-yl)-(2-thiophen-2-yl-ethyl)-amine | 15.8 |
| 89 | Cyclohexyl-(5-phenyl-thieno[2,3-d]pyrimidin-4-yl)-amine | 42.2 |
| 117 | 4-Benzyloxy-5-phenyl-thieno[2,3-d]pyrimidine | 57.2 |
| 119 | [5-(5-Methyl-pyridin-2-yl)-thieno[2,3-d]pyrimidin-4-yl]-pyridin-2-ylmethyl-amine | 44.1 |
| 120 | Furan-2-ylmethyl-[5-(5-methyl-pyridin-2-yl)-thieno[2,3-d]pyrimidin-4-yl]-amine | 25.0 |
| 121 | [5-(4-Fluorophenyl)-thieno[2,3-d]pyrimidin-4-yl]-pyridin-2-ylmethyl-amine | 82.6 |
| 122 | [5-(4-Fluorophenyl)-thieno[2,3-d]pyrimidin-4-yl]-furan-2-ylmethyl-amine | 36.3 |
| 92 | Furan-2-ylmethyl-(5-thiopen-2-yl-thieno[2,3-d]pyrimidin-4-yl)-amine | 27.6 |
| 93 | (4-Nitrobenzyl)-(5-thiophen-2-yl-thieno[2,3-d]pyrimidin-4-yl)-amine | 44.0 |
| 94 | (5-Thiophen-2-yl-thieno[2,3-d]pyrimidin-4-yl)-(3,4,5-trimethoxy-benzyl)-amine | 19.4 |
| 95 | Cyclopropylmethyl-(5-thiophen-2-yl-thieno[2,3-d]pyrimidin-4-yl)-amine | 32.6 |
| 96 | Isobutyl-(5-thiophen-2-yl-thieno[2,3-d]pyrimidin-4-yl)-amine | 22.9 |
| 97 | Benzyl-(5-thiophen-2-yl-thieno[2,3-d]pyrimidin-4-yl)-amine | 43.3 |
| 98 | Thiophen-2-ylmethyl-(5-thiophen-2-yl-thieno[2,3-d]pyrimidin-4-yl)-amine | 21.0 |
| 99 | Allyl-(5-thiophen-2-yl-thieno[2,3-d]pyrimidin-4-yl)-amine | 17.5 |
| 100 | Furan-2-ylmethyl-methyl-(5-thiophen-2-yl-thieno[2,3-d]pyrimidin-4-yl)-amine | 19.9 |
| 126 | Furan-2-ylmethyl-(5-p-tolyl-thieno[2,3-d]pyrimidin-4-yl)-amine | 83.4 |
| 127 | [2-(3,4-Dimethoxyphenyl)-ethyl]-(5-p-tolyl-thieno[2,3-d]pyrimidin-4-yl)-amine | 20.7 |
| 128 | 1-[2-(5-p-Tolyl-thieno[2,3-d]pyrimidin-4-ylamino)-ethyl]-imidazolidin-2-one | 25.0 |
| 129 | 4-(Naphthalen-2-yloxy)-5-p-tolyl-thieno[2,3-d]pyrimidine | 47.4 |
| 131 | 4-(5-p-Tolyl-thieno[2,3-d]pyrimidin-4-yloxy)-benzoic acid methyl ester | 29.8 |
| 132 | [2-(2,3-Dihydro-benzo[1,4]dioxin-6-yl)-ethyl]-[5-(3,4-dimethyl-phenyl)-thieno[2,3-d]pyrimidin-4-yl]-amine | 92.0 |
| 133 | [5-(3,4-Dimethyl-phenyl)-thieno[2,3-d]pyrimidin-4-yl]-furan-2-ylmethyl-amine | 77.0 |
| 135 | [5-(4-Chlorophenyl)-thieno[2,3-d]pyrimidin-4-yl]-furan-2-ylmethyl-amine | 84.8 |
| 136 | [5-(4-Chlorophenyl)-thieno[2,3-d]pyrimidin-4-yl]-phenethyl-amine | 27.2 |
| 137 | [5-(4-Bromophenyl)-thieno[2,3-d]pyrimidin-4-yl]-[2-(2,3-dihydro-benzo[1,4] dioxin-6-yl)-ethyl]-amine | 96.0 |
| 138 | [5-(4-Methoxyphenyl)-thieno[2,3-d]pyrimidin-4-yl]-pyridin-2-ylmethyl-amine | 92.6 |
| 139 | Furan-2-ylmethyl-[5-(4-methoxy-phenyl)-thieno[2,3-d]pyrimidin-4-yl]-amine | 46.9 |
| 140 | (6-Ethyl-5-phenyl-thieno[2,3-d]pyrimidin-4-yl)-furan-2-ylmethyl-amine | 74.2 |
| 141 | (6-Ethyl-5-furan-3-yl-thieno[2,3-d]pyrimidin-4-yl)-furan-2-ylmethyl-amine | 81.3 |
| 142 | Pyridin-2-ylmethyl-(5-o-tolyl-thieno[2,3-d]pyrimidin-4-yl)-amine | 49.7 |
| 143 | Furan-2-ylmethyl-(5-o-tolyl-thieno[2,3-d]pyrimidin-4-yl)-amine | 63.0 |
| 144 | [5-(4-tert-Butyl-phenyl)-thieno[2,3-d]pyrimidin-4-yl]-pyridin-2-ylmethyl-amine | 81.0 |
| 145 | [5-(4-tert-Butyl-phenyl)-thieno[2,3-d]pyrimidin-4-yl]-furan-2-ylmethyl-amine | 68.0 |
| 146 | (5-Cyclohexyl-thieno[2,3-d]pyrimidin-4-yl)-pyridin-2-ylmethyl-amine | 81.0 |
| 147 | (5-Cyclohexyl-thieno[2,3-d]pyrimidin-4-yl)-furan-2-ylmethyl-amine | 71.0 |
| 148 | (2-Isopropyl-5-phenyl-thieno[2,3-d]pyrimidin-4-yl)-pyridin-2-ylmethyl-amine | 88.0 |
| 149 | (5-Phenyl-thieno[2,3-d]pyrimidin-4-yl)-(1-pyridin-2-yl-ethyl)-amine | 78.0 |
| 150 | Furan-2-ylmethyl-(2-methyl-5-phenyl-thieno[2,3-d]pyrimidin-4-yl)-amine | 96.8 |
| 151 | (5-Phenyl-2-trifluoromethyl-thieno[2,3-d]pyrimidin-4-yl)-pyridin-2-ylmethyl-amine | 95.0 |
| 181 | (2-Chloro-5-phenyl-thieno[2,3-d]pyrimidin-4-yl)-pyridin-2-ylmethyl-amine | 96.0 |
| 182 | (2-Chloro-5-phenyl-thieno[2,3-d]pyrimidin-4-yl)-furan-2-ylmethyl-amine | 75.8 |
| 188 | N²-Cyclopropylmethyl-5-phenyl-N⁴-pyridin-2-ylmethyl-thieno[2,3-d]pyrimidine-2,4-diamine | 98.0 |
| 189 | N²-(2-Methoxy-ethyl)-5-phenyl-N4-pyridin-2-ylmethyl-thieno[2,3-d]pyrimidine-2,4-diamine | 97.4 |
| 190 | 2-{5-Phenyl-4-[(pyridin-2-ylmethyl)-amino]-thieno[2,3-d]pyrimidin-2-ylamino }-ethanol | 97.1 |
| 191 | (2-Methoxy-5-phenyl-thieno[2,3-d]pyrimidin-4-yl)-pyridin-2-ylmethyl-amine | 91.8 |
| 192 | 5-Phenyl-N⁴-pyridin-2-ylmethyl-thieno[2,3-d]pyrimidine-2,4-diamine | 90.0 |
| 193 | N²,N⁴-Bis-furan-2-ylmethyl-5-phenyl-thieno[2,3-d]pyrimidine-2,4-diamine | 89.2 |
| 194 | N⁴-Furan-2-ylmethyl-5-phenyl-N²-pyridin-2-ylmethyl-thieno[2,3-d]pyrimidine-2,4-diamine | 81.0 |
| 195 | (2-Benzyloxy-5-phenyl-thieno[2,3-d]pyrimidin-4-yl)-furan-2-ylmethyl-amine | 90.0 |
| 196 | N²-Methyl-5-phenyl-N⁴-pyridin-2-ylmethyl-thieno[2,3-d]pyrimidine-2,4-diamine | 97.0 |
| 197 | (2-Morpholin-4-yl-5-phenyl-thieno[2,3-d]pyrimidin-4-yl)-pyridin-2-ylmethyl-amine | 98.0 |
| 198 | N²,N²-Dimethyl-5-phenyl-N⁴-pyridin-2-ylmethyl-thieno[2,3-d]pyrimidine-2,4-diamine | 98.1 |
| 152 | (2-Ethyl-5-phenyl-thieno[2,3-d]pyrimidin-4-yl)-pyridin-2-ylmethyl-amine | 98.7 |
| 153 | (2-Ethyl-5-phenyl-thieno[2,3-d]pyrimidin-4-yl)-furan-2-ylmethyl-amine | 96.8 |
| 154 | (6-Methylpyridin-2-ylmethyl)-(5-phenyl-thieno[2,3-d]pyrimidin-4-yl)-amine | 93.0 |
| 155 | [5-(4-Fluorophenyl)-2-methyl-thieno[2,3-d]pyrimidin-4-yl]-pyridin-2-ylmethyl-amine | 92.2 |
| 156 | (6-Methyl-pyridin-2-ylmethyl)-(5-phenyl-thieno[2,3-d]pyrimidin-4-yl)-amine | 92.0 |
| 157 | (6-Methyl-5-phenyl-thieno[2,3-d]pyrimidin-4-yl)-pyridin-2-ylmethyl-amine | 88.0 |
| 158 | (5-Phenyl-thieno[2,3-d]pyrimidin-4-yl)-(1,2,3,4-tetrahydro-naphthalen-1-yl)-amine | 83.4 |
| 159 | 6-Methyl-5-phenyl-4-piperidin-1-yl-thieno[2,3-d]pyrimidine | 83.0 |
| 160 | 2-[(5-Phenyl-thieno[2,3-d]pyrimidin-4-ylamino)-methyl]-nicotinic acid ethyl ester | 80.0 |
| 161 | (5-Phenyl-thieno[2,3-d]pyrimidin-4-yl)-(2-thiophen-2-yl-thiazol-4-ylmethyl)-amine | 79.4 |
| 162 | (2-Phenyl-thiazol-4-ylmethyl)-(5-phenyl-thieno[2,3-d]pyrimidin-4-yl)-amine | 74.5 |
| 163 | Phenethyl-(5-thiophen-2-yl-thieno[2,3-d]pyrimidin-4-yl)-amine | 70.8 |
| 164 | (6-Bromo-5-phenyl-thieno[2,3-d]pyrimidin-4-yl)-pyridin-2-ylmethyl-amine | 92.8 |
| 165 | (6-Bromo-5-phenyl-thieno[2,3-d]pyrimidin-4-yl)-furan-2-ylmethyl-amine | 91.0 |
| 199 | 5-Phenyl-4-[(pyridin-2-ylmethyl)-amino]-thieno[2,3-d]pyrimidine-2-carbonitrile | 99.5 |
| 200 | 5-(4-Fluorophenyl)-N²,N²-dimethyl-N⁴-pylidin-2-ylmethyl-thieno[2,3-d] pyrimidine-2,4-diamine | 99.3 |
| 201 | 1 -{5-(4-Fluorophenyl)-4-[(pyridin-2-ylmethyl)-amino]-thieno[2,3-d]pyrimidin-2-yl}-piperidine-4-carboxylicacid methyl ester | 98.2 |
| 202 | 3-{5-Phenyl-4-[(pyridin-2-ylmethyl)-amino]-thieno[2,3-d]pyrimidin-2-ylamino}-propionic acid ethyl ester | 98.0 |
| 203 | [2-(2-Methoxy-ethoxy)-5-phenyl-thieno[2,3-d]pyrimidin-4-yl]-pyridin-2-ylmethyl-amine | 98.0 |
| 204 | 5-(4-Fluorophenyl)-N²-methyl-N⁴-pyridin-2-ylmethyl-thieno[2,3-d]pyrimidine-2,4-diamine | 97.9 |
| 205 | 5-(4-Fluorophenyl)-N⁴-pyridin-2-ylmethyl-thieno[2,3-d]pyrimidine-2,4-diamine | 97.5 |
| 206 | 2-(1-{5-Phenyl-4-[(pyridin-2-ylmethyl)-amino]-thieno[2,3-d]pyrimidin-2-yl}-piperidin-4-yl)-ethanol | 97.5 |
| 207 | [5-(4-Fluorophenyl)-2-morpholin-4-yl-thieno[2,3-d]pyrimidin-4-yl]-pyridin-2-ylmethyl-amine | 97.2 |
| 208 | 2-((2-Hydroxy-ethyl)-{5-phenyl-4-[(pyridin-2-ylmethyl)-amino]-thieno[2,3-d] pyrimidin-2-yl}-amino)-ethanol | 96.6 |
| 209 | 5-(4-Fluoraphenyl)-N²-(2-methoxy-ethyl)-N⁴-pyridin-2-ylmethyl-thieno[2,3-d] pyrimidine-2,4-diamine | 96.0 |
| 210 | 2-{5-Phenyl-4-[(pyridin-2-ylmethyl)-amino]-thieno[2,3-d]pyrimidin-2-ylamino}-propane-1,3-diol | 95.6 |
| 211 | [2-(2-Dimethylamino-ethoxy)-5-phenyl-thieno[2,3-d]pyrimidin-4-yl]-pyridin-2-ylmethyl-amine | 89.0 |
| 212 | 2-{5-(4-Fluorophenyl)-4-[(pyridin-2-ylmethyl)-amino]-thieno[2,3-d]pyrimidin-2-ylamino}-ethanol | 87.0 |
| 214 | 3-{5-Phenyl-4-[(pyridin-2-ylmethyl)-amino]-thieno[2,3-d]pyrimidin-2-ylamino}-propane-1,2-diol | 87.0 |
| 215 | (2-(4-Methyl-piperazin-1-yl)-5-phenyl-thieno[2,3-d]pyrimidin-4-yl]-pyridin-2-ylmethyl-amine | 82.0 |
| 237 | {5-Phenyl-4-[(pyridin-2-ylmethyl)amino]thieno[2,3-c]pyrimidin-2-yl}acetic acid ethyl ester | 95.0 |
| 238 | {4-[(Furan-2-ylmethyl)-amino]-5-phenyl-thieno[2,3-d]pyrimidin-2-yl}-acetic acid ethyl ester | 96.0 |
| 250 | 2-{5-Phenyl-4-[(pyridin-2-ylmethyl)amino]thieno[2,3-d]pyrimidin-2-yl}ethanol | 98.0 |
| 251 | 2-{4-[(Furan-2-ylmethyl)-amino]-5-phenyl-thieno[2,3-d]pyrimidin-2-yl}-ethanol | 98.0 |
| 275 | 2-{5-Phenyl-4-[(pyridin-2-ylmethyl)-amino]-thieno[2,3-d]pyrimidin-2-yl}-propane-1,3-diol | 93.0 |
| 287 | N-Methyl-2-{5-phenyl-4-[(pyridin-2-ylmethyl) amino]thieno[2,3-d]pyrimidin-2-yl} acetamide | 91.0 |
| 302 | (2-Dimethylaminomethyl-5-phenylthieno[2,3-d]pyrimidin- 4-yl)pyridin-2-ylmethylamine | 76.0 |
| 303 | (2-Morpholin-4-ylmethyl-5-phenyl-thieno[2,3-d]pyrimidin-4-yl)-pyridin-2-ylmethyl-amine | 63.0 |
| 304 | (2-Methylaminomethyl-5-phenyl-thieno[2,3-d]pyrimidin-4-yl)-pyridin-2-ylmethyl-amine | 58.0 |
| 305 | (2-Methoxymethyl-5-phenylthien[2,3-c]primidin-4-yl)pyridin-2-ylmethylamine | 94.0 |
| 313 | Pyridin-2-ylmethyl-[5-(4-trifluoromethyl-phenyl)-thieno[2,3-d]pyrimidin-4-yl]-amine | 98.3 |
| 314 | (5-Benzo[1,3]dioxol-5-yl-thieno[2,3-d]pyrimidin-4-yl)-pyridin-2-ylmethyl-amine | 97.3 |
| 315 | [5-(4-Dimethylamino-phenyl)-thieno[2,3-d]pyrimidin-4-yl]-pyridin-2-ylmethyl-amine | 98.0 |
| 316 | [5-(3,4-Dimethyl-phenyl)-thieno[2,3-d]pyrimidin-4-yl]-pyridin-2-ylmethyl-amine | 98.0 |
| 317 | Pyridin-2-ylmethyl-[5-(4-trifluoromethoxy-phenyl)-thieno[2,3-d]pyrimidin-4-yl]-amine | 98.0 |
| 318 | Pyridin-2-ylmethyl-(5-p-tolyl-thieno[2,3-d]pyrimidin-4-yl)-amine | 98.0 |
| 319 | (5-Benzo[1,3]dioxol-5-yl-thieno[2,3-d]pyrimidin-4-yl)-pyridin-2-ylmethyl-amine | 97.3 |
| 320 | [5-(2,3-Dihydro-benzo[1,4]dioxin-6-yl)-thieno[2,3-d]pyrimidin-4-yl]-pyridin-2-ylmethyl-amine | 97.0 |
| 321 | [5-(3-Chlorophenyl)-thieno[2,3-d]pyrimidin-4-yl]-pyridin-2-ylmethyl-amine | 95.0 |
| 322 | [5-(3-Methoxyphenyl)-thieno[2,3-d]pyrimidin-4-yl]-pyridin-2-ylmethyl-amine | 94.0 |
| 323 | [5-(1 H-Indol-6-yl)-thieno[2,3-d]pyrimidin-4-yl]-pyridin-2-ylmethyl-amine | 94.0 |
| 324 | [5-(4-Methoxymethoxy-phenyl)-thieno[2,3-d]pyrimidin-4-yl]-pyridin-2-ylmethyl-amine | 93.0 |
| 325 | [5-(4-Chlorophenyl)-thieno[2,3-d]pyrimidin-4-yl]-pyridin-2-ylmethyl-amine | 90.8 |
| 326 | [5-(2,2-Difluoro-benzo[1,3]dioxol-5-yl)-thieno[2,3-d]pyrimidin-4-yl]-pyridin-2-ylmethyl-amine | 89.0 |
| 327 | 4-{4-[(Pyridin-2-ylmethyl)-amino]-thieno[2,3-d]pyrimidin-5-yl}-benzoic acid methyl ester | 83.0 |
| 328 | [5-(6-Methoxy-pyridin-3-yl)-thieno[2,3-d]pyrimidin-4-yl]-pyridin-2-ylmethyl-amine | 83.0 |
| 329 | [5-(2,4-Dichlorophenyl)-thieno[2,3-d]pyrimidin-4-yl]-pyridin-2-ylmethyl-amine | 81.0 |
| 330 | [5-(4-Chloro-3-trifluoromethyl-phenyl)-thieno[2,3-d]pyrimidin-4-yl]-pyridin-2-ylmethyl-amine | 79.0 |
| 331 | [5-(3-Fluorophenyl)-thieno[2,3-d]pyrimidin-4-yl]-pyridin-2-ylmethyl-amine | 76.0 |
| 332 | [5-(4-Morpholin-4-yl-phenyl)-thieno[2,3-d]pyrimidin-4-yl]-pyridin-2-ylmethyl-amine | 73.0 |
| 333 | [5-(3,4-Difluoro-phenyl)-thieno[2,3-d]pyrimidin-4-yl]-pyridin-2-ylmethyl-amine | 63.0 |
| 166 | 6-Methyl-N-[(6-methylpyridin-2-yl)methyl]-5-phenylthieno[2,3-d]pyrimidin-4-amine | 97 |
| 167 | 6-Bromo-N-[(6-methylpyridin-2-yl)methyl]-5-phenylthieno[2,3-d]pyrimidin-4-amine | 99 |

### Abbreviations

- Kv₍ᵤᵣ₎: Cardiac Ultrarapid Delayed Rectifier
- CHO: Chinese Hamster Ovary Cells
- DMEM: Dulbecco's Modified Eagle media
- FCS: Fetal Calf Serum
- EBSS: Earls Balanced Salt Solution
- WCPC: Whole-Cell Patch-Clamp

### References

Herbert, "General principles of the structure of ion channels", Am. J. Med, 104, 87-98, 1998.
Armstrong & Hille, "Voltage-gated ion channels and electrical excitability", Neuron, 20, 371-380,1998.
Gutman GA, Chandy KG, Adelman JP, Aiyar J, Bayliss DA, Clapham DE, Covarriubias M, Desir GV, Furuichi K, Ganetzky B, Garcia ML, Grissmer S, Jan LY, Karschin A, Kim D, Kuperschmidt S, Kurachi Y, Lazdunski M, Lesage F, Lester HA, McKinnon D, Nichols CG, O'Kelly I, Robbins J, Robertson GA, Rudy B, Sanguinetti M, Seino S, Stuehmer W, Tamkun MM, Vandenberg CA, Wei A, Wulff H, Wymore RS International Union of Pharmacology. XLI. Compendium of voltage-gated ion channels: potassium channels. Pharmacol Rev. 2003 Dec;55(4):583-6.
Shieh et al. "Potassium channels: molecular defects, diseases, and therapeutic opportunities", Pharmacol Rev, 52 (4), 557-594, 2000.
Ford et al. "Potassium Channels: Gene Family, Therapeutic Relevance, High-Throughput Screening Technologies and Drug Discovery", Prog Drug Res, 58, 133-168, 2002.
Marban "Cardiac channelopalthies", Nature, 415, 213-218, 213-218, 2002.
Brendel and Peukert 'Blockers of the Kv1.5 Channel for the Treatment of Atrial Arrhythmias', Expert Opinion in Therapeutic Patents, 12 (11), 1589-1598 (2002).
Wang et al., "Sustained depolarization-induced outward current in human atrial myocytes. Evidence for a novel delayed rectifier K+ current similar to Kv1.5 cloned channel currents", Circ Res, 73, 1061-1076, 1993.
Fedida et al., "Identity of a novel delayed rectifier current from human heart with a cloned K+ channel current", Circ Res , 73, 210-216, 1993.
Feng et al., "Antisense oligodeoxynucleotides directed against Kv1.5 mRNA specifically inhibit ultrarapid delayed rectifier K+ current in cultured adult human atrial myocytes", Circ Res, 80, 572-579, 1997.
Amos et al., "Differences between outward currents of human atrial and subepicardial ventricular myocytes", J Physiol, 491, 31-50, 1996.
Li et al., "Evidence for two components of delayed rectifier K+ current in human ventricular myocytes", Circ Res, 78, 689-696, 1996.
Nattel, 'Therapeutic implications of atrial fibrillation mechanisms: can mechanistic insights be used to improve AF management Cardiovascular Research, Volume 54, Issue 2, 347-360, 2002.
Courtemanche et al., "Ionic targets for drug therapy and atrial fibrillation-induced electrical remodeling: insights from a mathematical model", Cardiovasc Res, 42(2), 477-489,1999.
Nattel et al., "Cardiac ultrarapid delayed rectifiers: a novel potassium current family of functional similarity and molecular diversity", Cell Physiol Biochem, 9(4-5), 217-226, 1999.
Knobloch K, Brendel J, Peukert S, Rosenstein B, Busch AE, Wirth KJ. Electrophysiological and antiarrhythmic effects of the novel I(Kur) channel blockers, S9947 and S20951, on left vs. right pig atrium in vivo in comparison with the I(Kr) blockers dofetilide, azimilide, d,I-sotalol and ibutilide. Naunyn Schmiedebergs Arch Pharmacol. 2002 Nov;366 (5):482-7.
Wirth KJ, Paehler T, Rosenstein B, Knobloch K, Maier T, Frenzel J, Brendel J, Busch AE, Bleich M.Atrial effects of the novel K(+)-channel-blockerAVE0118 in anesthetized pigs. Cardiovasc Res. Nov 1;60(2):298-306, 2003.
Colatsky et al., "Channel specificity in antiarrhythmic drug action. Mechanism of potassium channel block and its role in suppressing and aggravating cardiac arrhythmias", Circulation, 82(6), 2235-2242, 1990.
Feng et al., "Effects of class III antiarrhythmic drugs on transient outward and ultra-rapid delayed rectifier currents in human atrial myocytes", J Pharmacol Exp Ther, 281(1), 384-392, 1997.
Wang et al., "Effects of flecainide, quinidine, and 4-aminopyridine on transient outward and ultrarapid delayed rectifier currents in human atrial myocytes", J Pharmacol, 272(1), 184-196, 1995.
Malayev et al., "Mechanism of clofilium block ofthe human Kv1.5 delayed rectifier potassium channel", Mol Pharmaco, 147(1), 198-205, 1995.
Godreau et al., "Mechanisms of action of antiarrhythmic agent bertosamil on hKv1.5 channels and outward potassium current in human atrial myocytes", J Pharmacol Exp Ther 300(2), 612-620, 2002.
Matsuda et al., "Inhibition by a novel anti-arrhythmic agent, NIP-142, of cloned human cardiac K+channel Kv1.5 current", Life Sci, 68, 2017-2024, 2001.
Bachmann et al., "Characterization of a novel Kv1.5 channel blocker in Xenopus oocytes, CHO cells, human and rat cardiomyocytes", Naunyn Schmiedebergs Arch Pharmacol, 364(5), 472-478, 2001.
Peukert S, Brendel J, Pirard B, Bruggemann A, Below P, Kleemann HW, Hemmerle H, Schmidt W. Identification, synthesis, and activity of novel blockers of the voltage-gated potassium channel Kv1.5. J Med Chem. Feb 13;46(4): 486-98, 2003.
Xu & Xu, "The expression of arrhythmic related genes on Xenopus oocytes for evaluation of class III antiarrhythmic drugs from ocean active material", Yi Chuan Xue Bao, 27(3), 195-201, 2000.
Katada et al, 'Cytotoxic effects of NSL-1406, a new thienopyrimidine derivative, on leukocytes and osteoclasts.' Bioorg. Med. Chem. Lett., 9, 797-802, 1999.
Stewart et al, 'Discovery of inhibitors of cell adhesion molecule expression in human endothelial cells. 1. Selective inhibition of ICAM-1 and E-selectin expression', J. Med. Chem., 44, 988-1002, 2001.
Hozien et al, 'Synthesis and application of some new thienopyrimidine derivatives as antimicrobial agents', Synthetic Communications, 26(20), 3733-3755, 1996.
Ismail et al., 'Synthesis and antimicrobial activity of some tetramethylenethienopyrimidine derivatives', Farmaco, 50 (9), 611-616,1995.
Konno et al., 'Synthesis of thienopyrimidine derivatives and their antifungal activities', Yakugaku Zasshi, 109(7), 464-473, 1989.
Ram et al., "Thienopyrimidines as potential chemotherapeutic agents 11', J. Het. Chem., 18(7), 1277-1280, 1981.
Ram et al., 'Thienopyrimidines as potential chemotherapeutic agents', Archiv der Pharmazie, 312(1 ), 19-25, 1979.
Shehata et al., 'Synthesis, antitumour and anti-HIV-1 testing of certain thienopyrimidine, thienoimidazopyrimidine and thienothiazine derivatives' Med. Chem. Res., 6(3), 148-163, 1996.
Moneer et al, 'Reaction of 30amino and 4-hydrazino-5,6-tetramethylenethienopyrimidine derivatives with azlactones', Egyptian Journal of Pharm. Sci., 34 (4-6), 599-609, 1994.
Jordis et al., '7,9-Dideaza-9-thiaadenines (4-aminothieno[2,3-d]pyrimidines) as potential anticytokinins'Vestnik Slovenskega Kemijskega Drustva , 33(3), 217-38, 1986.
Noravyan et al., 'Synthesis and anticonvulsive activity of 4-alkyl (or aryl)amino-6,6-dimethyl-5,6-dihydro-8H-pyrano (or thiopyrano)[3,4-b]thieno[5,4-d]pyrimidines' Khimiko-Farmatsevticheskii Zhurnal, 11 (9), 38-42, 1977.
Hosni et al., 'Thienopyrimidines II: synthesis of newer thieno[2,3-d]pyrimidines and their quatemized derivatives with molluscicidal activity' Acta Poloniae Pharmaceutica, 56(1) 49-56, 1999.
Munchof et al., 'Design and SAR of thienopyrimidine and thienopyridine inhibitors of VEGFR-2 kinase activity'. Bioorganic & Medicinal Chemistry Letters, 14(1), 21-24, 2004

## Claims

1. A compound of formula (I) 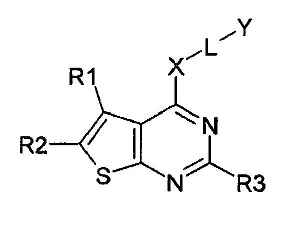 Wherein
R1 is C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂ NR⁹R¹⁰ or hydroxyl; heteroaryl which is unsubstituted or substituted with cyano, nitro, halogen, alkyl, alkylthio, alkoxy, NR⁹R¹⁰, CO₂R₇, C(O)NR⁹R¹⁰, NHC(O)R8, SO₂NR⁹R¹⁰ or hydroxyl; or C₃₋₆ cycloalkyl;
R2 is H, nitro, -CO₂R7, CONR4R5 halo, or C₁₋₆ alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, NHC(O)R8, halo, trifluoromethyl, nitrile, C₁₋₃ alkoxy, or C₁₋₆ alkyl which is unsubstituted or substituted with halogen, cyano, nitro, NR9R10, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R4 and R5 may be the same or different, and may be H; C₁₋₆ alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; C₆₋₁₀ aryl which is unsubstituted or substituted with cyano, halogen, nitro, trifluoromethyl, alkyl, alkylthio, alkoxy, NR9R10, CO₂R7, C(O)NR9R10, NHC(O)R8, SO₂NR9R10 or hydroxyl; heteroaryl which is unsubstituted or substituted with cyano, nitro, halogen, alkyl, alkylthio, alkoxy, NR9R10, CO₂R7, C(O)NR9R10, NHC(O)R8, SO₂NR9R10 or hydroxyl; or C₃₋₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁₋₆ alkyl which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is C₆-C₁₀ aryl which is unsubstituted or substituted with cyano, halogen, nitro, trifluoromethyl, alkyl, alkylthio, alkoxy, NR9R10, CO₂R7, C(O)NR9R10, NHC(O)R8, SO₂NR9R10 or hydroxyl; a heterocyclic group, which is unsubstituted or substituted with cyano, nitro, halogen, alkyl, alkylthio, alkoxy, NR9R10, CO₂R7, C(O)NR9R10, NHC(O)R8, SO₂NR9R10 or hydroxyl; alkenyl; C₃₋₆ cycloalkyl; an unsubstituted C₁-C₆ alkyl or C₁-C₆ alkyl substituted with halogen, cyano, nitro, NR9R10, alkoxy, unsubstituted aryl, unsubstituted heteroaryl, C(O)NR9R10, NHC(O)R8 or SO₂NR9R10;
R9 and R10 can be the same or different, and may be selected from H, unsubstituted C₁₋₆ alkyl, unsubstituted C₆-C₁₀ aryl, unsubstituted heteroaryl, unsubstituted cycloalkyl, aminoethyl, methylaminoethyl, dimethylaminoethyl, hydroxylethyl, alkoxyethyl, or R9 and R10 may together form a saturated or partially saturated 4-7 member ring, wherein said ring may optionally comprise one, two or three further heteroatoms or pharmaceutically acceptable salts thereof;
with the proviso that when Y is phenyl, phenyl monosubstituted by Cl or methoxy, furanyl, tetrahydrofurayl, pyrimidinyl, pyrrolidinyl or 1,3-benzodioxolyl, then R1 is not phenyl, phenyl monosubstituted by halogen or phenyl substituted by methyl;
and wherein the compound is not:
N-butyl-5-phenylthieno[2,3-d]pyrimidin-4-amine;
5-phenyl-N-(pyridin-2-ylmethyl)thieno[2,3-d]pyrimidin-4-amine;
5-(4-chlorophenyl)-N-[3-(1H-imidazol-1-yl)propyl]thieno[2,3-d]pyrimidin-4-amine;
5-(4-chlorophenyl)-N-(pyridin-2-ylmethyl)thieno[2,3-d]pyrimidin-4-amine;
5-(4-chlorophenyl)-N-(2-cyclohex-1-en-1-ylethyl)thieno[2,3-d]pyrimidin-4-amine;
5-(4-chlorophenyl)-N-(pyridin-3-ylmethyl)thieno[2,3-d]pyrimidin-4-amine;
5-(4-chlorophenyl)-N-(2-furylmethyl)thieno[2,3-d]pyrimidin-4-amine;
5-(4-fluorophenyl)-N-(pyridin-3-ylmethyl)thieno[2,3-d]pyrimidin-4-amine;
N-allyl-5-phenylthieno[2,3-d]pyrimidin-4-amine;
5-(4-methylphenyl)-N-(2-thien-2-ylethyl)thieno[2,3-d]pyrimidin-4-amine;
N-(2-furylmethyl)-5-phenylthieno[2,3-d]pyrimidin-4-amine;
5-(4-chlorophenyl)-N-(2-thien-2-ylethyl)thieno[2,3-d]pyrimidin-4-amine;
5-(4-fluorophenyl)-N-(2-thien-2-ylethyl)thieno[2,3-d]pyrimidin-4-amine;
N-allyl-5-(4-chlorophenyl)thieno[2,3-d]pyrimidin-4-amine;
5-(4-chlorophenyl)-N-(tetrahydrofuran-2-ylmethyl)thieno[2,3-d]pyrimidin-4-amine;
5-phenyl-N-(tetrahydrofuran-2-ylmethyl)thieno[2,3-d]pyrimidin-4-amine;
5-(4-bromophenyl)-N-(pyridin-3-ylmethyl)thieno[2,3-d]pyrimidin-4-amine;
N-[3-(1H-imidazol-1-yl)propyl]-5-phenylthieno[2,3-d]pyrimidin-4-amine;
1-(2- {[5-(4-methylphenyl)thieno[2,3-d]pyrimidin-4-yl]amino}ethyl)imidazolidin-2-one;
N-(2-furylmethyl)-5-(4-methylphenyl)thieno[2,3-d]pyrimidin-4-amine; or
N-ethyl-2-methyl-5-(thiophen-2-yl)thieno[2,3-d]pyrimidin-4-amine.

2. A compound as claimed in claim 1 wherein R1 is aryl or heteroaryl, R2 is H or alkyl, R3 is H, NR4R5, alkoxy or alkýl, X is O or NR6, R6 is H, n is 1 or 2 and Y is an unsubstituted alkyl or alkyl substituted with halogen, cyano, nitro, NR9R10, alkoxy, unsubstituted aryl, unsubstituted heteroaryl, C(O)NR9R10, NHC(O)R8 or SO₂NR9R10; cycloalkyl, aryl or heteroaryl.

3. A compound as claimed in claim 2 wherein R1 is aryl or heteroaryl, R2 is H or methyl, R3 is H, NR4R5, alkoxy or alkyl, X is NR6, R6 is H, n is 1 and Y is heteroaryl.

4. A compound as claimed in any one of claims 1 to 3 wherein Y is furanyl, thienyl or pyridyl.

5. A compound as claimed in any one of claims 1 to 4 wherein Y is optionally substituted furan-2-yl or optionally substituted pyridin-2-yl.

6. A compound as claimed in claim 1 which is:
2-{5-Phenyl-4-[(pyridin-2-ylmethyl)-amino]-thieno[2,3-d]pyrimidin-2-yl}-propane-1,3-diol;
2-{5-(4-Fluoro-phenyl)-4-[(pyridin-2-ylmethyl)-amino]-thieno[2,3-d]pyrimidin-2-ylamino}-ethanol;
Pyridin-2-ylmethyl-(5-p-tolyl-thieno[2,3-d]pyrimidin-4-yl)-amine;
2-{5-Phenyl-4-[(pyridin-2-ylmethyl)-amino]-thieno[2,3-d]pyrimidin-2-ylamino}-ethanol;
2-{5-phenyl-4-[(pyridin-2-ylmethyl)amino]thieno[2,3-d]pyrimidin-2-yl}ethanol;
2-((2-Hydroxy-ethyl)-{5-phenyl-4-[(pyridin-2-ylmethyl)-amino]-thieno[2,3-d]pyrimidin-2-yl}-amino)-ethanol;
2-Methyl-N-(2-pyridyl)methyl-5-phenylthieno[2,3-d]pyrimidin-4-ylamine;
2-{4-[(Furan-2-ylmethyl)-amino]-5-phenyl-thieno[2,3-d]pyrimidin-2-yl}-ethanol;
[2-(2-Methoxy-ethoxy)-5-phenyl-thieno[2,3-d]pyrimidin-4-yl]-pyridin-2-ylmethyl-amine;
(2-Methoxy-5-phenyl-thieno [2,3-d]pyrimidin-4-yl)-pyridin-2-ylmethyl-amine;
5-(4-Fluoro-phenyl)-N*2*-(2-methoxy-ethyl)-N*4*-pyridin-2-ylmethyl-thieno[2,3-d]pyrimidine-2,4-diamine;
[5-(4-Dimethylamino-phenyl)-thieno[2,3-d]pyrimidin-4-yl]-pyridin-2-ylmethyl-amine;
5-(4-Fluoro-phenyl)-N*2*,N*2*-dimethyl-N*4*-pyridin-2-ylmethyl-thieno[2,3-d]pyrimidine-2,4-diamine;
Pyridin-2-ylmethyl-[5-(4-trifluoromethyl-phenyl)-thieno[2,3-d]pyrimidin-4-yl]-amine;
[5-(1H-Indol-6-yl)-thieno[2,3-d]pyrimidin-4-yl]-pyridin-2-ylmethyl-amine;
(5-Benzo[1,3]dioxol-5-yl-thieno[2,3-d]pyrimidin-4-yl)-pyridin-2-ylmethyl-amine;
2-{5-Phenyl-4-[(pyridin-2-ylmethyl)-amino]-thieno[2,3-d]pyrimidin-2-ylamino}-propane-1,3-diol;
3-{5-Phenyl-4-[(pyridin-2-ylmethyl)-amino]-thieno[2,3-d]pyrimidin-2-ylamino}-propane-1,2-diol;
N-methyl-2-{5-phenyl-4-[(pyridin-2-ylmethyl)amino]thieno[2,3-d]pyrimidin-2-yl}acetamide; or
6-methyl-N-[(6-methylpyridin-2-yl)methyl]-5-phenylthieno[2,3-d]pyrimidin-4-amine; and pharmaceutically acceptable salts thereof.

7. A process for preparing a compound as claimed in any one of claims 1 to 6 comprising:
(i) by reacting a compound of formula II with a suitable nucleophile X-L-Y, optionally in the presence of a solvent and a base, and optionally at elevated temperature or with microwave irradiation; or 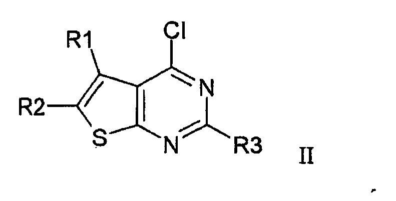
(ii) reacting a compound of formula IX by displacement of the 2-chloro substituent with a suitable nucleophilic species; or 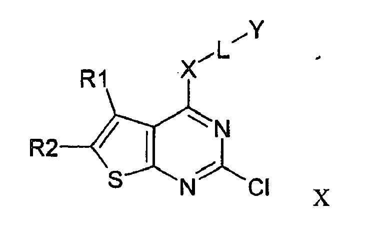
(iii) reacting a compound of formula XVIII with an aryl or heteroaryl boronic acid, optionally in the presence of a palladium catalyst, and optionally at elevated temperature or with microwave irradiation.

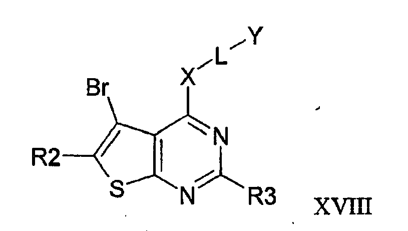

8. A pharmaceutical composition comprising at least one compound and optionally one or more excipients, diluents and/or carriers, wherein said compound has the formula 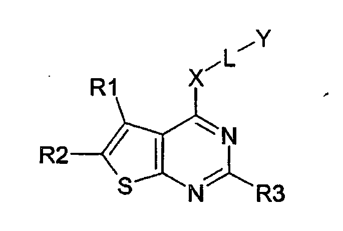 wherein
R1 is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl;
or C₃-C₆ cycloalkyl;
R2 is H;C₁₋₆ alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; nitro; -CO₂R7, CONR4R5; or halo;
R3 is H, NR4R5, NHC(O)R8, halo, trifluoromethyl, C₁₋₆ alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; nitrile or alkoxy;
R4 and R5 may be the same or different, and may be H, C₁₋₆ alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl;
heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁₋₆ alkyl which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl;, heterocyclic group which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; alkenyl;
C₃₋₆ cycloalkyl; C₁₋₆ alkyl which is unsubstituted or substituted with halogen, cyano, nitro, NR9R10, alkoxy, unsubstituted aryl, unsubstituted heteroaryl, C(O)NR9R10, NHC(O)R8 or SO₁NR9R10;
R9 and R10 can be the same or different, and may be selected from H, unsubstituted C₁₋₆ alkyl, unsubstituted C₆-C₁₀ aryl, unsubstituted heteroaryl, unsubstituted cycloalkyl, aminoethyl, methylaminoethyl, dimethylaminoethyl, hydroxyethyl, alkoxyethyl, or R9 and R10 may together form a saturated or partially saturated 4-7 member ring, wherein said ring may optionally comprise one, two or three further heteroatoms or pharmaceutically acceptable salts thereof;
with the proviso that when Y is phenyl, phenyl monosubstituted by Cl or methoxy, furanyl, tetrahydrofurayl, pyrimidinyl, pyrrolidinyl or 1,3-benzodioxolyl, then R1 is not phenyl, phenyl monosubstituted by halogen or phenyl substituted by methyl.

9. A pharmaceutical composition as claimed in claim 8 wherein said compound is a compound as claimed in any one of claims 1 to 6.

10. A compound, or a pharmaceutical composition comprising said compound for use in medicine, wherein said compound has the formula: 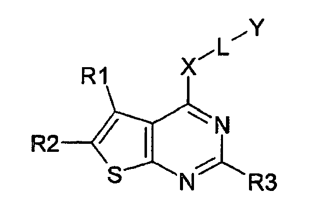 wherein
R1 is C₃-C₆ cycloalkyl; C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; or
heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl;
R2 is H, nitro, -CO₂R7, CONR4R5, halo or C₁₋₆ alkyl which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, NHC(O)R8, halo, trifluoromethyl, nitrile, alkoxy, or C₁₋₆ alkyl which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R4 and R5 may be the same or different, and may be H; C₁₋₆ alkyl which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl;
heteroaryl which is unsubstituted or with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁₋₆ alkyl, which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is ethyl or methyl;
R9 and R10 can be the same or different, and may be selected from H, unsubstituted C₁₋₆ alkyl, unsubstituted C₆-C₁₀ aryl, unsubstituted heteroaryl, unsubstituted cycloalkyl, aminoethyl, methylaminoethyl, dimethylaminoethyl, hydroxyethyl, alkoxyethyl, or R9 and R10 may together form a saturated or partially saturated 4-7 member ring, wherein said ring may optionally comprise one, two or three further heteroatoms L is (CH₂)ₙ, where n is 1, 2 or 3; and
Y is alkenyl, C₃-C₆ cycloalkyl; C₆-C₁₀ aryl which is unsubstituted or substituted with cyano, halogen, nitro, trifluoromethyl, alkyl, alkylthio, alkoxy,
NR⁹R¹⁰, CO₂R7, C(O)NR⁹R¹⁰, NHC(O)R8, SO₂NR⁹R¹⁰ or hydroxyl; heterocyclic group which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₁₋₆ Alkyl, which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰, or pharmaceutically acceptable salts thereof;
with the proviso that when Y is phenyl, phenyl monosubstituted by C1 or methoxy, furanyl, tetrahydrofurayl, pyrimidinyl, pyrrolidinyl or 1,3-benzodioxolyl, then R1 is not phenyl, phenyl monosubstituted by halogen or phenyl substituted by methyl.

11. A compound or pharmaceutical composition as claimed in claim 10, wherein said compound is a compound as claimed in any one of claims 1 to 6.

12. A compound having the formula: 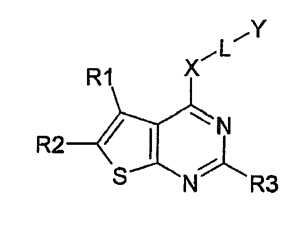 wherein
R1 is C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl;
heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl;
C₃-C₆ cycloalkyl; or C₁₋₆ alkyl which is unsubstituted or substituted with halogen, cyano, nitro, NR⁹R¹⁰, alkoxyl, hydroxyl, unsubstituted aryl, unsubstituted heteroaryl, CO₂R⁷, C(O)NR⁹R¹⁰, NHC(O)R8, or SO₂NR⁹R¹⁰;
R2 is H, nitro, -CO₂R7, CONR4R5, halo, or C₁₋₆alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, halo, trifluoromethyl, nitrile, alkoxy or C₁₋₆alkyl which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R4 and R5 may be the same or different, and may be H; C₁₋₆ alkyl, which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁴R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl; or R4 and R5 may together form a saturated, unsaturated or partially saturated 4 to 7 member ring wherein said ring may optionally comprise one or more further heteroatoms selected from N, O or S;
X is O, S or NR6;
R6 is H or C₁₋₆ alkyl, which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is alkenyl; C₃₋₆ cycloalkyl; C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; a heterocyclic group which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₁₋₆ alkyl, which is unsubstituted or substituted with halogen, cyano, nitro, NR⁹R¹⁰, alkoxyl, hydroxyl, unsubstituted aryl, unsubstituted heteroaryl, CO₂R⁷, C(O)NR⁹R¹⁰, NHC(O)R8 , or pharmaceutically acceptable salts thereof for use as a potassium channel inhibitor.

13. A compound as claimed in claim 12, wherein the compound is a compound as claimed in any one of claims 1-6.

14. The use of a compound having the formula: 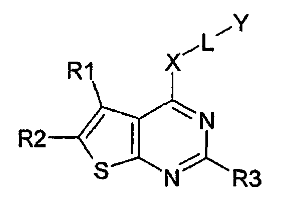 wherein
R1 is C₆-C₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl; or C₃-C₆ cycloalkyl;
R2 is H, nitro, -CO₂R7, CONR4R5, halo or C₁₋₆ alkyl, which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R3 is H, NR4R5, NHC(O)R8, halo, trifluoromethyl, nitrile, alkoxy or C₁₋₆alkyl, which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R4 and R5 may be the same or different, and may be H; C₁₋₆ alkyl, which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰; C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl; heteroaryl which is unsubstituted or substituted with cyano, nitro, halogen, alkyl, alkylthio, alkoxy, NR⁹R¹⁰, CO₂R7, C(O)NR⁹R¹⁰, NHC(O)R8, SO₂NR⁹R¹⁰ or hydroxyl or C₃₋₆ cycloalkyl; or R4 and R5 may together form a saturated, unsaturated or partially saturated 4 to 7 member ring, wherein said ring may optionally comprise one or more further heteroatoms selected from N, O or S;
X is O, S or NR6;
R6 is H or C₁₋₆alkyl, which is unsubstituted or substituted with halogen, cyano, nitro, NR⁹NR¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰;
R7 is hydrogen, methyl or ethyl;
R8 is methyl or ethyl;
L is (CH₂)ₙ, where n is 1, 2 or 3; and
Y is alkenyl; C₃₋₆ cycloalkyl; C₆₋₁₀ aryl which is unsubstituted or substituted with cyano, halogen, nitro, trifluoromethyl, alkyl, alkylthio, alkoxy, NR⁹R¹⁰, CO₂R7, C(O)NR⁹R¹⁰, NHC(O)R8, SO₂NR⁹R¹⁰ or hydroxyl, an heterocyclic group which is unsubstituted or substituted with cyano, nitro, halogen, alkyl, alkylthio, alkoxy, NR⁹R¹⁰, Co₂R7, C(O)NR⁹R¹⁰, NHC(O)R8, SO₂NR⁹R¹⁰ or hydroxyl, cycloalkyl; or C₁₋₆ alkyl, which is unsubstituted or substituted with halogen, cyano, nitro, NR⁹R¹⁰, alkoxy, hydroxyl, unsubstituted aryl, unsubstituted heteroaryl, CO₂R⁷, C(O)NR⁹R¹⁰, NHC(O)R8 or SO₂NR⁹R¹⁰, or pharmaceutically acceptable salts thereof in the manufacture of a medicament for use in potassium channel inhibition.

15. The use as claimed in claim 14 wherein the medicament is for use in the treatment or prevention of arrhythmia.

## Patentansprüche

1. Verbindung der Formel (I) 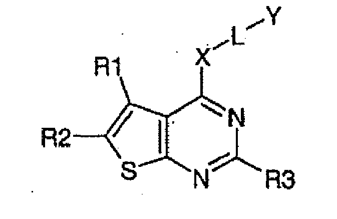 worin
R1 für C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂N⁹R¹⁰ oder Hydroxyl; oder C₃₋₆-Cycloalkyl steht;
R2 für H, Nitro, -CO₂R7, CONR4R5 Halogen oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R3 für H, NR4R5, NHC(O)R8, Halogen, Trifluormethyl, Nitril, C₁₋₃-Alkoxy oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R4 und R5 gleich oder verschieden sein können und H; C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; C₆-C₁₀-Aryl, welches unsubstituiert ist oder substituiert mit Cyano, Halogen, Nitro, Trifluormethyl, Alkyl, Alkylthio, Alkoxy, NR9R10, CO2R7, C(O)NR9R10, NHC(O)R8, SO2NR9R10 oder Hydroxyl; Heteroaryl, welches unsubstituiert ist oder substituiert mit Cyano, Nitro, Halogen, Alkyl, Alkylthio, Alkoxy, NR9R10, CO2R7, C(O)NR9R10, NHC(O)R8, SO2NR9R10 oder Hydroxyl; oder C₃-C₆-Cycloalkyl darstellen können; oder R4 und R5 zusammen einen gesättigten, ungesättigten oder teilweise gesättigten 4- bis 7-gliedrigen Ring bilden können, worin besagter Ring gegebenenfalls ein oder mehrere weitere Heteroatome umfassen kann, ausgewählt aus N, O oder S;
X für O, S oder NR6 steht;
R6 für H oder C₁₋₆-Alkyl steht, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰;
R7 für Wasserstoff, Methyl oder Ethyl steht;
R8 für Methyl oder Ethyl steht;
L für (CH₂)ₙ steht, wo n 1, 2 oder 3 ist; und
Y für C₆-C₁₀-Aryl, welches unsubstituiert ist oder substituiert mit Cyano, Halogen, Nitro, Trifluormethyl, Alkyl, Alkylthio, Alkoxy, NR9R10, CO2R7, C(O)NR9R10, NHC(O)R8, SO2NR9R10 oder Hydroxyl; eine heterocyclische Gruppe, welche unsubstituiert ist oder substituiert mit Cyano, Nitro, Halogen, Alkyl, Alkylthio, Alkoxy, NR9R10, CO2R7, C(O)NR9R10, NHC(O)R8, SO2NR9R10 oder Hydroxyl; Alkenyl; C₃-C₆-Cycloalkyl; ein unsubstituiertes C₁-C₆-Alkyl oder C₁-C₆-Alkyl, substituiert mit Halogen, Cyano, Nitro, NR9R10, Alkoxy, unsubstituiertem Aryl, unsubstituiertem Heteroaryl, C(O)NR9R10, NHC(O)R8 oder SO₂NR9R10 steht;
R9 und R10 gleich oder verschieden sein können und ausgewählt sein können aus H, unsubstituiertem C₁₋₆-Alkyl, unsubstituiertem C₆-C₁₀-Aryl, unsubstituiertem Heteroaryl, unsubstituiertem Cycloalkyl, Aminoethyl, Methylaminoethyl, Dimethylaminoethyl, Hydroxylethyl, Alkoxyethyl, oder R9 und R10 zusammen einen gesättigten oder teilweise gesättigten 4-7-gliedrigen Ring bilden können, wobei besagter Ring gegebenenfalls ein, zwei oder drei weitere Heteroatome umfassen kann, oder pharmazeutisch annehmbare Salze davon;
mit der Maßgabe, dass wenn Y für Phenyl, Phenyl monosubstituiert durch Cl oder Methoxy, Furanyl, Tetrahydrofuranyl, Pyrimidinyl, Pyrrolidinyl oder 1,3-Benzodioxolyl steht, dann R1 nicht für Phenyl, Phenyl, monosubstituiert durch Halogen oder Phenyl, substituiert durch Methyl steht;
und wobei die Verbindung nicht:
N-Butyl-5-phenylthieno[2,3-d]pyrimidin-4-amin;
5-Phenyl-N-(pyridin-2-ylmethyl)thieno[2,3-d]pyrimidin-4-amin;
5-(4-Chlorphenyl)-N-[3-(1H-imidazol-1-yl)propyl]thieno[2,3-d]pyrimidin-4-amin;
5-(4-Chlorphenyl)-N-(pyridin-2-ylmethyl)thieno[2,3-d]pyrimidin-4-amin;
5-(4-Chlorphenyl)-N-(2-cyclohex-1-en-1-ylethyl)thieno[2,3-d]pyrimidin-4-amin;
5-(4-Chlorphenyl)-N-(pyridin-3-ylmethyl)thieno[2,3-d]pyrimidin-4-amin;
5-(4-Chlorphenyl)-N-(2-furylmethyl)thieno[2,3-d]pyrimidin-4-amin;
5-(4-Fluorphenyl)-N-(pyridin-3-ylmethyl)thieno[2,3-d]pyrimidin-4-amin;
N-Allyl-5-phenylthieno[2,3-d]pyrimidin-4-amin;
5-(4-Methylphenyl)-N-(2-thien-2-ylethyl)thieno[2,3-d]pyrimidin-4-amin;
N-(2-Furylmethyl)-5-phenylthieno[2,3-d]pyrimidin-4-amin;
5-(4-Chlorphenyl)-N-(2-thien-2-ylethyl)thieno[2,3-d]pyrimidin-4-amin;
5-(4-Fluorphenyl)-N-(2-thien-2-ylethyl)thieno[2,3-d]pyrimidin-4-amin;
N-Allyl-5-(4-chlorphenyl)thieno[2,3-d]pyrimidin-4-amin;
5-(4-Chlorphenyl)-N-(tetrahydrofuran-2-ylmethyl)thieno[2,3-d]pyrimidin-4-amin;
5-Phenyl-N-(tetrahydrofuran-2-ylmethyl)thieno[2,3-d]pyrimidin-4-amin;
5-(4-Bromphenyl)-N-(pyridin-3-ylmethyl)thieno[2,3-d]pyrimidin-4-amin;
N-[3-(1H-Imidazol-1-yl)propyl]-5-phenylthieno[2,3-d]pyrimidin-4-amin;
1-(2-{[5-(4-Methylphenyl)thieno[2,3-d]pyrimidin-4-yl]amino}ethyl)imidazolidin-2-on;
N-(2-Furylmethyl)-5-(4-methylphenyl)thieno[2,3-d]pyrimidin-4-amin; oder
N-ethyl-2-methyl-5-(thiophen-2-yl)thieno[2,3-d]pyrimidin-4-aminist.

2. Verbindung wie in Anspruch 1 beansprucht, worin R1 für Aryl oder Heteroaryl steht, R2 für H oder Alkyl steht, R3 für H, NR4R5, Alkoxy oder Alkyl steht, X für O oder NR6 steht, R6 für H steht, n 1 oder 2 ist und Y ein unsubstituiertes Alkyl oder Aryl, substituiert mit Halogen, Cyano, Nitro, NR9R10, Alkoxy, unsubstituiertem Aryl, unsubstituiertem Heteroaryl, C(O)NR9R10, NHC(O)R8 oder SO₂NR9R10, Cycloalkyl, Aryl oder Heteroaryl darstellt.

3. Verbindung wie in Anspruch 2 beansprucht, worin R1 für Aryl oder Heteroaryl steht, R2 für H oder Methyl steht, R3 für H, NR4R5, Alkoxy oder Alkyl steht, X für NR6 steht, R6 für H steht, n 1 ist und Y für Heteroaryl steht.

4. Verbindung wie in einem von Ansprüchen 1 bis 3 beansprucht, worin Y für Furanyl, Thienyl oder Pyridyl steht.

5. Verbindung wie in einem von Ansprüchen 1 bis 4 beansprucht, worin Y gegebenenfalls substituiertes Furan-2-yl oder gegebenenfalls substituiertes Pyridin-2-yl darstellt.

6. Verbindung wie in Anspruch 1 beansprucht, welche ist:
2-{5-Phenyl-4-[(pyridin-2-ylmethyl)-amino]-thieno[2,3-d]pyrimidin-2-yl}-propan-1,3-diol;
2-{5-(4-Fluor-phenyl)-4-[(pyridin-2-ylmethyl)-amino]-thieno[2,3-d]pyrimidin-2-ylamino}-ethanol;
Pyridin-2-ylmethyl-(5-p-tolyl-thieno[2,3-d]pyrimidin-4-yl)-amin;
2-{5-Phenyl-4-[(pyridin-2-ylmethyl)-amino]-thieno[2,3-d]pyrimidin-2-ylamino}-ethanol;
2-{5-Phenyl-4-[(pyridin-2-ylmethyl)amino]thieno[2,3-d]pyrimidin-2-yl}-ethanol;
2-((2-Hydroxy-ethyl)-{5-phenyl-4-[(pyridin-2-ylmethyl)-amino]-thieno[2,3-d]pyrimidin-2-yl}-amino)-ethanol;
2-Methyl-N-(2-pyridyl)methyl-5-phenylthieno[2,3-d]pyrimidin-4-ylamin;
2-{4-[(Furan-2-ylmethyl)-amino]-5-phenyl-thieno[2,3-d]pyrimidin-2-yl}-ethanol;
[2-(2-Methoxy-ethoxy)-5-phenyl-thieno[2,3-d]pyrimidin-4-yl]-pyridin-2-ylmethyl-amin;
(2-Methoxy-5-phenyl-thieno[2,3-d]pyrimidin-4-yl)-pyridin-2-ylmethyl-amin;
5-(4-Fluor-phenyl)-N*2*-(2-methoxy-ethyl)-N*4*-pyridin-2-ylmethyl-thieno[2,3-d]pyrimidin-2,4-diamin;
[5-(4-Dimethylamino-phenyl)-thieno[2,3-d]pyrimidin-4-yl]-pyridin-2-ylmethyl-amin;
5(4-Fluor-phenyl)-N*2*,N*2*-dimethyl-N*4*-pyridin-2-ylmethyl-thieno[2,3-d]pyrimidin-2,4-diamin;
Pyridin-2-ylmethyl-[5-(4-trifluormethyl-phenyl)-thieno[2,3-d]pyrimidin-4-yl]-amin;
[5-(1H-Indol-6-yl)-thieno[2,3-d]pyrimidin-4-yl]-pyridin-2-ylmethyl-amin;
(5-Benzo[1,3]dioxol-5-yl-thieno[2,3-d]pyrimidin-4-yl)-pyridin-2-ylmethyl-amin;
2-{5-Phenyl-4-[(pyridin-2-ylmethyl)-amino]-thieno[2,3-d]pyrimidin-2-ylamino}-propan-1,3-diol;
3-{5-Phenyl-4-[(pyridin-2-ylmethyl)-amino]-thieno[2,3-d]pyrimidin-2-ylamino}-propan-1,2-diol;
N-Methyl-2-{5-phenyl-4-[(pyridin-2-ylmethyl)amino]thieno[2,3-d]pyrimidin-2-yl}acetamid; oder
6-Methyl-N-[(5-methylpyridin-2-yl)methyl]-5-phenylthieno[2,3-d]pyrimidin-4-amin;
und pharmazeutisch annehmbare Salze davon.

7. Verfahren zum Herstellen einer Verbindung wie in einem von Ansprüchen 1 bis 6 beansprucht, welches umfasst:
(i) durch Umsetzen einer Verbindung der Formel II mit einem geeigneten Nucleophil X-L-Y, gegebenenfalls in Gegenwart eines Lösungsmittels und einer Base, und gegebenenfalls bei erhöhter Temperatur oder mit Mikrowellenstrahlung; oder 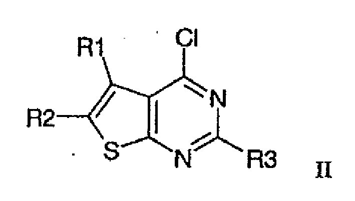
(ii) Umsetzen einer Verbindung der Formel IX durch Ersetzung des 2-Chlor-Substituenten mit einer geeigneten nucleophilen Art;
oder 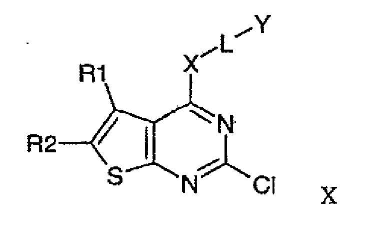
(iii) Umsetzen einer Verbindung der Formel XVIII mit einer Aryl- oder Heteroarylboronsäure, gegebenenfalls in Gegenwart eines Palladiumkatalysators und gegebenenfalls bei erhöhter Temperatur oder mit Mikrowellenstrahlung.

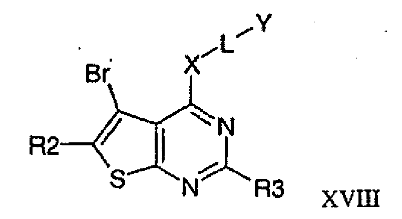

8. Pharmazeutische Zusammensetzung, welche mindestens eine Verbindung und gegebenenfalls einen oder mehrere Arzneimittelträger, Verdünnungsmittel und/oder Träger umfasst, wobei besagte Verbindung die Formel 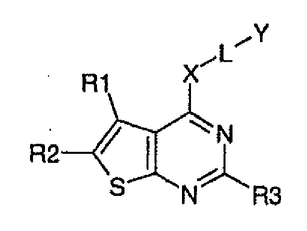 hat, worin
R1 für C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl ;
oder C₃₋C₆-Cycloalkyl steht;
R2 für H; C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; Nitro; -CO₂R7, CONR4R5; oder Halogen steht;
R3 für H, NR4R5, NHC(O)R8, Halogen, Trifluormethyl, C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; Nitril oder Alkoxy steht;
R4 und R5 gleich oder verschieden sein können und H; C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl;
Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; oder C₃-C₆-Cycloalkyl darstellen können; oder R4 und R5 zusammen einen gesättigten, ungesättigten oder teilweise gesättigten 4- bis 7-gliedrigen Ring bilden können, worin besagter Ring gegebenenfalls ein oder mehrere weitere Heteroatome umfassen kann, ausgewählt aus N, O oder S;
X für O, S oder NR6 steht;
R6 für H oder C₁₋₆-Alkyl steht, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰;
R7 für Wasserstoff, Methyl oder Ethyl steht;
R8 für Methyl oder Ethyl steht;
L für (CH₂)ₙ steht, wo n 1, 2 oder 3 ist; und
Y für C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; heterocyclische Gruppe, welche unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Alkenyl;
C₃-C₆-Cycloalkyl; C₁-C₆-Alkyl, welches unsubstituiert ist oder substituiert mit Halogen, Cyano, Nitro, NR9R10, Alkoxy, unsubstituiertem Aryl, unsubstituiertem Heteroaryl; C(O)NR9R10, NHC(O)R8 oder SO₂NR9R10 steht;
R9 und R10 gleich oder verschieden sein können und ausgewählt sein können aus H, unsubstituiertem C₁₋₆-Alkyl, unsubstituiertem C₆-C₁₀-Aryl, unsubstituiertem Heteroaryl, unsubstituiertem Cycloalkyl, Aminoethyl, Methylaminoethyl, Dimethylaminoethyl, Hydroxylethyl, Alkoxyethyl, oder R9 und R10 zusammen einen gesättigten oder teilweise gesättigten 4-7-gliedrigen Ring bilden können, wobei besagter Ring gegebenenfalls ein, zwei oder drei weitere Heteroatome umfassen kann, oder pharmazeutisch annehmbare Salze davon;
mit der Maßgabe, dass wenn Y für Phenyl, Phenyl monosubstituiert durch Cl oder Methoxy, Furanyl, Tetrahydrofuranyl, Pyrimidinyl, Pyrrolidinyl oder 1,3-Benzodioxolyl steht, dann R1 nicht für Phenyl, Phenyl, monosubstituiert durch Halogen oder Phenyl, substituiert durch Methyl steht.

9. Pharmazeutische Zusammensetzung wie in Anspruch 8 beansprucht, worin besagte Verbindung eine Verbindung wie in einem von Ansprüchen 1 bis 6 beansprucht ist.

10. Verbindung oder pharmazeutische Zusammensetzung, welche besagte Verbindung umfasst, zur Verwendung in Medizin, worin besagte Verbindung die Formel: 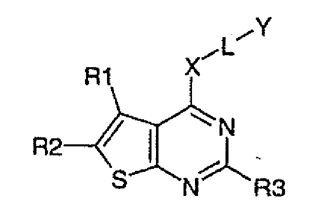 hat, worin
R1 für C₃-C₆-Cycloalkyl; C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O) NR⁹R¹⁰, NHC(O) R8, SO₂NR⁹R¹⁰ oder Hydroxyl; oder
Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂N⁹R¹⁰ oder Hydroxyl steht;
R2 für H; Nitro, -CO₂R7, CONR4R5, Halogen oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R3 für H, NR4R5, NHC(O)R8, Halogen, Trifluormethyl, Nitril, Alkoxy oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R4 und R5 gleich oder verschieden sein können und H; C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; oder C₃-C₆-Cycloalkyl darstellen können; oder R4 und R5 zusammen einen gesättigten, ungesättigten oder teilweise gesättigten 4-bis 7-gliedrigen Ring bilden können, worin besagter Ring gegebenenfalls ein oder mehrere weitere Heteroatome umfassen kann, ausgewählt aus N, O oder S;
X für O, S oder NR6 steht;
R6 für H oder C₁₋₆-Alkyl steht, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰;
R7 für Wasserstoff, Methyl oder Ethyl steht;
R8 für Ethyl oder Methyl steht;
R9 und R10 gleich oder verschieden sein können und ausgewählt sein können aus H, unsubstituiertem C₁₋₆-Alkyl, unsubstituiertem C₆-C₁₀-Aryl, unsubstituiertem Heteroaryl, unsubstituiertem Cycloalkyl, Aminoethyl, Methylaminoethyl, Dimethylaminoethyl, Hydroxylethyl, Alkoxyethyl, oder R9 und R10 zusammen einen gesättigten oder teilweise gesättigten 4-7-gliedrigen Ring bilden können, wobei besagter Ring gegebenenfalls ein, zwei oder drei weitere Heteroatome umfassen kann, L für (CH₂)ₙ steht, wo n 1, 2 oder 3 ist; und
Y für Alkenyl, C₃-C₆-Cycloalkyl, C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; heterocyclische Gruppe, welche unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; oder C₁₋₆-Alkyl, welches unsubstituiert ist oder substituiert oder mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht; oder pharmazeutisch annehmbare Salze davon;
mit der Maßgabe, dass wenn Y für Phenyl, Phenyl monosubstituiert durch C1 oder Methoxy, Furanyl, Tetrahydrofuranyl, Pyrimidinyl, Pyrrolidinyl oder 1,3-Benzodioxolyl steht, dann R1 nicht für Phenyl, Phenyl, monosubstituiert durch Halogen oder Phenyl, substituiert durch Methyl steht.

11. Verbindung oder pharmazeutische Zusammensetzung wie in Anspruch 10 beansprucht, worin besagte Verbindung eine Verbindung wie in einem von Ansprüchen 1 bis 6 beansprucht ist.

12. Verbindung mit der Formel: 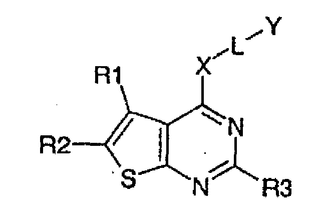 worin
R1 für C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl;
Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂N⁹R¹⁰ oder Hydroxyl ;
C₃-C₆-Cycloalkyl; oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R2 für H; Nitro, -CO₂R7, CONR4R5, Halogen oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R3 für H, NR4R5, Halogen, Trifluormethyl, Nitril, Alkoxy oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R4 und R5 gleich oder verschieden sein können und H; C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; oder C₃-C₆-Cycloalkyl darstellen können; oder R4 und R5 zusammen einen gesättigten, ungesättigten oder teilweise gesättigten 4-bis 7-gliedrigen Ring bilden können, worin besagter Ring gegebenenfalls ein oder mehrere weitere Heteroatome umfassen kann, ausgewählt aus N, O oder S;
X für O, S oder NR6 steht;
R6 für H oder C₁₋₆-Alkyl steht, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰;
R7 für Wasserstoff, Methyl oder Ethyl steht;
R8 für Methyl oder Ethyl steht;
L für (CH₂)ₙ steht, wo n 1, 2 oder 3 ist; und
Y für Alkenyl; C₃-C₆-Cycloalkyl; C₆-C₁₀-Aryl, welches unsubstituiert ist oder substituiert mit Cyano, Halogen, Nitro, Trifluorethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; eine heterocyclische Gruppe, welche unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰, oder Hydroxyl; oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 steht; oder pharmazeutisch annehmbare Salze davon, zur Verwendung als Kaliumkanalhemmer.

13. Verbindung wie in Anspruch 12 beansprucht, worin die Verbindung eine Verbindung wie in einem von Ansprüchen 1-6 beansprucht ist.

14. Verwendung einer Verbindung mit der Formel: 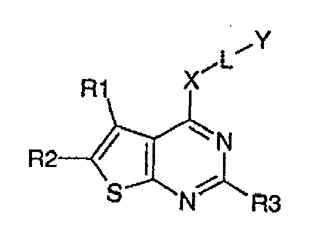 worin
R1 für C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Heteroaryl, welches unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R⁸, SO₂N⁹R¹⁰ oder Hydroxyl; oder C₃-C₆-Cycloalkyl steht;
R2 für H; Nitro, -CO₂R7, CONR4R5, Halogen oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht;
R3 für H, NR4R5, NHC(O)R8, Halogen, Trifluormethyl, Nitril, Alkoxy oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O) R8 oder SO₂NR⁹R¹⁰ steht;
R4 und R5 gleich oder verschieden sein können und H; C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰; C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; Heteroaryl, welches unsubstituiert ist oder substituiert mit Cyan, Nitro, Halogen, Alkyl, Alkylthio, Alkoxy, NR⁹R¹⁰, CO₂R7, C(O)NR⁹R¹⁰, NHC(O)R⁸, SO₂NR⁹R¹⁰ oder Hydroxyl; oder C₃-C₆-Cycloalkyl darstellen können; oder R4 und R5 zusammen einen gesättigten, ungesättigten oder teilweise gesättigten 4-bis 7-gliedrigen Ring bilden können, worin besagter Ring gegebenenfalls ein oder mehrere weitere Heteroatome umfassen kann, ausgewählt aus N, O oder S;
X für O, S oder NR6 steht;
R6 für H oder C₁₋₆-Alkyl steht, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰;
R7 für Wasserstoff, Methyl oder Ethyl steht;
R8 für Methyl oder Ethyl steht;
L für (CH₂)ₙ steht, wo n 1, 2 oder 3 ist; und
Y für Alkenyl; C₃-C₆-Cycloalkyl; C₆-C₁₀-Aryl, welches unsubstituiert ist oder substituiert mit Cyano, Halogen, Nitro, Trifluormethyl, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; eine heterocyclische Gruppe, welche unsubstituiert ist oder substituiert mit Cyano, Nitro, Halogen, Alkyl, Alkylthio, Alkoxy, NR⁹R¹⁰, CO₂R7, C(O)NR⁹R¹⁰, NHC(O)R8, SO₂NR⁹R¹⁰ oder Hydroxyl; oder C₁₋₆-Alkyl, welches unsubstituiert ist oder substituiert mit Halogen, Cyano, Nitro, NR⁹NR¹⁰, Alkoxyl, Hydroxyl, unsubstituiertem Aryl, unsubstituiertem Heteroaryl, CO₂R⁷, C(O)NR⁹R¹⁰, NHC(O)R8 oder SO₂NR⁹R¹⁰ steht; oder pharmazeutisch annehmbare Salze davon, in der Herstellung eines Medikaments zur Verwendung in Kaliumkanalhemmung.

15. Verwendung wie in Anspruch 14 beansprucht, worin das Medikament zur Verwendung in der Behandlung oder Verminderung von Arrhythmie ist.

## Revendications

1. Composé de formule (I) 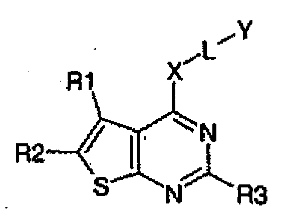 dans laquelle
R1 est un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; un hétéroaryle qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; ou un cycloalkyle en C_{3 à 6} ;
R2 est H, un nitro, -CO₂R7, CONR4R5, un halo, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R3 est H, NR4R5, NHC(O)R8, un halo, un trifluorométhyle, un nitrile, un alcoxy en C_{1 à 3}, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R4 et R5 peuvent être identiques ou différents, et peuvent être H ; un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰; un aryle en C₆ à C₁₀ qui est non substitué ou substitué avec un cyano, un halogène, un nitro, un trifluorométhyle, un alkyle, un alkylthio, un alcoxy, NR9R10, CO₂R7, C(O)NR9R10, NHC(O)R8, SO₂NR9R10 ou un hydroxyle ; un hétéroaryle qui est non substitué ou substitué avec un cyano, un nitro, un halogène, un alkyle, un alkylthio, un alcoxy, NR9R10, CO₂R7, C(O)NR9R10, NHC(O)R8, SO₂NR9R10 ou un hydroxyle ; ou un cycloalkyle en C₃ à C₆ ; ou R4 et R5 peuvent former ensemble un cycle saturé, insaturé ou partiellement saturé à 4 à 7 éléments, où ledit cycle peut facultativement comprendre un ou plusieurs hétéroatomes supplémentaires choisis parmi N, O ou S ;
X est O, S ou NR6 ;
R6 est H ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C (O) NR⁹R¹⁰, NHC (O) R8 ou SO₂NR⁹R¹⁰;
R7 est un hydrogène, un méthyle ou un éthyle ;
R8 est un méthyle ou un éthyle ;
L est (CH₂)ₙ, où n est 1, 2 ou 3 ; et
Y est un aryle en C₆ à C₁₀ qui est non substitué ou substitué avec un cyano, un halogène, un nitro, un trifluorométhyle, un alkyle, un alkylthio, un alcoxy, NR9R10, CO₂R7, C(O)NR9R10, NHC(O)R8, SO₂NR9R10 ou un hydroxyle ; un groupe hétérocyclique, qui est non substitué ou substitué avec un cyano, un nitro, un halogène, un alkyle, un alkylthio, un alcoxy, NR9R10, CO₂R7 C(O)NR9R10, NHC(O)R8, SO₂NR9R10 ou un hydroxyle ; un alcényle ; un cycloalkyl en C₃ à C₆ ; un alkyle non substitué en C₁ - C₆ ou un alkyle en C₁ - C₆ substitué avec un halogène, un cyano, un nitro, NR9R10, un alcoxy, un aryle non substitué, un hétéroaryle non substitué, C(O)NR9R10, NHC(O)R8 ou SO₂NR9R10 ;
R9 et R10 peuvent être identiques ou différents, et peuvent être choisis parmi H, un alkyle en C_{1 à 6} non substitué, un aryle en C_{6 à} C₁₀ non substitué, un hétéroaryle non substitué, un cycloalkyle non substitué, un aminoéthyle, un méthylaminoéthyle, un diméthylaminoéthyle, un hydroxyléthyle, un alcoxyéthyle, ou R9 et R10 peuvent former ensemble un cycle saturé, insaturé ou partiellement saturé à 4 à 7 éléments, où ledit cycle peut facultativement comprendre un ou plusieurs hétéroatomes supplémentaires ou leurs sels acceptables en pharmacie ;
à condition que lorsque Y est un phényle, un phényle monosubstitué par Cl ou méthoxy, un furanyle, un tetrahydrofurayle, un pyrimidinyle, un pyrrolidinyle ou un 1,3-benzodioxolyle, alors R1 n'est pas un phényle, un phényle monosubstitué par un halogène ou un phényle substitué par un méthyle ;
et dans laquelle le composé n'est pas :
N-butyl-5--phénylthiéno[2,3-d]pyrimidin-4-amine ; 5-phényl-N-(pyridin-2-ylméthyl)thiéno[2,3-d]pyrimidin-4-amine ;
5-(4-chlorophényl)-N-[3-(1H-imidazol-1-yl)propyl]thiéno[2,3-d]pyrimidin-4-amine ;
5-(4-chlorophényl)-N-(pyridin-2-ylméthyl)thiéno[2,3-d]pyrimidin-4-amine ;
5-(4-chlorophényl)-N-(2-cyclohex-1-én-1-yléthyl)thiéno[2,3-d]pyrimidin-4-amine ;
5-(4-chlorophényl)-N-(pyridin-3-ylméthyl)thiéno[2,3-d]pyrimidin-4-amine ;
5-(4-chlorophényl)-N-(2-furylméthyl)thiéno[2,3-d]pyrimidin-4-amine ;
5-(4-fluorophényl)-N-(pyridin-3-ylméthyl)thiéno[2,3-d]pyrimidin-4-amine ;
N-allyl-5-phénylthiéno[2,3-d]pyrimidin-4-amine ;
5-(4-méthylphényl)-N-(2-thién-2-yléthyl)thiéno[2,3-d]pyrimidin-4-amine ;
N-(2-furylméthyl)-5-phénylthiéno[2,3-d]pyrimidin-4-amine ;
5-(4-chlorophényl)-N-(2-thién-2-yléthyl)thiéno[2,3-d]pyrimidin-4-amine ;
5-(4-fluorophényl)-N-(2-thién-2-yléthyl)thiéno[2,3-d]pyrimidin-4-amine ;
N-allyl-5-(4-Chlorophényl)thiéno[2,3-d]pyrimidin-9-amine ;
5-(4-chlorophényl)-N-(tétrahydrofuran-2-ylméthyl)thiéno[2,3-d]pyrimidin-4-amine ;
5-phényl-N-(tétrahydrofuran-2-ylméthyl)thiéno[2,3-d]pyrimidin-4-amine ;
5-(4-bromophényl)-N-(pyridin-3-ylméthyl)thiéno[2,3-d]pyrimidin-4-amine ;
N-[3-(1H-imidazol-1-yl)propyl]-5-phénylthiéno[2,3-d]pyrimidin-4-amine ;
1-(2-{[5-(4-méthylphényl)thiéno[2,3-d]pyrimidin-4-yl]amino}éthyl)imidazolidin-2-one ;
N-(2-furylméthyl)-5-(4-méthylphényl)thiéno[2,3-d]pyrimidin-4-amine ;
ou
N-éthyl-2-méthyl-5-(thiophèn-2-yl)thiéno[2,3-d]pyrimidin-4-amine.

2. Composé selon la revendication 1, dans lequel R1 est un aryle ou un hétéroaryle, R2 est H ou un alkyle, R3 est H, NR4R5, un alcoxy ou un alkyle, X est O ou NR6, R6 est H, n est 1 ou 2 et Y est un alkyle non substitué ou un alkyle substitué avec un halogène, un cyano, un nitro, NR9R10, un alcoxy, un aryle non substitué, un hétéroaryle non substitué, C(O)NR9R10, NHC(O)R8 ou SO₂NR9R10, un cycloalkyle, un aryle ou un hétéroaryle.

3. Composé selon la revendication 2, dans lequel R1 est un aryle ou un hétéroaryle, R2 est H ou un méthyle, R3 est H, NR4R5, un alcoxy ou un alkyle, X est NR6, R6 est H, n est 1 et Y est un hétéroaryle.

4. Composé selon l'une quelconque des revendications 1 à 3, dans lequel Y est un furanyle, un thiényle ou un pyridyle.

5. Composé selon l'une quelconque des revendications 1 à 4, dans lequel Y est un furan-2-yle éventuellement substitué ou un pyridin-2-yle éventuellement substitué.

6. Composé selon la revendication 1 qui est :
2-{5-phényl-4-[(pyridin-2-ylméthyl)-amino]-thiéno[2,3-d]pyrimidin-2-yl}-propane-1,3-diol ;
2-{5-(4-fluoro-phényl)-4-[(pyridin-2-ylméthyl)-amino]-thiéno[2,3-d]pyrimidin-2-ylamino}-éthanol ;
pyridin-2-ylméthyl-(5-p-tolyl-thiéno[2,3-d]pyrimidin-4-yl)-amine :
2-{5-phényl-4-[(pyridin-2-ylméthyl)-amino]-thiéno[2,3-d]pyrimidin-2-ylamino}-éthanol ;
2-{5-phényl-4-[(pyridin-2-ylméthyl)amino]thiéno[2,3-d]pyrimidin-2-yl}éthanol ;
2-((2-hydroxy-éthyl)-{5-phényl-4-[(pyridin-2-ylméthyl)-amino]-thiéno[2,3-d]pyrimidin-2-yl}-amino)-éthanol ;
2-méthyl-N-(2-pyridyl)méthyl-5-phénylthiéno[2,3-d]pyrimidin-4-ylamine ;
2-{4-[(furan-2-ylméthyl)-amino]-5-phényl-thiéno[2,3-d]pyrimidin-2-yl}-éthanol ;
[2-(2-méthoxy-éthoxy)-5-phényl-thiéno[2,3-d]pyrimidin-4-yl]-pyridin-2-ylméthyl-amine ;
(2-méthoxy-5-phényl-thiéno[2,3-d]pyrimidin-4-yl)-pyridin-2-ylméthyl-amine ;
5-(4-fluoro-phényl)-N*2*-(2-méthoxy-éthyl)-N*4*-pyridin-2-ylméthyl-thiéno[2,3-d]pyrimidine-2,4-diamine ;
[5-(4-diméthylamino-phényl)-thiéno[2,3-d]pyrimidin-4-yl]-pyridin-2-ylméthyl-amine ;
5-(4-fluoro-phényl)-N*2*,N*2*-diméthyl-N*4*-pyridin-2-ylméthyl-thiéno[2,3-d]pyrimidine-2,4-diamine ;
pyridin-2-ylméthyl-[5-(4-trifluorométhyl-phényl)-thiéno[2,3-d]pyrimidin-4-yl]-amine ;
[5-(1H-indol-6-yl)-thiéno[2,3-d]pyrimidin-4-yl]-pyridin-2-ylméthyl-amine ;
(5-benzo[1,3]dioxol-5-yl-thiéno[2,3-d]pyrimidin-4-yl)-pyridin-2-ylméthyl-amine ;
2-{5-phényl-4-[(pyridin-2-ylméthyl)-amino]-thiéno[2,3-d]pyrimidin-2-ylamino}-propane-1,3-diol ;
3-{5-phényl-4-[(pyridin-2-ylmethyl)-amino]-thiéno[2,3-d]pyrimidin-2-ylamino}-propane-1,2-diol ;
N-méthyl-2-{5-phényl-4-[(pyridin-2-ylméthyl)amino]thiéno[2,3-d]pyrimidin-2-yl}acétamide ; ou
6-méthyl-N-[(6-méthylpyridin-2-yl)méthyl]-5-phénylthiéno[2,3-d]pyrimidin-4-amine ;
et leurs sels acceptables en pharmacie.

7. Procédé pour préparer un composé selon l'une quelconque des revendications 1 à 6 comprenant :
(i) la réaction d'un composé de formule II avec un nucléophile approprié X-L-Y, facultativement en présence d'un solvant et d'une base, et facultativement à température élevée ou avec une irradiation aux micro-ondes ; ou 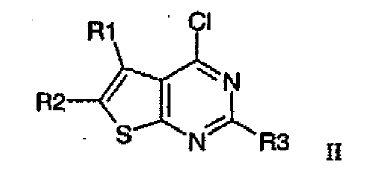
(ii) la réaction d'un composé de formule IX par déplacement du substituant 2-chloro avec une espèce nucléophile appropriée ; ou 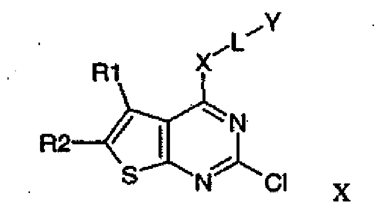
(iii) la réaction d'un composé de formule XVIII avec un acide aryl ou hétéroaryl boronique, facultativement en présence d'un catalyseur au palladium, et facultativement à température élevée ou avec une irradiation aux micro-ondes.

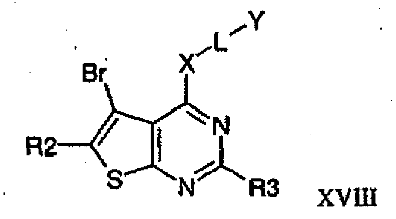

8. Composition pharmaceutique comprenant au moins un composé et facultativement un ou plusieurs excipients, diluants et/ou véhicules, dans laquelle ledit composé est de formule 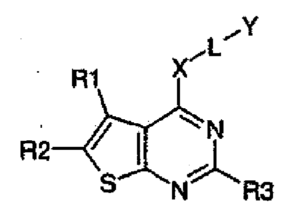 dans laquelle
R1 est un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; un hétéroaryle qui est non substitué ou substitué avec une cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ;
ou un cycloalkyle en C₃ à C₆ ;
R2 est H ; un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰ ; un nitro ; -CO₂R7, CNR4R5 ; ou un halo ;
R3 est H, NR4R5, NHC(O)R8, un halo, un trifluorométhyle, un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰; un nitrile ou un alcoxy ;
R4 et R5 peuvent être identiques ou différents, et peuvent être H, un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰; un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R⁷, C(O)NR⁹R¹⁰, NHC(O)R8, SO₂NR⁹R¹⁰ ou un hydroxyle ;
un hétéroaryle qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; ou un cycloalkyle en C₃ à C₆ ; ou R4 et R5 peuvent former ensemble un cycle saturé, insaturé ou partiellement saturé à 4 à 7 éléments, où ledit cycle peut facultativement comprendre un ou plusieurs hétéroatomes supplémentaires choisis parmi N, O ou S ;
X est O, S ou NR6 ;
R6 est H ou un alkyl en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C (O)NR⁹R¹⁰, NHC (O) R8 ou SO₂NR⁹R¹⁰;
R7 est un hydrogène, un méthyle ou un éthyle ; R8 est un méthyle ou un éthyle ;
L est (CH₂)ₙ, où n est 1, 2 ou 3 ; et
Y est un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; un groupe hétérocyclique qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; un alcényle ;
un cycloalkyle en C₃ à C₆ ; un alkyle en C_{1 à 6} qui est non substitué ou substitué avec un halogène, un cyano, un nitro, NR9R10, un alcoxy, un aryle non substitué, un hétéroaryle non substitué C(O)NR9R10, NHC(O)R8 or SO₂NR9R10 ;
R9 et R10 peuvent être identiques ou différents, et peuvent être choisis parmi H, un alkyle en C_{1 à 6} non substitué, un aryle en C₆ à C₁₀ non substitué, un hétéroaryle non substitué, un cycloalkyle non substitué, un aminoéthyle, un méthylaminoéthyle, un diméthylaminoéthyle, un hydroxyléthyle, un alcoxyéthyle, ou R9 et R10 peuvent former ensemble un cycle saturé, insaturé ou partiellement saturé à 4 à 7 éléments, où ledit cycle peut facultativement comprendre un ou plusieurs hétéroatomes supplémentaires ou leurs sels acceptables en pharmacie ;
à condition que lorsque Y est un phényle, un phényle monosubstitué par Cl ou un méthoxy, un furanyle, un tetrahydrofurayle, un pyrimidinyle, un pyrrolidinyle ou un 1,3-benzodioxolyle, alors R1 n'est pas un phényle, un phényle monosubstitué par un halogène ou un phényle substitué par un méthyle ;

9. Composition pharmaceutique selon la revendication 8, où ledit composé est un composé selon l'une quelconque des revendications 1 à 6.

10. Composé, ou composition pharmaceutique comprenant ledit composé pour une utilisation en médecine, où ledit composé est de formule : 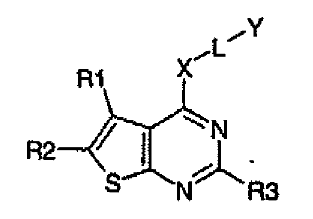 dans laquelle
R1 est un cycloalkyle en C₃ à C₆ ; un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; ou
un hétéroaryle qui est non substitué ou substitué avec un cyano, un nitro, un halogène, un alkyle, un alkylthio, un alcoxy, NR⁹R¹⁰, CO₂R₇, C(O)NR⁹R¹⁰, NHC(O)R8, SO₂NR⁹R¹⁰ ou un hydroxyle ;
R2 est H, un nitro, -CO₂R7, CONR4R5, un halo, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R3 est H, NR4R5, NHC(O)R8, un halo, un trifluorométhyle, un nitrile, un alcoxy, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰ ;
R4 et R5 peuvent être identiques ou différents, et peuvent être H ; un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰; un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle :
un hétéroaryle qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR₉R¹⁰ ou un hydroxyle ; ou un cycloalkyle en C₃ à C₆ ; ou R4 et R5 peuvent former ensemble un cycle saturé, insaturé ou partiellement saturé à 4 à 7 éléments, où ledit cycle peut facultativement comprendre un ou plusieurs hétéroatomes supplémentaires choisis parmi N, O ou S ;
X est O, S ou NR6 ;
R6 est H ou un alkyle en C_{1 à 6}. qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R7 est un hydrogène, un méthyle ou un éthyle ;
R8 est un éthyle ou un méthyle ;
R9 et R10 peuvent être identiques ou différents, et peuvent être choisis parmi H, un alkyle en C_{1 à 6} non substitué, un aryle en C₆ à C₁₀ non substitué, un hétéroaryle non substitué, un cycloalkyle non substitué, un aminoéthyle, un méthylaminoéthyle, un diméthylaminoéthyle, un hydroxyléthyle, un alcoxyéthyle, ou R9 et R10 peuvent former ensemble un cycle saturé, insaturé ou partiellement saturé à 4 à 7 éléments, où ledit cycle peut facultativement comprendre un, deux ou trois hétéroatomes supplémentaires L est (CH₂)ₙ, où n est 1, 2 ou 3 ; et
Y est un alcényle, un cycloalkyle en C₃ à C₆, un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R₇, C (O) NR⁹R¹⁰, NHC(O)R8, SO₂NR⁹R¹⁰ ou un hydroxyle ; un groupe hétérocyclique qui est non substitué ou substitué avec un cyano, un nitro, un halogène, un alkyle, un alkylthio, un alcoxy, NR⁹R¹⁰, CO₂R7 , C(O)NR⁹R¹⁰, NHC(O)R8, SO₂NR⁹R¹⁰ ou un hydroxyle ; ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰, ou leurs sels acceptables en pharmacie ;
à condition que lorsque Y est un phényle, un phényle monosubstitué par Cl ou méthoxy, un furanyle, un tetrahydrofurayle, un pyrimidinyle, un pyrrolidinyle ou un 1,3-benzodioxolyle, alors R1 n'est pas un phényle, un phényle monosubstitué par un halogène ou un phényle substitué par un méthyle ;

11. Composé ou composition pharmaceutique selon la revendication 10, où ledit composé est un composé selon l'une quelconque des revendications 1 à 6.

12. Composé de formule : 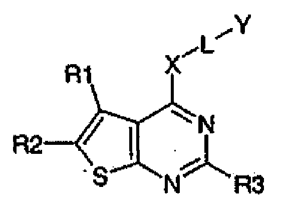 dans laquelle
R1 est un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ;
un hétéroaryle qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; un cycloalkyle en C₃ à C₆ ; ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰ ;
R2 est H, un nitro, -CO₂R7, CONR4R5, un halo, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R3 est H, NR4R5, un halo, un trifluorométhyle, un nitrile, un alcoxy, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰ ;
R4 et R5 peuvent être identiques ou différents, et peuvent être H ; un alkyle en C_{1 à 6}, qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰; un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R⁷, C(O)NR⁹R¹⁰, NHC(O)R8, SO₂NR⁹R¹⁰ ou un hydroxyle, un hétéroaryle qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; ou un cycloalkyle en C₃ à C₆ ; ou R4 et R5 peuvent former ensemble un cycle saturé, insaturé ou partiellement saturé à 4 à 7 éléments, où ledit cycle peut facultativement comprendre un ou plusieurs hétéroatomes supplémentaires choisis parmi N, O ou S ;
X est O, S ou NR6 ;
R6 est H ou un alkyle en C_{1 à 6}, qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R7 est un hydrogène, un méthyle ou un éthyle ;
R8 est un méthyle ou un éthyle ;
L est (CH₂)ₙ, où n est 1, 2 ou 3 ; et
Y est un alcényle ; un cycloalkyle en C₃ à C₆ ;
un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R⁷, C (O) NR⁹R¹⁰, NHC(O)R8, SO₂NR⁹R¹⁰ ou un hydroxyle : un groupe hétérocyclique qui est non substitué ou substitué avec un cyano, un nitro, un halogène, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle : ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou leurs sels acceptables en pharmacie pour une utilisation en tant qu'inhibiteur des canaux potassiques.

13. Composé selon la revendication 12, où le composé est un composé selon l'une quelconque des revendications 1 à 6.

14. Utilisation d'un composé de formule : 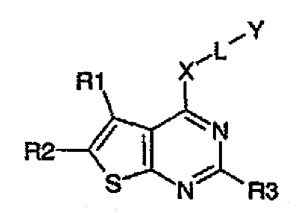 dans laquelle
R1 est un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle ; un hétéroaryle qui est non substitué ou substitué avec un cyano, un nitro, un halogène, un alkyle, un alkylthio, un alcoxy, NR⁹R¹⁰, CO₂R₇, C(O)NR⁹R¹⁰, NHC(O)R8, SO₂NR⁹R¹⁰ ou un hydroxyle ; ou un cycloalkyle en C₃ à C₆;
R2 est H, un nitro, -CO₂R7, CONR4R5, un halo, ou un alkyle en C_{1 à 6} qui est non substitué ou substitué avec un halogène, un cyano, un nitro, NR⁹NR¹⁰ un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R3 est H, NR4R5, NHC(O)R8, un halo, un trifluorométhyle, un nitrile, un alcoxy, ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R8 ou SO₂NR⁹R¹⁰;
R4 et R5 peuvent être identiques ou différents, et peuvent être H ; un alkyle en C_{1 à 6}, qui est non substitué ou substitué avec un halogène, un cyano, un nitro, NR⁹NR¹⁰, un alcoyle, un hydroxyle, un aryle non substitué, un hétéroaryle non substitué, CO₂R⁷, C(O)NR⁹R¹⁰, NHC(O)R8 ou SO₂NR⁹R¹⁰; un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R⁷, C(O)NR⁹R¹⁰, NHC(O)R8, SO₂NR⁹R¹⁰ ou un hydroxyle ; un hétéroaryle qui est non substitué ou substitué avec un cyano, un halogène, un nitro, un trifluorométhyle, un alkyle, un alkylthio, un alcoxy, NR⁹R¹⁰, CO₂R⁷, C(O)NR⁹R¹⁰, NHC(O)R⁸, SO₂NR⁹R¹⁰ ou un hydroxyle ou un cycloalkyle en C₃ à C₆ ; ou R4 et R5 peuvent former ensemble un cycle saturé, insaturé ou partiellement saturé à 4 à 7 éléments, où ledit cycle peut facultativement comprendre un ou plusieurs hétéroatomes supplémentaires choisis parmi N, O ou S ;
X est 0, S ou NR6 ;
R6 est H ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R⁸ ou SO₂NR⁹R¹⁰;
R7 est un hydrogène, un méthyle ou un éthyle ;
R8 est un méthyle ou un éthyle :
L est (CH₂)ₙ, où n est 1, 2 ou 3 ; et
Y est un alcényle ; un cycloalkyle en C₃ à C₆ :
un aryle en C₆ à C₁₀ qui est non substitué ou substitué avec un cyano, un halogène, un nitro, un trifluorométhyle, un alkyle, un alkylthio, un alcoxy, NR⁹R¹⁰, CO₂R7, C(O)NR⁹R¹⁰, NHC(O)R8, SO₂NR⁹R¹⁰ ou un hydroxyle, un groupe hétérocyclique qui est non substitué ou substitué avec un cyano, un nitro, un halogène, un alkyle, un alkylthio, un alkoxy, NR⁹R¹⁰, CO₂R7, C(O)NR⁹R¹⁰, NHC(O)R⁸, SO₂NR⁹R¹⁰ ou un hydroxyle, un cycloalkyle ; ou un alkyle en C_{1 à 6} qui est non substitué ou substitué avec un halogène, un cyano, un nitro, NR⁹NR¹⁰, un alcoxyle, un hydroxyle, un aryle non substitué, un hétéroaryle non substitué, CO₂R⁷, C(O)NR⁹R¹⁰, NHC(O)R⁸ ou SO₂NR⁹R¹⁰ dans la fabrication d'un médicament pour une utilisation dans l'inhibition des canaux potassiques.

15. Utilisation selon la revendication 14, dans laquelle le médicament est destiné à une utilisation dans le traitement ou la prévention de l'arythmie.
